# EUROPEAN PATENT APPLICATION

(11) **EP 3 954 769 A1**
(43) Date of publication of application: **16.02.2022**
(21) Application number: 20190205.3
(22) Date of filing: 10.08.2020
(51) Int. Cl.: C12N 9/10, C12P 19/18

(54) **PRODUCTION OF OLIGOSACCHARIDE MIXTURES BY A CELL**

(71) Applicant: Inbiose N.V., 9052 Zwijnaarde (BE)
(72) Inventor: AESAERT, Sofie, 9000 Gent (BE); BEAUPREZ, Joeri, 8450 Bredene (BE); COUSSEMENT, Pieter, 9050 Gentbrugge (BE); DECOENE, Thomas, 9032 Wondelgem (BE); LANNOO, Nausicaä, 9810 Eke-Nazareth (BE); PETERS, Gert, 9040 Sint-Amandsberg (BE); VANDEWALLE, Kristof, 8700 Tielt (BE)
(74) Representative: Saelens, Claire

(57) **Abstract**

The present invention is in the technical field of synthetic biology and metabolic engineering. More particularly, the present invention is in the technical field of fermentation of metabolically engineered cells. The present invention provides a method for the production of a mixture of at least two different oligosaccharides by a cell as well as the purification of at least one of said oligosaccharides from the cultivation. In addition, the present invention provides a method for the production of a mixture of at least two different oligosaccharides by a metabolically engineered cell as well as the purification of at least one of said oligosaccharides from the cultivation.

SEQUENCE LISTING

<110>  Inbiose N.V.

<120>  Production of oligosaccharide mixtures by a cell

<130>  025-EP-prio-1

<160>  32

<170>  PatentIn version 3.5

<210>  1
<211>  415
<212>  PRT
<213>  Escherichia coli W

<400>  1

Met Ala Leu Asn Ile Pro Phe Arg Asn Ala Tyr Tyr Arg Phe Ala Ser
1               5                   10                  15

Ser Tyr Ser Phe Leu Phe Phe Ile Ser Trp Ser Leu Trp Trp Ser Leu
            20                  25                  30

Tyr Ala Ile Trp Leu Lys Gly His Leu Gly Leu Thr Gly Thr Glu Leu
        35                  40                  45

Gly Thr Leu Tyr Ser Val Asn Gln Phe Thr Ser Ile Leu Phe Met Met
    50                  55                  60

Phe Tyr Gly Ile Val Gln Asp Lys Leu Gly Leu Lys Lys Pro Leu Ile
65                  70                  75                  80

Trp Cys Met Ser Phe Ile Leu Val Leu Thr Gly Pro Phe Met Ile Tyr
                85                  90                  95

Val Tyr Glu Pro Leu Leu Gln Ser Asn Phe Ser Val Gly Leu Ile Leu
            100                 105                 110

Gly Ala Leu Phe Phe Gly Leu Gly Tyr Leu Ala Gly Cys Gly Leu Leu
        115                 120                 125

Asp Ser Phe Thr Glu Lys Met Ala Arg Asn Phe His Phe Glu Tyr Gly
    130                 135                 140

Thr Ala Arg Ala Trp Gly Ser Phe Gly Tyr Ala Ile Gly Ala Phe Phe
145                 150                 155                 160

Ala Gly Ile Phe Phe Ser Ile Ser Pro His Ile Asn Phe Trp Leu Val
                165                 170                 175

Ser Leu Phe Gly Ala Val Phe Met Met Ile Asn Met Arg Phe Lys Asp
            180                 185                 190

Lys Asp His Gln Cys Val Ala Ala Asp Ala Gly Gly Val Lys Lys Glu
        195                 200                 205

Asp Phe Ile Ala Val Phe Lys Asp Arg Asn Phe Trp Val Phe Val Ile
    210                 215                 220

Phe Ile Val Gly Thr Trp Ser Phe Tyr Asn Ile Phe Asp Gln Gln Leu
225                 230                 235                 240

Phe Pro Val Phe Tyr Ser Gly Leu Phe Glu Ser His Asp Val Gly Thr
                245                 250                 255

Arg Leu Tyr Gly Tyr Leu Asn Ser Phe Gln Val Val Leu Glu Ala Leu
            260                 265                 270

Cys Met Ala Ile Ile Pro Phe Phe Val Asn Arg Val Gly Pro Lys Asn
        275                 280                 285

Ala Leu Leu Ile Gly Val Val Ile Met Ala Leu Arg Ile Leu Ser Cys
    290                 295                 300

Ala Leu Phe Val Asn Pro Trp Ile Ile Ser Leu Val Lys Leu Leu His
305                 310                 315                 320

Ala Ile Glu Val Pro Leu Cys Val Ile Ser Val Phe Lys Tyr Ser Val
                325                 330                 335

Ala Asn Phe Asp Lys Arg Leu Ser Ser Thr Ile Phe Leu Ile Gly Phe
            340                 345                 350

Gln Ile Ala Ser Ser Leu Gly Ile Val Leu Leu Ser Thr Pro Thr Gly
        355                 360                 365

Ile Leu Phe Asp His Ala Gly Tyr Gln Thr Val Phe Phe Ala Ile Ser
    370                 375                 380

Gly Ile Val Cys Leu Met Leu Leu Phe Gly Ile Phe Phe Leu Ser Lys
385                 390                 395                 400

Lys Arg Glu Gln Ile Val Met Glu Thr Pro Val Pro Ser Ala Ile
                405                 410                 415

<210>  2
<211>  301
<212>  PRT

<213>  Zymomonas mobilis

<400>  2

Met Lys Asn Asp Lys Lys Ile Tyr Gly Cys Ile Glu Gly Gly Gly Thr
1               5                   10                  15

Lys Phe Met Leu Ala Leu Ile Asp Ser Asp Arg Lys Met Leu Ala Val
            20                  25                  30

Glu Arg Val Pro Thr Thr Thr Pro Glu Glu Thr Leu Gly Lys Ser Val
        35                  40                  45

Glu Phe Phe Lys Lys Ala Leu Pro Gln Tyr Ala Asp Ser Phe Ala Ser
    50                  55                  60

Phe Gly Ile Ala Ser Phe Gly Pro Leu Cys Leu Asp Arg Lys Ser Pro
65                  70                  75                  80

Lys Trp Gly Tyr Ile Thr Asn Thr Pro Lys Pro Phe Trp Pro Asn Thr
                85                  90                  95

Asp Val Val Thr Pro Phe Lys Glu Ala Phe Gly Cys Pro Val Glu Ile
            100                 105                 110

Asp Thr Asp Val Asn Gly Ala Ala Leu Ala Glu Asn Phe Trp Gly Ala
        115                 120                 125

Ser Lys Gly Thr His Thr Ser Val Tyr Val Thr Val Gly Thr Gly Phe
    130                 135                 140

Gly Gly Gly Val Leu Ile Asp Gly Lys Pro Ile His Gly Leu Ala His
145                 150                 155                 160

Pro Glu Met Gly His Gly Ile Pro Ile Arg His Pro Asp Asp Arg Asp
                165                 170                 175

Phe Glu Gly Cys Cys Pro Tyr His Gly Gly Cys Tyr Glu Gly Leu Ala
            180                 185                 190

Ser Gly Thr Ala Ile Arg Lys Arg Trp Gly Lys Ala Leu Asn Glu Met
        195                 200                 205

Glu Pro Ala Glu Phe Glu Lys Ala Arg Glu Ile Ile Ala Phe Tyr Leu
    210                 215                 220

Ala His Phe Asn Val Thr Leu Gln Ala Phe Ile Ser Pro Glu Arg Ile
225                 230                 235                 240

Val Phe Gly Gly Gly Val Met His Val Asp Gly Met Leu Ala Ser Val
                245                 250                 255

Arg Arg Gln Thr Ala Glu Ile Ala Asn Ser Tyr Phe Glu Gly Ala Asp
            260                 265                 270

Phe Glu Lys Ile Ile Val Leu Pro Gly Leu Gly Asp Gln Ala Gly Met
        275                 280                 285

Met Gly Ala Phe Ala Leu Ala Leu Ala Ala Glu Asn Lys
    290                 295                 300

<210>  3
<211>  504
<212>  PRT
<213>  Bifidobacterium adolescentis

<400>  3

<210>  4
<211>  299
<212>  PRT
<213>  Helicobacter pylori UA1234

<400>  4

Met Ala Phe Lys Val Val Gln Ile Cys Gly Gly Leu Gly Asn Gln Met
1               5                   10                  15

Phe Gln Tyr Ala Phe Ala Lys Ser Leu Gln Lys His Ser Asn Thr Pro
            20                  25                  30

Val Leu Leu Asp Ile Thr Ser Phe Asp Trp Ser Asn Arg Lys Met Gln
        35                  40                  45

Leu Glu Leu Phe Pro Ile Asp Leu Pro Tyr Ala Ser Glu Lys Glu Ile
    50                  55                  60

Ala Ile Ala Lys Met Gln His Leu Pro Lys Leu Val Arg Asn Val Leu
65                  70                  75                  80

Lys Cys Met Gly Phe Asp Arg Val Ser Gln Glu Ile Val Phe Glu Tyr
                85                  90                  95

Glu Pro Lys Leu Leu Lys Thr Ser Arg Leu Thr Tyr Phe Tyr Gly Tyr
            100                 105                 110

Phe Gln Asp Pro Arg Tyr Phe Asp Ala Ile Ser Pro Leu Ile Lys Gln
        115                 120                 125

Thr Phe Thr Leu Pro Pro Pro Pro Glu Asn Gly Asn Asn Lys Lys Lys
    130                 135                 140

Glu Glu Glu Tyr His Arg Lys Leu Ala Leu Ile Leu Ala Ala Lys Asn
145                 150                 155                 160

Ser Val Phe Val His Ile Arg Arg Gly Asp Tyr Val Gly Ile Gly Cys
                165                 170                 175

Gln Leu Gly Ile Asp Tyr Gln Lys Lys Ala Leu Glu Tyr Met Ala Lys
            180                 185                 190

Arg Val Pro Asn Met Glu Leu Phe Val Phe Cys Glu Asp Leu Glu Phe
        195                 200                 205

Thr Gln Asn Leu Asp Leu Gly Tyr Pro Phe Met Asp Met Thr Thr Arg
    210                 215                 220

Asp Lys Glu Glu Glu Ala Tyr Trp Asp Met Leu Leu Met Gln Ser Cys
225                 230                 235                 240

Lys His Gly Ile Ile Ala Asn Ser Thr Tyr Ser Trp Trp Ala Ala Tyr
                245                 250                 255

Leu Ile Asn Asn Pro Glu Lys Ile Ile Ile Gly Pro Lys His Trp Leu
            260                 265                 270

Phe Gly His Glu Asn Ile Leu Cys Lys Glu Trp Val Lys Ile Glu Ser
        275                 280                 285

His Phe Glu Val Lys Ser Gln Lys Tyr Asn Ala
    290                 295

<210>  5
<211>  478
<212>  PRT
<213>  Helicobacter pylori UA1234

<400>  5

Met Phe Gln Pro Leu Leu Asp Ala Tyr Val Glu Ser Ala Ser Ile Glu
1               5                   10                  15

Lys Met Ala Ser Lys Ser Pro Pro Pro Leu Lys Ile Ala Val Ala Asn
            20                  25                  30

Trp Trp Gly Asp Glu Glu Ile Lys Glu Phe Lys Asn Ser Val Leu Tyr
        35                  40                  45

Phe Ile Leu Ser Gln Arg Tyr Thr Ile Thr Leu His Gln Asn Pro Asn
    50                  55                  60

Glu Phe Ser Asp Leu Val Phe Gly Asn Pro Leu Gly Ser Ala Arg Lys
65                  70                  75                  80

Ile Leu Ser Tyr Gln Asn Ala Lys Arg Val Phe Tyr Thr Gly Glu Asn
                85                  90                  95

Glu Ser Pro Asn Phe Asn Leu Phe Asp Tyr Ala Ile Gly Phe Asp Glu
            100                 105                 110

Leu Asp Phe Asn Asp Arg Tyr Leu Arg Met Pro Leu Tyr Tyr Asp Arg
        115                 120                 125

Leu His His Lys Ala Glu Ser Val Asn Asp Thr Thr Ala Pro Tyr Lys
    130                 135                 140

Leu Lys Asp Asn Ser Leu Tyr Ala Leu Lys Lys Pro Ser His Cys Phe
145                 150                 155                 160

Lys Glu Lys His Pro Asn Leu Cys Ala Val Val Asn Asp Glu Ser Asp
                165                 170                 175

Pro Leu Lys Arg Gly Phe Ala Ser Phe Val Ala Ser Asn Pro Asn Ala
            180                 185                 190

Pro Ile Arg Asn Ala Phe Tyr Asp Ala Leu Asn Ser Ile Glu Pro Val
        195                 200                 205

Thr Gly Gly Gly Ser Val Arg Asn Thr Leu Gly Tyr Asn Val Lys Asn
    210                 215                 220

Lys Asn Glu Phe Leu Ser Gln Tyr Lys Phe Asn Leu Cys Phe Glu Asn
225                 230                 235                 240

Thr Gln Gly Tyr Gly Tyr Val Thr Glu Lys Ile Ile Asp Ala Tyr Phe
                245                 250                 255

Ser His Thr Ile Pro Ile Tyr Trp Gly Ser Pro Ser Val Ala Lys Asp
            260                 265                 270

Phe Asn Pro Lys Ser Phe Val Asn Val His Asp Phe Lys Asn Phe Asp
        275                 280                 285

Glu Ala Ile Asp Tyr Ile Lys Tyr Leu His Thr His Lys Asn Ala Tyr

290                 295                 300

Leu Asp Met Leu Tyr Glu Asn Pro Leu Asn Thr Leu Asp Gly Lys Ala
305                 310                 315                 320

Tyr Phe Tyr Gln Asn Leu Ser Phe Lys Lys Ile Leu Ala Phe Phe Lys
                325                 330                 335

Thr Ile Leu Glu Asn Asp Thr Ile Tyr His Asp Asn Pro Phe Ile Phe
            340                 345                 350

Cys Arg Asp Leu Asn Glu Pro Leu Val Thr Ile Asp Asp Leu Arg Val
        355                 360                 365

Asn Tyr Asp Asp Leu Arg Val Asn Tyr Asp Asp Leu Arg Ile Asn Tyr
    370                 375                 380

Asp Asp Leu Arg Val Asn Tyr Asp Asp Leu Arg Ile Asn Tyr Asp Asp
385                 390                 395                 400

Leu Arg Val Asn Tyr Asp Asp Leu Arg Val Asn Tyr Asp Asp Leu Arg
                405                 410                 415

Ile Asn Tyr Asp Asp Leu Arg Val Asn Tyr Asp Asp Leu Arg Val Asn
            420                 425                 430

Tyr Glu Arg Leu Leu Ser Lys Ala Thr Pro Leu Leu Glu Leu Ser Gln
        435                 440                 445

Asn Thr Thr Ser Lys Ile Tyr Arg Lys Ala Tyr Gln Lys Ser Leu Pro
    450                 455                 460

Leu Leu Arg Ala Ile Arg Arg Trp Val Lys Lys Leu Gly Leu
465                 470                 475

<210>  6
<211>  412
<212>  PRT
<213>  Helicobacter pylori UA1234

<400>  6

Met Phe Gln Pro Leu Leu Asp Ala Tyr Val Glu Ser Ala Ser Ile Glu
1               5                   10                  15

Lys Met Ala Ser Lys Ser Pro Pro Pro Leu Lys Ile Ala Val Ala Asn
            20                  25                  30

Trp Trp Gly Asp Glu Glu Ile Lys Glu Phe Lys Asn Ser Val Leu Tyr

        35                  40                  45

Phe Ile Leu Ser Gln Arg Tyr Thr Ile Thr Leu His Gln Asn Pro Asn
    50                  55                  60

Glu Phe Ser Asp Leu Val Phe Gly Asn Pro Leu Gly Ser Ala Arg Lys
65                  70                  75                  80

Ile Leu Ser Tyr Gln Asn Ala Lys Arg Val Phe Tyr Thr Gly Glu Asn
                85                  90                  95

Glu Ser Pro Asn Phe Asn Leu Phe Asp Tyr Ala Ile Gly Phe Asp Glu
            100                 105                 110

Leu Asp Phe Asn Asp Arg Tyr Leu Arg Met Pro Leu Tyr Tyr Asp Arg
        115                 120                 125

Leu His His Lys Ala Glu Ser Val Asn Asp Thr Thr Ala Pro Tyr Lys
    130                 135                 140

Leu Lys Asp Asn Ser Leu Tyr Ala Leu Lys Lys Pro Ser His Cys Phe
145                 150                 155                 160

Lys Glu Lys His Pro Asn Leu Cys Ala Val Val Asn Asp Glu Ser Asp
                165                 170                 175

Pro Leu Lys Arg Gly Phe Ala Ser Phe Val Ala Ser Asn Pro Asn Ala
            180                 185                 190

Pro Ile Arg Asn Ala Phe Tyr Asp Ala Leu Asn Ser Ile Glu Pro Val
        195                 200                 205

Thr Gly Gly Gly Ser Val Arg Asn Thr Leu Gly Tyr Asn Val Lys Asn
    210                 215                 220

Lys Asn Glu Phe Leu Ser Gln Tyr Lys Phe Asn Leu Cys Phe Glu Asn
225                 230                 235                 240

Thr Gln Gly Tyr Gly Tyr Val Thr Glu Lys Ile Ile Asp Ala Tyr Phe
                245                 250                 255

Ser His Thr Ile Pro Ile Tyr Trp Gly Ser Pro Ser Val Ala Lys Asp
            260                 265                 270

Phe Asn Pro Lys Ser Phe Val Asn Val His Asp Phe Lys Asn Phe Asp
        275                 280                 285

Glu Ala Ile Asp Tyr Ile Lys Tyr Leu His Thr His Lys Asn Ala Tyr
    290                 295                 300

Leu Asp Met Leu Tyr Glu Asn Pro Leu Asn Thr Leu Asp Gly Lys Ala
305                 310                 315                 320

Tyr Phe Tyr Gln Asn Leu Ser Phe Lys Lys Ile Leu Ala Phe Phe Lys
                325                 330                 335

Thr Ile Leu Glu Asn Asp Thr Ile Tyr His Asp Asn Pro Phe Ile Phe
            340                 345                 350

Cys Arg Asp Leu Asn Glu Pro Leu Val Thr Ile Asp Asp Leu Arg Val
        355                 360                 365

Asn Tyr Asp Asp Leu Arg Val Asn Tyr Asp Asp Leu Arg Ile Asn Tyr
    370                 375                 380

Asp Asp Leu Arg Val Asn Tyr Asp Asp Leu Arg Ile Asn Tyr Asp Asp
385                 390                 395                 400

Leu Arg Val Asn Tyr Asp Asp Leu Arg Val Asn Tyr
                405                 410

<210>  7
<211>  264
<212>  PRT
<213>  Escherchia coli

<400>  7

Met Lys Gln Tyr Leu Glu Leu Met Gln Lys Val Leu Asp Glu Gly Thr
1               5                   10                  15

Gln Lys Asn Asp Arg Thr Gly Thr Gly Thr Leu Ser Ile Phe Gly His
            20                  25                  30

Gln Met Arg Phe Asn Leu Gln Asp Gly Phe Pro Leu Val Thr Thr Lys
        35                  40                  45

Arg Cys His Leu Arg Ser Ile Ile His Glu Leu Leu Trp Phe Leu Gln
    50                  55                  60

Gly Asp Thr Asn Ile Ala Tyr Leu His Glu Asn Asn Val Thr Ile Trp
65                  70                  75                  80

Asp Glu Trp Ala Asp Glu Asn Gly Asp Leu Gly Pro Val Tyr Gly Lys
                85                  90                  95

Gln Trp Arg Ala Trp Pro Thr Pro Asp Gly Arg His Ile Asp Gln Ile
            100                 105                 110

Thr Thr Val Leu Asn Gln Leu Lys Asn Asp Pro Asp Ser Arg Arg Ile
        115                 120                 125

Ile Val Ser Ala Trp Asn Val Gly Glu Leu Asp Lys Met Ala Leu Ala
    130                 135                 140

Pro Cys His Ala Phe Phe Gln Phe Tyr Val Ala Asp Gly Lys Leu Ser
145                 150                 155                 160

Cys Gln Leu Tyr Gln Arg Ser Cys Asp Val Phe Leu Gly Leu Pro Phe
                165                 170                 175

Asn Ile Ala Ser Tyr Ala Leu Leu Val His Met Met Ala Gln Gln Cys
            180                 185                 190

Asp Leu Glu Val Gly Asp Phe Val Trp Thr Gly Gly Asp Thr His Leu
        195                 200                 205

Tyr Ser Asn His Met Asp Gln Thr His Leu Gln Leu Ser Arg Glu Pro
    210                 215                 220

Arg Pro Leu Pro Lys Leu Ile Ile Lys Arg Lys Pro Glu Ser Ile Phe
225                 230                 235                 240

Asp Tyr Arg Phe Glu Asp Phe Glu Ile Glu Gly Tyr Asp Pro His Pro
                245                 250                 255

Gly Ile Lys Ala Pro Val Ala Ile
            260

<210>  8
<211>  391
<212>  PRT
<213>  Escherchia coli

<400>  8

Met Gln Lys Leu Ile Asn Ser Val Gln Asn Tyr Ala Trp Gly Ser Lys
1               5                   10                  15

Thr Ala Leu Thr Glu Leu Tyr Gly Met Glu Asn Pro Ser Ser Gln Pro
            20                  25                  30

Met Ala Glu Leu Trp Met Gly Ala His Pro Lys Ser Ser Ser Arg Val
        35                  40                  45

Gln Asn Ala Ala Gly Asp Ile Val Ser Leu Arg Asp Val Ile Glu Ser
    50                  55                  60

Asp Lys Ser Thr Leu Leu Gly Glu Ala Val Ala Lys Arg Phe Gly Glu
65                  70                  75                  80

Leu Pro Phe Leu Phe Lys Val Leu Cys Ala Ala Gln Pro Leu Ser Ile
                85                  90                  95

Gln Val His Pro Asn Lys His Asn Ser Glu Ile Gly Phe Ala Lys Glu
            100                 105                 110

Asn Ala Ala Gly Ile Pro Met Asp Ala Ala Glu Arg Asn Tyr Lys Asp
        115                 120                 125

Pro Asn His Lys Pro Glu Leu Val Phe Ala Leu Thr Pro Phe Leu Ala
    130                 135                 140

Met Asn Ala Phe Arg Glu Phe Ser Glu Ile Val Ser Leu Leu Gln Pro
145                 150                 155                 160

Val Ala Gly Ala His Pro Ala Ile Ala His Phe Leu Gln Gln Pro Asp
                165                 170                 175

Ala Glu Arg Leu Ser Glu Leu Phe Ala Ser Leu Leu Asn Met Gln Gly
            180                 185                 190

Glu Glu Lys Ser Arg Ala Leu Ala Ile Leu Lys Ser Ala Leu Asp Ser
        195                 200                 205

Gln Gln Gly Glu Pro Trp Gln Thr Ile Arg Leu Ile Ser Glu Phe Tyr
    210                 215                 220

Pro Glu Asp Ser Gly Leu Phe Ser Pro Leu Leu Leu Asn Val Val Lys
225                 230                 235                 240

Leu Asn Pro Gly Glu Ala Met Phe Leu Phe Ala Glu Thr Pro His Ala
                245                 250                 255

Tyr Leu Gln Gly Val Ala Leu Glu Val Met Ala Asn Ser Asp Asn Val
            260                 265                 270

Leu Arg Ala Gly Leu Thr Pro Lys Tyr Ile Asp Ile Pro Glu Leu Val
        275                 280                 285

Ala Asn Val Lys Phe Glu Ala Lys Pro Ala Asn Gln Leu Leu Thr Gln
    290                 295                 300

Pro Val Lys Gln Gly Ala Glu Leu Asp Phe Pro Ile Pro Val Asp Asp
305                 310                 315                 320

Phe Ala Phe Ser Leu His Asp Leu Ser Asp Lys Glu Thr Thr Ile Ser
                325                 330                 335

Gln Gln Ser Ala Ala Ile Leu Phe Cys Val Glu Gly Asp Ala Thr Leu
            340                 345                 350

Trp Lys Gly Ser Gln Gln Leu Gln Leu Lys Pro Gly Glu Ser Ala Phe
        355                 360                 365

Ile Ala Ala Asn Glu Ser Pro Val Thr Val Lys Gly His Gly Arg Leu
    370                 375                 380

Ala Arg Val Tyr Asn Lys Leu
385                 390

<210>  9
<211>  456
<212>  PRT
<213>  Escherchia coli

<400>  9

Met Lys Lys Leu Thr Cys Phe Lys Ala Tyr Asp Ile Arg Gly Lys Leu
1               5                   10                  15

Gly Glu Glu Leu Asn Glu Asp Ile Ala Trp Arg Ile Gly Arg Ala Tyr
            20                  25                  30

Gly Glu Phe Leu Lys Pro Lys Thr Ile Val Leu Gly Gly Asp Val Arg
        35                  40                  45

Leu Thr Ser Glu Thr Leu Lys Leu Ala Leu Ala Lys Gly Leu Gln Asp
    50                  55                  60

Ala Gly Val Asp Val Leu Asp Ile Gly Met Ser Gly Thr Glu Glu Ile
65                  70                  75                  80

Tyr Phe Ala Thr Phe His Leu Gly Val Asp Gly Gly Ile Glu Val Thr
                85                  90                  95

Ala Ser His Asn Pro Met Asp Tyr Asn Gly Met Lys Leu Val Arg Glu
            100                 105                 110

Gly Ala Arg Pro Ile Ser Gly Asp Thr Gly Leu Arg Asp Val Gln Arg
        115                 120                 125

Leu Ala Glu Ala Asn Asp Phe Pro Pro Val Asp Glu Thr Lys Arg Gly
    130                 135                 140

Arg Tyr Gln Gln Ile Asn Leu Arg Asp Ala Tyr Val Asp His Leu Phe
145                 150                 155                 160

Gly Tyr Ile Asn Val Lys Asn Leu Thr Pro Leu Lys Leu Val Ile Asn
                165                 170                 175

Ser Gly Asn Gly Ala Ala Gly Pro Val Val Asp Ala Ile Glu Ala Arg
            180                 185                 190

Phe Lys Ala Leu Gly Ala Pro Val Glu Leu Ile Lys Val His Asn Thr
        195                 200                 205

Pro Asp Gly Asn Phe Pro Asn Gly Ile Pro Asn Pro Leu Leu Pro Glu
    210                 215                 220

Cys Arg Asp Asp Thr Arg Asn Ala Val Ile Lys His Gly Ala Asp Met
225                 230                 235                 240

Gly Ile Ala Phe Asp Gly Asp Phe Asp Arg Cys Phe Leu Phe Asp Glu
                245                 250                 255

Lys Gly Gln Phe Ile Glu Gly Tyr Tyr Ile Val Gly Leu Leu Ala Glu
            260                 265                 270

Ala Phe Leu Glu Lys Asn Pro Gly Ala Lys Ile Ile His Asp Pro Arg
        275                 280                 285

Leu Ser Trp Asn Thr Val Asp Val Val Thr Ala Ala Gly Gly Thr Pro
    290                 295                 300

Val Met Ser Lys Thr Gly His Ala Phe Ile Lys Glu Arg Met Arg Lys
305                 310                 315                 320

Glu Asp Ala Ile Tyr Gly Gly Glu Met Ser Ala His His Tyr Phe Arg
                325                 330                 335

Asp Phe Ala Tyr Cys Asp Ser Gly Met Ile Pro Trp Leu Leu Val Ala
            340                 345                 350

Glu Leu Val Cys Leu Lys Asp Lys Thr Leu Gly Glu Leu Val Arg Asp
        355                 360                 365

Arg Met Ala Ala Phe Pro Ala Ser Gly Glu Ile Asn Ser Lys Leu Ala
    370                 375                 380

Gln Pro Val Glu Ala Ile Asn Arg Val Glu Gln His Phe Ser Arg Glu
385                 390                 395                 400

Ala Leu Ala Val Asp Arg Thr Asp Gly Ile Ser Met Thr Phe Ala Asp
                405                 410                 415

Trp Arg Phe Asn Leu Arg Thr Ser Asn Thr Glu Pro Val Val Arg Leu
            420                 425                 430

Asn Val Glu Ser Arg Gly Asp Val Pro Leu Met Glu Ala Arg Thr Arg
        435                 440                 445

Thr Leu Leu Thr Leu Leu Asn Glu
    450                 455

<210>  10
<211>  478
<212>  PRT
<213>  Escherchia coli

<400>  10

Met Ala Gln Ser Lys Leu Tyr Pro Val Val Met Ala Gly Gly Ser Gly
1               5                   10                  15

Ser Arg Leu Trp Pro Leu Ser Arg Val Leu Tyr Pro Lys Gln Phe Leu
            20                  25                  30

Cys Leu Lys Gly Asp Leu Thr Met Leu Gln Thr Thr Ile Cys Arg Leu
        35                  40                  45

Asn Gly Val Glu Cys Glu Ser Pro Val Val Ile Cys Asn Glu Gln His
    50                  55                  60

Arg Phe Ile Val Ala Glu Gln Leu Arg Gln Leu Asn Lys Leu Thr Glu
65                  70                  75                  80

Asn Ile Ile Leu Glu Pro Ala Gly Arg Asn Thr Ala Pro Ala Ile Ala
                85                  90                  95

Leu Ala Ala Leu Ala Ala Lys Arg His Ser Pro Glu Ser Asp Pro Leu
            100                 105                 110

Met Leu Val Leu Ala Ala Asp His Val Ile Ala Asp Glu Asp Ala Phe
        115                 120                 125

Arg Ala Ala Val Arg Asn Ala Met Pro Tyr Ala Glu Ala Gly Lys Leu
    130                 135                 140

Val Thr Phe Gly Ile Val Pro Asp Leu Pro Glu Thr Gly Tyr Gly Tyr
145                 150                 155                 160

Ile Arg Arg Gly Glu Val Ser Ala Gly Glu Gln Asp Met Val Ala Phe
                165                 170                 175

Glu Val Ala Gln Phe Val Glu Lys Pro Asn Leu Glu Thr Ala Gln Ala
            180                 185                 190

Tyr Val Ala Ser Gly Glu Tyr Tyr Trp Asn Ser Gly Met Phe Leu Phe
        195                 200                 205

Arg Ala Gly Arg Tyr Leu Glu Glu Leu Lys Lys Tyr Arg Pro Asp Ile
    210                 215                 220

Leu Asp Ala Cys Glu Lys Ala Met Ser Ala Val Asp Pro Asp Leu Asn
225                 230                 235                 240

Phe Ile Arg Val Asp Glu Glu Ala Phe Leu Ala Cys Pro Glu Glu Ser
                245                 250                 255

Val Asp Tyr Ala Val Met Glu Arg Thr Ala Asp Ala Val Val Val Pro
            260                 265                 270

Met Asp Ala Gly Trp Ser Asp Val Gly Ser Trp Ser Ser Leu Trp Glu
        275                 280                 285

Ile Ser Ala His Thr Ala Glu Gly Asn Val Cys His Gly Asp Val Ile
    290                 295                 300

Asn His Lys Thr Glu Asn Ser Tyr Val Tyr Ala Glu Ser Gly Leu Val
305                 310                 315                 320

Thr Thr Val Gly Val Lys Asp Leu Val Val Val Gln Thr Lys Asp Ala
                325                 330                 335

Val Leu Ile Ala Asp Arg Asn Ala Val Gln Asp Val Lys Lys Val Val
            340                 345                 350

Glu Gln Ile Lys Ala Asp Gly Arg His Glu His Arg Val His Arg Glu
        355                 360                 365

Val Tyr Arg Pro Trp Gly Lys Tyr Asp Ser Ile Asp Ala Gly Asp Arg
    370                 375                 380

Tyr Gln Val Lys Arg Ile Thr Val Lys Pro Gly Glu Gly Leu Ser Val

385                 390                 395                 400

Gln Met His His His Arg Ala Glu His Trp Val Val Val Ala Gly Thr
                405                 410                 415

Ala Lys Val Thr Ile Asp Gly Asp Ile Lys Leu Leu Gly Glu Asn Glu
            420                 425                 430

Ser Ile Tyr Ile Pro Leu Gly Ala Thr His Cys Leu Glu Asn Pro Gly
        435                 440                 445

Lys Ile Pro Leu Asp Leu Ile Glu Val Arg Ser Gly Ser Tyr Leu Glu
    450                 455                 460

Glu Asp Asp Val Val Arg Phe Ala Asp Arg Tyr Gly Arg Val
465                 470                 475

<210>  11
<211>  373
<212>  PRT
<213>  Escherchia coli

<400>  11

<210>  12
<211>  321
<212>  PRT
<213>  Escherchia coli

<400>  12

Met Ser Lys Gln Arg Val Phe Ile Ala Gly His Arg Gly Met Val Gly
1               5                   10                  15

Ser Ala Ile Arg Arg Gln Leu Glu Gln Arg Gly Asp Val Glu Leu Val
            20                  25                  30

Leu Arg Thr Arg Asp Glu Leu Asn Leu Leu Asp Ser Arg Ala Val His
        35                  40                  45

Asp Phe Phe Ala Ser Glu Arg Ile Asp Gln Val Tyr Leu Ala Ala Ala
    50                  55                  60

Lys Val Gly Gly Ile Val Ala Asn Asn Thr Tyr Pro Ala Asp Phe Ile
65                  70                  75                  80

Tyr Gln Asn Met Met Ile Glu Ser Asn Ile Ile His Ala Ala His Gln
                85                  90                  95

Asn Asp Val Asn Lys Leu Leu Phe Leu Gly Ser Ser Cys Ile Tyr Pro
            100                 105                 110

Lys Leu Ala Lys Gln Pro Met Ala Glu Ser Glu Leu Leu Gln Gly Thr
        115                 120                 125

Leu Glu Pro Thr Asn Glu Pro Tyr Ala Ile Ala Lys Ile Ala Gly Ile
    130                 135                 140

Lys Leu Cys Glu Ser Tyr Asn Arg Gln Tyr Gly Arg Asp Tyr Arg Ser
145                 150                 155                 160

Val Met Pro Thr Asn Leu Tyr Gly Pro His Asp Asn Phe His Pro Ser
                165                 170                 175

Asn Ser His Val Ile Pro Ala Leu Leu Arg Arg Phe His Glu Ala Thr
            180                 185                 190

Ala Gln Asn Ala Pro Asp Val Val Val Trp Gly Ser Gly Thr Pro Met
        195                 200                 205

Arg Glu Phe Leu His Val Asp Asp Met Ala Ala Ala Ser Ile His Val
    210                 215                 220

Met Glu Leu Ala His Glu Val Trp Leu Glu Asn Thr Gln Pro Met Leu
225                 230                 235                 240

Ser His Ile Asn Val Gly Thr Gly Val Asp Cys Thr Ile Arg Glu Leu
                245                 250                 255

Ala Gln Thr Ile Ala Lys Val Val Gly Tyr Lys Gly Arg Val Val Phe
            260                 265                 270

Asp Ala Ser Lys Pro Asp Gly Thr Pro Arg Lys Leu Leu Asp Val Thr
        275                 280                 285

Arg Leu His Gln Leu Gly Trp Tyr His Glu Ile Ser Leu Glu Ala Gly
    290                 295                 300

Leu Ala Ser Thr Tyr Gln Trp Phe Leu Glu Asn Gln Asp Arg Phe Arg
305                 310                 315                 320

Gly

<210>  13
<211>  438
<212>  PRT
<213>  Escherchia coli

<400>  13

Met Gly Asn Thr Ser Ile Gln Thr Gln Ser Tyr Arg Ala Val Asp Lys
1               5                   10                  15

Asp Ala Gly Gln Ser Arg Ser Tyr Ile Ile Pro Phe Ala Leu Leu Cys
            20                  25                  30

Ser Leu Phe Phe Leu Trp Ala Val Ala Asn Asn Leu Asn Asp Ile Leu
        35                  40                  45

Leu Pro Gln Phe Gln Gln Ala Phe Thr Leu Thr Asn Phe Gln Ala Gly
    50                  55                  60

Leu Ile Gln Ser Ala Phe Tyr Phe Gly Tyr Phe Ile Ile Pro Ile Pro
65                  70                  75                  80

Ala Gly Ile Leu Met Lys Lys Leu Ser Tyr Lys Ala Gly Ile Ile Thr
                85                  90                  95

Gly Leu Phe Leu Tyr Ala Leu Gly Ala Ala Leu Phe Trp Pro Ala Ala
            100                 105                 110

Glu Ile Met Asn Tyr Thr Leu Phe Leu Val Gly Leu Phe Ile Ile Ala
        115                 120                 125

Ala Gly Leu Gly Cys Leu Glu Thr Ala Ala Asn Pro Phe Val Thr Val
    130                 135                 140

Leu Gly Pro Glu Ser Ser Gly His Phe Arg Leu Asn Leu Ala Gln Thr
145                 150                 155                 160

Phe Asn Ser Phe Gly Ala Ile Ile Ala Val Val Phe Gly Gln Ser Leu
                165                 170                 175

Ile Leu Ser Asn Val Pro His Gln Ser Gln Asp Val Leu Asp Lys Met
            180                 185                 190

Ser Pro Glu Gln Leu Ser Ala Tyr Lys His Ser Leu Val Leu Ser Val
        195                 200                 205

Gln Thr Pro Tyr Met Ile Ile Val Ala Ile Val Leu Leu Val Ala Leu
    210                 215                 220

Leu Ile Met Leu Thr Lys Phe Pro Ala Leu Gln Ser Asp Asn His Ser
225                 230                 235                 240

Asp Ala Lys Gln Gly Ser Phe Ser Ala Ser Leu Ser Arg Leu Ala Arg
                245                 250                 255

Ile Arg His Trp Arg Trp Ala Val Leu Ala Gln Phe Cys Tyr Val Gly
            260                 265                 270

Ala Gln Thr Ala Cys Trp Ser Tyr Leu Ile Arg Tyr Ala Val Glu Glu
        275                 280                 285

Ile Pro Gly Met Thr Ala Gly Phe Ala Ala Asn Tyr Leu Thr Gly Thr
    290                 295                 300

Met Val Cys Phe Phe Ile Gly Arg Phe Thr Gly Thr Trp Leu Ile Ser
305                 310                 315                 320

Arg Phe Ala Pro His Lys Val Leu Ala Ala Tyr Ala Leu Ile Ala Met
                325                 330                 335

Ala Leu Cys Leu Ile Ser Ala Phe Ala Gly Gly His Val Gly Leu Ile
            340                 345                 350

Ala Leu Thr Leu Cys Ser Ala Phe Met Ser Ile Gln Tyr Pro Thr Ile
        355                 360                 365

Phe Ser Leu Gly Ile Lys Asn Leu Gly Gln Asp Thr Lys Tyr Gly Ser
    370                 375                 380

Ser Phe Ile Val Met Thr Ile Ile Gly Gly Gly Ile Val Thr Pro Val
385                 390                 395                 400

Met Gly Phe Val Ser Asp Ala Ala Gly Asn Ile Pro Thr Ala Glu Leu
                405                 410                 415

Ile Pro Ala Leu Cys Phe Ala Val Ile Phe Ile Phe Ala Arg Phe Arg
            420                 425                 430

Ser Gln Thr Ala Thr Asn
        435

<210>  14
<211>  949
<212>  PRT
<213>  Bacteroides fragilis NCTC 9343

<400>  14

Met Gln Lys Leu Leu Ser Leu Pro Ser Asn Leu Val Gln Ser Phe His
1               5                   10                  15

Glu Leu Glu Arg Val Asn Arg Thr Asp Trp Phe Cys Thr Ser Asp Pro
            20                  25                  30

Val Gly Lys Lys Leu Gly Ser Gly Gly Gly Thr Ser Trp Leu Leu Glu
        35                  40                  45

Glu Cys Tyr Asn Glu Tyr Ser Asp Gly Ala Thr Phe Gly Glu Trp Leu
    50                  55                  60

Glu Lys Glu Lys Arg Ile Leu Leu His Ala Gly Gly Gln Ser Arg Arg
65                  70                  75                  80

Leu Pro Gly Tyr Ala Pro Ser Gly Lys Ile Leu Thr Pro Val Pro Val
                85                  90                  95

Phe Arg Trp Glu Arg Gly Gln His Leu Gly Gln Asn Leu Leu Ser Leu
            100                 105                 110

Gln Leu Pro Leu Tyr Glu Lys Ile Met Ser Leu Ala Pro Asp Lys Leu
        115                 120                 125

His Thr Leu Ile Ala Ser Gly Asp Val Tyr Ile Arg Ser Glu Lys Pro
    130                 135                 140

Leu Gln Ser Ile Pro Glu Ala Asp Val Val Cys Tyr Gly Leu Trp Val
145                 150                 155                 160

Asp Pro Ser Leu Ala Thr His His Gly Val Phe Ala Ser Asp Arg Lys
                165                 170                 175

His Pro Glu Gln Leu Asp Phe Met Leu Gln Lys Pro Ser Leu Ala Glu
            180                 185                 190

Leu Glu Ser Leu Ser Lys Thr His Leu Phe Leu Met Asp Ile Gly Ile
        195                 200                 205

Trp Leu Leu Ser Asp Arg Ala Val Glu Ile Leu Met Lys Arg Ser His
    210                 215                 220

Lys Glu Ser Ser Glu Glu Leu Lys Tyr Tyr Asp Leu Tyr Ser Asp Phe
225                 230                 235                 240

Gly Leu Ala Leu Gly Thr His Pro Arg Ile Glu Asp Glu Glu Val Asn
                245                 250                 255

Thr Leu Ser Val Ala Ile Leu Pro Leu Pro Gly Gly Glu Phe Tyr His
            260                 265                 270

Tyr Gly Thr Ser Lys Glu Leu Ile Ser Ser Thr Leu Ser Val Gln Asn
        275                 280                 285

Lys Val Tyr Asp Gln Arg Arg Ile Met His Arg Lys Val Lys Pro Asn
    290                 295                 300

Pro Ala Met Phe Val Gln Asn Ala Val Val Arg Ile Pro Leu Cys Ala
305                 310                 315                 320

Glu Asn Ala Asp Leu Trp Ile Glu Asn Ser His Ile Gly Pro Lys Trp
                325                 330                 335

Lys Ile Ala Ser Arg His Ile Ile Thr Gly Val Pro Glu Asn Asp Trp
            340                 345                 350

Ser Leu Ala Val Pro Ala Gly Val Cys Val Asp Val Val Pro Met Gly
        355                 360                 365

Asp Lys Gly Phe Val Ala Arg Pro Tyr Gly Leu Asp Asp Val Phe Lys
    370                 375                 380

Gly Asp Leu Arg Asp Ser Lys Thr Thr Leu Thr Gly Ile Pro Phe Gly
385                 390                 395                 400

Glu Trp Met Ser Lys Arg Gly Leu Ser Tyr Thr Asp Leu Lys Gly Arg
                405                 410                 415

Thr Asp Asp Leu Gln Ala Val Ser Val Phe Pro Met Val Asn Ser Val
            420                 425                 430

Glu Glu Leu Gly Leu Val Leu Arg Trp Met Leu Ser Glu Pro Glu Leu
        435                 440                 445

Glu Glu Gly Lys Asn Ile Trp Leu Arg Ser Glu His Phe Ser Ala Asp
    450                 455                 460

Glu Ile Ser Ala Gly Ala Asn Leu Lys Arg Leu Tyr Ala Gln Arg Glu
465                 470                 475                 480

Glu Phe Arg Lys Gly Asn Trp Lys Ala Leu Ala Val Asn His Glu Lys
                485                 490                 495

Ser Val Phe Tyr Gln Leu Asp Leu Ala Asp Ala Ala Glu Asp Phe Val
            500                 505                 510

Arg Leu Gly Leu Asp Met Pro Glu Leu Leu Pro Glu Asp Ala Leu Gln
        515                 520                 525

Met Ser Arg Ile His Asn Arg Met Leu Arg Ala Arg Ile Leu Lys Leu
    530                 535                 540

Asp Gly Lys Asp Tyr Arg Pro Glu Glu Gln Ala Ala Phe Asp Leu Leu
545                 550                 555                 560

Arg Asp Gly Leu Leu Asp Gly Ile Ser Asn Arg Lys Ser Thr Pro Lys
                565                 570                 575

Leu Asp Val Tyr Ser Asp Gln Ile Val Trp Gly Arg Ser Pro Val Arg
            580                 585                 590

Ile Asp Met Ala Gly Gly Trp Thr Asp Thr Pro Pro Tyr Ser Leu Tyr
        595                 600                 605

Ser Gly Gly Asn Val Val Asn Leu Ala Ile Glu Leu Asn Gly Gln Pro
    610                 615                 620

Pro Leu Gln Val Tyr Val Lys Pro Cys Lys Asp Phe His Ile Val Leu
625                 630                 635                 640

Arg Ser Ile Asp Met Gly Ala Met Glu Ile Val Ser Thr Phe Asp Glu

                645                 650                 655

Leu Gln Asp Tyr Lys Lys Ile Gly Ser Pro Phe Ser Ile Pro Lys Ala
            660                 665                 670

Ala Leu Ser Leu Ala Gly Phe Ala Pro Ala Phe Ser Ala Val Ser Tyr
        675                 680                 685

Ala Ser Leu Glu Glu Gln Leu Lys Asp Phe Gly Ala Gly Ile Glu Val
    690                 695                 700

Thr Leu Leu Ala Ala Ile Pro Ala Gly Ser Gly Leu Gly Thr Ser Ser
705                 710                 715                 720

Ile Leu Ala Ser Thr Val Leu Gly Ala Ile Asn Asp Phe Cys Gly Leu
                725                 730                 735

Ala Trp Asp Lys Asn Glu Ile Cys Gln Arg Thr Leu Val Leu Glu Gln
            740                 745                 750

Leu Leu Thr Thr Gly Gly Gly Trp Gln Asp Gln Tyr Gly Gly Val Leu
        755                 760                 765

Gln Gly Val Lys Leu Leu Gln Thr Glu Ala Gly Phe Ala Gln Ser Pro
    770                 775                 780

Leu Val Arg Trp Leu Pro Asp His Leu Phe Thr His Pro Glu Tyr Lys
785                 790                 795                 800

Asp Cys His Leu Leu Tyr Tyr Thr Gly Ile Thr Arg Thr Ala Lys Gly
                805                 810                 815

Ile Leu Ala Glu Ile Val Ser Ser Met Phe Leu Asn Ser Ser Leu His
            820                 825                 830

Leu Asn Leu Leu Ser Glu Met Lys Ala His Ala Leu Asp Met Asn Glu
        835                 840                 845

Ala Ile Gln Arg Gly Ser Phe Val Glu Phe Gly Arg Leu Val Gly Lys
    850                 855                 860

Thr Trp Glu Gln Asn Lys Ala Leu Asp Ser Gly Thr Asn Pro Pro Ala
865                 870                 875                 880

Val Glu Ala Ile Ile Asp Leu Ile Lys Asp Tyr Thr Leu Gly Tyr Lys
                885                 890                 895

Leu Pro Gly Ala Gly Gly Gly Gly Tyr Leu Tyr Met Val Ala Lys Asp
            900                 905                 910

Pro Gln Ala Ala Val Arg Ile Arg Lys Ile Leu Thr Glu Asn Ala Pro
        915                 920                 925

Asn Pro Arg Ala Arg Phe Val Glu Met Thr Leu Ser Asp Lys Gly Phe
    930                 935                 940

Gln Val Ser Arg Ser
945

<210>  15
<211>  417
<212>  PRT
<213>  Escherchia coli

<400>  15

<210>  16
<211>  159
<212>  PRT
<213>  Saccharomyces cerevisiae

<400>  16

Met Ser Leu Pro Asp Gly Phe Tyr Ile Arg Arg Met Glu Glu Gly Asp
1               5                   10                  15

Leu Glu Gln Val Thr Glu Thr Leu Lys Val Leu Thr Thr Val Gly Thr
            20                  25                  30

Ile Thr Pro Glu Ser Phe Ser Lys Leu Ile Lys Tyr Trp Asn Glu Ala
        35                  40                  45

Thr Val Trp Asn Asp Asn Glu Asp Lys Lys Ile Met Gln Tyr Asn Pro
    50                  55                  60

Met Val Ile Val Asp Lys Arg Thr Glu Thr Val Ala Ala Thr Gly Asn
65                  70                  75                  80

Ile Ile Ile Glu Arg Lys Ile Ile His Glu Leu Gly Leu Cys Gly His
                85                  90                  95

Ile Glu Asp Ile Ala Val Asn Ser Lys Tyr Gln Gly Gln Gly Leu Gly
            100                 105                 110

Lys Leu Leu Ile Asp Gln Leu Val Thr Ile Gly Phe Asp Tyr Gly Cys
        115                 120                 125

Tyr Lys Ile Ile Leu Asp Cys Asp Glu Lys Asn Val Lys Phe Tyr Glu
    130                 135                 140

Lys Cys Gly Phe Ser Asn Ala Gly Val Glu Met Gln Ile Arg Lys
145                 150                 155

<210>  17
<211>  421
<212>  PRT
<213>  Bacteroides ovatus

<400>  17

Met Asp Ser Lys Asn Asn Ile Gly His Ser Ala Asp Ile Ser Leu Thr
1               5                   10                  15

Ala Glu Leu Pro Ile Pro Ile Tyr Asn Gly Asn Thr Ile Met Asp Phe
            20                  25                  30

Lys Lys Leu Ala Ser Leu Tyr Lys Asp Glu Leu Leu Asp Asn Val Leu
        35                  40                  45

Pro Phe Trp Leu Glu His Ser Gln Asp His Glu Tyr Gly Gly Tyr Phe
    50                  55                  60

Thr Cys Leu Asp Arg Glu Gly Lys Val Phe Asp Thr Asp Lys Phe Ile
65                  70                  75                  80

Trp Leu Gln Ser Arg Glu Val Trp Met Phe Ser Met Leu Tyr Asn Lys
                85                  90                  95

Val Glu Lys Arg Gln Glu Trp Leu Asp Cys Ala Ile Gln Gly Gly Glu
            100                 105                 110

Phe Leu Lys Lys Tyr Gly His Asp Gly Asn Tyr Asn Trp Tyr Phe Ser
        115                 120                 125

Leu Asp Arg Ser Gly Arg Pro Leu Val Glu Pro Tyr Asn Ile Phe Ser
    130                 135                 140

Tyr Thr Phe Ala Thr Met Ala Phe Gly Gln Leu Ser Leu Thr Thr Gly
145                 150                 155                 160

Asn Gln Glu Tyr Ala Asp Ile Ala Lys Lys Thr Phe Asp Ile Ile Leu
                165                 170                 175

Ser Lys Val Asp Asn Pro Lys Gly Arg Trp Asn Lys Leu His Pro Gly
            180                 185                 190

Thr Arg Asn Leu Lys Asn Phe Ala Leu Pro Met Ile Leu Cys Asn Leu
        195                 200                 205

Ala Leu Glu Ile Glu His Leu Leu Asp Glu Thr Tyr Leu Arg Glu Thr
    210                 215                 220

Met Asp Thr Cys Ile His Glu Val Met Glu Val Phe Tyr Arg Pro Glu
225                 230                 235                 240

Leu Gly Gly Ile Ile Val Glu Asn Val Asp Ile Asp Gly Asn Leu Val
                245                 250                 255

Asp Cys Phe Glu Gly Arg Gln Val Thr Pro Gly His Ala Ile Glu Ala
            260                 265                 270

Met Trp Phe Ile Met Asp Leu Gly Lys Arg Leu Asn Arg Pro Glu Leu
        275                 280                 285

Ile Glu Lys Ala Lys Glu Thr Thr Leu Thr Met Leu Asn Tyr Gly Trp
    290                 295                 300

Asp Lys Gln Tyr Gly Gly Ile Tyr Tyr Phe Met Asp Arg Asn Gly Cys
305                 310                 315                 320

Pro Pro Gln Gln Leu Glu Trp Asp Gln Lys Leu Trp Trp Val His Ile
                325                 330                 335

Glu Thr Leu Ile Ser Leu Leu Lys Gly Tyr Gln Leu Thr Gly Asp Lys
            340                 345                 350

Lys Cys Leu Glu Trp Phe Glu Lys Val His Asp Tyr Thr Trp Glu His
        355                 360                 365

Phe Lys Asp Lys Glu Tyr Pro Glu Trp Tyr Gly Tyr Leu Asn Arg Arg
    370                 375                 380

Gly Glu Val Leu Leu Pro Leu Lys Gly Gly Lys Trp Lys Gly Cys Phe
385                 390                 395                 400

His Val Pro Arg Gly Leu Tyr Gln Cys Trp Lys Thr Leu Glu Glu Ile
                405                 410                 415

Lys Asn Ile Val Ser
            420

<210>  18
<211>  349
<212>  PRT
<213>  Neisseria meningitidis

<400>  18

Met Gln Asn Asn Asn Glu Phe Lys Ile Gly Asn Arg Ser Val Gly Tyr
1               5                   10                  15

Asn His Glu Pro Leu Ile Ile Cys Glu Ile Gly Ile Asn His Glu Gly
            20                  25                  30

Ser Leu Lys Thr Ala Phe Glu Met Val Asp Ala Ala Tyr Asn Ala Gly
        35                  40                  45

Ala Glu Val Val Lys His Gln Thr His Ile Val Glu Asp Glu Met Ser
    50                  55                  60

Asp Glu Ala Lys Gln Val Ile Pro Gly Asn Ala Asp Val Ser Ile Tyr
65                  70                  75                  80

Glu Ile Met Glu Arg Cys Ala Leu Asn Glu Glu Asp Glu Ile Lys Leu
                85                  90                  95

Lys Glu Tyr Val Glu Ser Lys Gly Met Ile Phe Ile Ser Thr Pro Phe
            100                 105                 110

Ser Arg Ala Ala Ala Leu Arg Leu Gln Arg Met Asp Ile Pro Ala Tyr
        115                 120                 125

Lys Ile Gly Ser Gly Glu Cys Asn Asn Tyr Pro Leu Ile Lys Leu Val
    130                 135                 140

Ala Ser Phe Gly Lys Pro Ile Ile Leu Ser Thr Gly Met Asn Ser Ile
145                 150                 155                 160

Glu Ser Ile Lys Lys Ser Val Glu Ile Ile Arg Glu Ala Gly Val Pro
                165                 170                 175

Tyr Ala Leu Leu His Cys Thr Asn Ile Tyr Pro Thr Pro Tyr Glu Asp
            180                 185                 190

Val Arg Leu Gly Gly Met Asn Asp Leu Ser Glu Ala Phe Pro Asp Ala
        195                 200                 205

Ile Ile Gly Leu Ser Asp His Thr Leu Asp Asn Tyr Ala Cys Leu Gly
    210                 215                 220

Ala Val Ala Leu Gly Gly Ser Ile Leu Glu Arg His Phe Thr Asp Arg
225                 230                 235                 240

Met Asp Arg Pro Gly Pro Asp Ile Val Cys Ser Met Asn Pro Asp Thr
                245                 250                 255

Phe Lys Glu Leu Lys Gln Gly Ala His Ala Leu Lys Leu Ala Arg Gly
            260                 265                 270

Gly Lys Lys Asp Thr Ile Ile Ala Gly Glu Lys Pro Thr Lys Asp Phe
        275                 280                 285

Ala Phe Ala Ser Val Val Ala Asp Lys Asp Ile Lys Lys Gly Glu Leu
    290                 295                 300

Leu Ser Gly Asp Asn Leu Trp Val Lys Arg Pro Gly Asn Gly Asp Phe
305                 310                 315                 320

Ser Val Asn Glu Tyr Glu Thr Leu Phe Gly Lys Val Ala Ala Cys Asn
                325                 330                 335

Ile Arg Lys Gly Ala Gln Ile Lys Lys Thr Asp Ile Glu
            340                 345

<210>  19
<211>  609
<212>  PRT
<213>  Escherichia coli

<400>  19

Met Cys Gly Ile Val Gly Ala Ile Ala Gln Arg Asp Val Ala Glu Ile
1               5                   10                  15

Leu Leu Glu Gly Leu Arg Arg Leu Glu Tyr Arg Gly Tyr Asp Ser Ala
            20                  25                  30

Gly Leu Ala Val Val Asp Thr Glu Gly His Met Thr Arg Leu Arg Arg
        35                  40                  45

Leu Gly Lys Val Gln Met Leu Ala Gln Ala Ala Glu Glu His Pro Leu
    50                  55                  60

His Gly Gly Thr Gly Ile Ala His Thr Arg Trp Ala Thr His Gly Glu
65                  70                  75                  80

Pro Ser Glu Val Asn Ala His Pro His Val Ser Glu His Ile Val Val
                85                  90                  95

Val His Asn Gly Ile Ile Glu Asn His Glu Pro Leu Arg Glu Glu Leu
            100                 105                 110

Lys Ala Arg Gly Tyr Thr Phe Val Ser Glu Thr Asp Thr Glu Val Ile
        115                 120                 125

Ala His Leu Val Asn Trp Glu Leu Lys Gln Gly Gly Thr Leu Arg Glu
    130                 135                 140

Ala Val Leu Arg Ala Ile Pro Gln Leu Arg Gly Ala Tyr Gly Thr Val
145                 150                 155                 160

Ile Met Asp Ser Arg His Pro Asp Thr Leu Leu Ala Ala Arg Ser Gly
                165                 170                 175

Ser Pro Leu Val Ile Gly Leu Gly Met Gly Glu Asn Phe Ile Ala Ser
            180                 185                 190

Asp Gln Leu Ala Leu Leu Pro Val Thr Arg Arg Phe Ile Phe Leu Glu
        195                 200                 205

Glu Gly Asp Ile Ala Glu Ile Thr Arg Arg Ser Val Asn Ile Phe Asp
    210                 215                 220

Lys Thr Gly Ala Glu Val Lys Arg Gln Asp Ile Glu Ser Asn Leu Gln
225                 230                 235                 240

Tyr Asp Ala Gly Asp Lys Gly Ile Tyr Cys His Tyr Met Gln Lys Glu
                245                 250                 255

Ile Tyr Glu Gln Pro Asn Ala Ile Lys Asn Thr Leu Thr Gly Arg Ile
            260                 265                 270

Ser His Gly Gln Val Asp Leu Ser Glu Leu Gly Pro Asn Ala Asp Glu
        275                 280                 285

Leu Leu Ser Lys Val Glu His Ile Gln Ile Leu Ala Cys Gly Thr Ser
    290                 295                 300

Tyr Asn Ser Gly Met Val Ser Arg Tyr Trp Phe Glu Ser Leu Ala Gly
305                 310                 315                 320

Ile Pro Cys Asp Val Glu Ile Ala Ser Glu Phe Arg Tyr Arg Lys Ser
                325                 330                 335

Ala Val Arg Arg Asn Ser Leu Met Ile Thr Leu Ser Gln Ser Gly Glu
            340                 345                 350

Thr Ala Asp Thr Leu Ala Gly Leu Arg Leu Ser Lys Glu Leu Gly Tyr
        355                 360                 365

Leu Gly Ser Leu Ala Ile Cys Asn Val Pro Gly Ser Ser Leu Val Arg
    370                 375                 380

Glu Ser Asp Leu Ala Leu Met Thr Asn Ala Gly Thr Glu Ile Gly Val
385                 390                 395                 400

Ala Ser Thr Lys Ala Phe Thr Thr Gln Leu Thr Val Leu Leu Met Leu
                405                 410                 415

Val Ala Lys Leu Ser Arg Leu Lys Gly Leu Asp Ala Ser Ile Glu His
            420                 425                 430

Asp Ile Val His Gly Leu Gln Ala Leu Pro Ser Arg Ile Glu Gln Met

435                 440                 445

Leu Ser Gln Asp Lys Arg Ile Glu Ala Leu Ala Glu Asp Phe Ser Asp
    450                 455                 460

Lys His His Ala Leu Phe Leu Ser Arg Gly Asp Gln Tyr Pro Ile Ala
465                 470                 475                 480

Leu Glu Gly Ala Leu Lys Leu Lys Glu Ile Ser Tyr Ile His Ala Glu
                485                 490                 495

Ala Tyr Ala Ala Gly Glu Leu Lys His Gly Pro Leu Ala Leu Ile Asp
            500                 505                 510

Ala Asp Met Pro Val Ile Val Val Ala Pro Asn Asn Glu Leu Leu Glu
        515                 520                 525

Lys Leu Lys Ser Asn Ile Glu Glu Val Arg Ala Arg Gly Gly Gln Leu
    530                 535                 540

Tyr Val Phe Ala Asp Gln Asp Ala Gly Phe Val Ser Ser Asp Asn Met
545                 550                 555                 560

His Ile Ile Glu Met Pro His Val Glu Glu Val Ile Ala Pro Ile Phe
                565                 570                 575

Tyr Thr Val Pro Leu Gln Leu Leu Ala Tyr His Val Ala Leu Ile Lys
            580                 585                 590

Gly Thr Asp Val Asp Gln Pro Arg Asn Leu Ala Lys Ser Val Thr Val
        595                 600                 605

Glu

<210>  20
<211>  188
<212>  PRT
<213>  Escherichia coli

<400>  20

Met Tyr Glu Arg Tyr Ala Gly Leu Ile Phe Asp Met Asp Gly Thr Ile
1               5                   10                  15

Leu Asp Thr Glu Pro Thr His Arg Lys Ala Trp Arg Glu Val Leu Gly
            20                  25                  30

His Tyr Gly Leu Gln Tyr Asp Ile Gln Ala Met Ile Ala Leu Asn Gly

35                  40                  45

Ser Pro Thr Trp Arg Ile Ala Gln Ala Ile Ile Glu Leu Asn Gln Ala
    50                  55                  60

Asp Leu Asp Pro His Ala Leu Ala Arg Glu Lys Thr Glu Ala Val Arg
65                  70                  75                  80

Ser Met Leu Leu Asp Ser Val Glu Pro Leu Pro Leu Val Asp Val Val
                85                  90                  95

Lys Ser Trp His Gly Arg Arg Pro Met Ala Val Gly Thr Gly Ser Glu
            100                 105                 110

Ser Ala Ile Ala Glu Ala Leu Leu Ala His Leu Gly Leu Arg His Tyr
        115                 120                 125

Phe Asp Ala Val Val Ala Ala Asp His Val Lys His His Lys Pro Ala
    130                 135                 140

Pro Asp Thr Phe Leu Leu Cys Ala Gln Arg Met Gly Val Gln Pro Thr
145                 150                 155                 160

Gln Cys Val Val Phe Glu Asp Ala Asp Phe Gly Ile Gln Ala Ala Arg
                165                 170                 175

Ala Ala Gly Met Asp Ala Val Asp Val Arg Leu Leu
            180                 185

<210>  21
<211>  373
<212>  PRT
<213>  Campylobacter jejuni

<400>  21

Met Val Lys Lys Ile Leu Phe Ile Thr Gly Ser Arg Ala Asp Tyr Ser
1               5                   10                  15

Lys Ile Lys Ser Leu Met Tyr Arg Val Gln Asn Ser Ser Glu Phe Glu
            20                  25                  30

Leu Tyr Ile Phe Ala Thr Gly Met His Leu Ser Lys Asn Phe Gly Tyr
        35                  40                  45

Thr Val Lys Glu Leu Tyr Lys Asn Gly Phe Lys Asn Ile Tyr Glu Phe
    50                  55                  60

Ile Asn Tyr Asp Lys Tyr Tyr Gln Thr Asp Lys Ala Leu Ala Thr Thr

65                  70                  75                  80

Ile Asp Gly Phe Ser Arg Tyr Ala Asn Glu Leu Lys Pro Asp Leu Ile
                85                  90                  95

Val Val His Gly Asp Arg Ile Glu Pro Leu Ala Ala Ala Ile Val Gly
            100                 105                 110

Ala Leu Asn Asn Ile Leu Val Ala His Ile Glu Gly Gly Glu Ile Ser
        115                 120                 125

Gly Thr Ile Asp Asp Ser Leu Arg His Ala Ile Ser Lys Leu Ala His
    130                 135                 140

Ile His Leu Val Asn Asp Glu Phe Ala Lys Arg Arg Leu Met Gln Leu
145                 150                 155                 160

Gly Glu Asp Glu Lys Ser Ile Phe Ile Ile Gly Ser Pro Asp Leu Glu
                165                 170                 175

Leu Leu Asn Asp Asn Lys Ile Ser Leu Ser Glu Ala Lys Lys Tyr Tyr
            180                 185                 190

Asp Ile Asn Tyr Glu Asn Tyr Ala Leu Leu Met Phe His Pro Val Thr
        195                 200                 205

Thr Glu Ile Thr Ser Ile Lys Asn Gln Ala Asp Asn Leu Val Lys Ala
    210                 215                 220

Leu Ile Gln Ser Asn Lys Asn Tyr Ile Val Ile Tyr Pro Asn Asn Asp
225                 230                 235                 240

Leu Gly Phe Glu Leu Ile Leu Gln Ser Tyr Glu Glu Phe Lys Asn Asn
                245                 250                 255

Pro Arg Phe Lys Leu Phe Pro Ser Leu Arg Phe Glu Tyr Phe Ile Thr
            260                 265                 270

Leu Leu Lys Asn Ala Asp Phe Ile Ile Gly Asn Ser Ser Cys Ile Leu
        275                 280                 285

Lys Glu Ala Leu Tyr Leu Lys Thr Ala Gly Ile Leu Val Gly Ser Arg
    290                 295                 300

Gln Asn Gly Arg Leu Gly Asn Glu Asn Thr Leu Lys Val Asn Ala Asn
305                 310                 315                 320

Ser Asp Glu Ile Leu Lys Ala Ile Asn Thr Ile His Lys Lys Gln Asp
                325                 330                 335

Leu Phe Ser Ala Lys Leu Glu Ile Leu Asp Ser Ser Lys Leu Phe Phe
            340                 345                 350

Glu Tyr Leu Gln Ser Gly Asp Phe Phe Lys Leu Ser Thr Gln Lys Val
        355                 360                 365

Phe Lys Asp Ile Lys
    370

<210> 22
<211> 223
<212> PRT
<213> Pasteurella multocida

<400> 22

Met Thr Asn Ile Ala Ile Ile Pro Ala Arg Ala Gly Ser Lys Gly Ile
1               5                   10                  15

Pro Asp Lys Asn Leu Gln Pro Val Gly Gly His Ser Leu Ile Gly Arg
            20                  25                  30

Ala Ile Leu Ala Ala Lys Asn Ala Asp Val Phe Asp Met Ile Val Val
        35                  40                  45

Thr Ser Asp Gly Asp Asn Ile Leu Arg Glu Ala Glu Lys Tyr Gly Ala
    50                  55                  60

Leu Ala Leu Lys Arg Pro Ala Glu Leu Ala Gln Asp Asn Ser Arg Thr
65                  70                  75                  80

Ile Asp Ala Ile Leu His Ala Leu Glu Ser Leu Asn Ile Arg Glu Gly
                85                  90                  95

Thr Cys Thr Leu Leu Gln Pro Thr Ser Pro Leu Arg Asp His Leu Asp
            100                 105                 110

Ile Lys Asn Ala Met Asp Met Tyr Val Asn Gly Gly Val His Ser Val
        115                 120                 125

Val Ser Ala Cys Glu Cys Glu His His Pro Tyr Lys Ala Phe Ala Leu
    130                 135                 140

Ser Lys Asp His Glu Val Leu Pro Val Arg Glu Ile Ala Asp Phe Glu
145                 150                 155                 160

Ala Ala Arg Gln Thr Leu Pro Lys Met Tyr Arg Ala Asn Gly Ala Ile
                165                 170                 175

Tyr Ile Asn Asp Ile Ala Gln Leu Leu Lys Glu Lys Tyr Phe Phe Ile
            180                 185                 190

Pro Pro Leu Lys Phe Tyr Leu Met Pro Thr Tyr Arg Ser Val Asp Ile
        195                 200                 205

Asp Val Lys Gln Asp Leu Glu Leu Ala Glu Ile Leu Ser Asn Lys
    210                 215                 220

<210>  23
<211>  268
<212>  PRT
<213>  Pasteurella multocida

<400>  23

Met Asp Lys Phe Ala Glu His Glu Ile Pro Lys Ala Val Ile Val Ala
1               5                   10                  15

Gly Asn Gly Glu Ser Leu Ser Gln Ile Asp Tyr Arg Leu Leu Pro Lys
            20                  25                  30

Asn Tyr Asp Val Phe Arg Cys Asn Gln Phe Tyr Phe Glu Glu Arg Tyr
        35                  40                  45

Phe Leu Gly Asn Lys Ile Lys Ala Val Phe Phe Thr Pro Gly Val Phe
    50                  55                  60

Leu Glu Gln Tyr Tyr Thr Leu Tyr His Leu Lys Arg Asn Asn Glu Tyr
65                  70                  75                  80

Phe Val Asp Asn Val Ile Leu Ser Ser Phe Asn His Pro Thr Val Asp
                85                  90                  95

Leu Glu Lys Ser Gln Lys Ile Gln Ala Leu Phe Ile Asp Val Ile Asn
            100                 105                 110

Gly Tyr Glu Lys Tyr Leu Ser Lys Leu Thr Ala Phe Asp Val Tyr Leu
        115                 120                 125

Arg Tyr Lys Glu Leu Tyr Glu Asn Gln Arg Ile Thr Ser Gly Val Tyr
    130                 135                 140

Met Cys Ala Val Ala Ile Ala Met Gly Tyr Thr Asp Ile Tyr Leu Thr
145                 150                 155                 160

Gly Ile Asp Phe Tyr Gln Ala Ser Glu Glu Asn Tyr Ala Phe Asp Asn
                165                 170                 175

Lys Lys Pro Asn Ile Ile Arg Leu Leu Pro Asp Phe Arg Lys Glu Lys
            180                 185                 190

Thr Leu Phe Ser Tyr His Ser Lys Asp Ile Asp Leu Glu Ala Leu Ser
        195                 200                 205

Phe Leu Gln Gln His Tyr His Val Asn Phe Tyr Ser Ile Ser Pro Met
    210                 215                 220

Ser Pro Leu Ser Lys His Phe Pro Ile Pro Thr Val Glu Asp Asp Cys
225                 230                 235                 240

Glu Thr Thr Phe Val Ala Pro Leu Lys Glu Asn Tyr Ile Asn Asp Ile
                245                 250                 255

Leu Leu Pro Pro His Phe Val Tyr Glu Lys Leu Gly
            260                 265

<210>  24
<211>  391
<212>  PRT
<213>  Photobacterium damselae

<400>  24

Met Thr Val Val Ala Pro Thr Leu Glu Val Tyr Ile Asp His Ala Ser
1               5                   10                  15

Leu Pro Ser Leu Gln Gln Leu Ile His Ile Ile Gln Ala Lys Asp Glu
            20                  25                  30

Tyr Pro Ser Asn Gln Arg Phe Val Ser Trp Lys Arg Val Thr Val Asp
        35                  40                  45

Ala Asp Asn Ala Asn Lys Leu Asn Ile His Thr Tyr Pro Leu Lys Gly
    50                  55                  60

Asn Asn Thr Ser Pro Glu Met Val Ala Ala Ile Asp Glu Tyr Ala Gln
65                  70                  75                  80

Ser Lys Asn Arg Leu Asn Ile Glu Phe Tyr Thr Asn Thr Ala His Val
                85                  90                  95

Phe Asn Asn Leu Pro Pro Ile Ile Gln Pro Leu Tyr Asn Asn Glu Lys
            100                 105                 110

Val Lys Ile Ser His Ile Ser Leu Tyr Asp Asp Gly Ser Ser Glu Tyr
        115                 120                 125

Val Ser Leu Tyr Gln Trp Lys Asp Thr Pro Asn Lys Ile Glu Thr Leu
    130                 135                 140

Glu Gly Glu Val Ser Leu Leu Ala Asn Tyr Leu Ala Gly Thr Ser Pro
145                 150                 155                 160

Asp Ala Pro Lys Gly Met Gly Asn Arg Tyr Asn Trp His Lys Leu Tyr
                165                 170                 175

Asp Thr Asp Tyr Tyr Phe Leu Arg Glu Asp Tyr Leu Asp Val Glu Ala
            180                 185                 190

Asn Leu His Asp Leu Arg Asp Tyr Leu Gly Ser Ser Ala Lys Gln Met
        195                 200                 205

Pro Trp Asp Glu Phe Ala Lys Leu Ser Asp Ser Gln Gln Thr Leu Phe
    210                 215                 220

Leu Asp Ile Val Gly Phe Asp Lys Glu Gln Leu Gln Gln Gln Tyr Ser
225                 230                 235                 240

Gln Ser Pro Leu Pro Asn Phe Ile Phe Thr Gly Thr Thr Thr Trp Ala
                245                 250                 255

Gly Gly Glu Thr Lys Glu Tyr Tyr Ala Gln Gln Gln Val Asn Val Ile
            260                 265                 270

Asn Asn Ala Ile Asn Glu Thr Ser Pro Tyr Tyr Leu Gly Lys Asp Tyr
        275                 280                 285

Asp Leu Phe Phe Lys Gly His Pro Ala Gly Gly Val Ile Asn Asp Ile
    290                 295                 300

Ile Leu Gly Ser Phe Pro Asp Met Ile Asn Ile Pro Ala Lys Ile Ser
305                 310                 315                 320

Phe Glu Val Leu Met Met Thr Asp Met Leu Pro Asp Thr Val Ala Gly
                325                 330                 335

Ile Ala Ser Ser Leu Tyr Phe Thr Ile Pro Ala Asp Lys Val Asn Phe
            340                 345                 350

Ile Val Phe Thr Ser Ser Asp Thr Ile Thr Asp Arg Glu Glu Ala Leu
        355                 360                 365

Lys Ser Pro Leu Val Gln Val Met Leu Thr Leu Gly Ile Val Lys Glu
    370                 375                 380

Lys Asp Val Leu Phe Trp Ala
385                 390

<210>  25
<211>  348
<212>  PRT
<213>  Neisseria meningitidis

<400>  25

Met Pro Ser Glu Ala Phe Arg Arg His Arg Ala Tyr Arg Glu Asn Lys
1               5                   10                  15

Leu Gln Pro Leu Val Ser Val Leu Ile Cys Ala Tyr Asn Val Glu Lys
            20                  25                  30

Tyr Phe Ala Gln Ser Leu Ala Ala Val Val Asn Gln Thr Trp Cys Asn
        35                  40                  45

Leu Asp Ile Leu Ile Val Asp Asp Gly Ser Thr Asp Gly Thr Leu Ala
    50                  55                  60

Ile Ala Gln Arg Phe Gln Glu Gln Asp Gly Arg Ile Lys Ile Leu Ala
65                  70                  75                  80

Gln Ala Gln Asn Ser Gly Leu Ile Pro Ser Leu Asn Ile Gly Leu Asp
                85                  90                  95

Glu Leu Ala Lys Ser Gly Met Gly Glu Tyr Ile Ala Arg Thr Asp Ala
            100                 105                 110

Asp Asp Ile Ala Ala Pro Asp Trp Ile Glu Lys Ile Val Gly Glu Met
        115                 120                 125

Glu Lys Asp Arg Ser Ile Ile Ala Met Gly Ala Trp Leu Glu Val Leu
    130                 135                 140

Ser Glu Glu Lys Asp Gly Asn Arg Leu Ala Arg His His Glu His Gly
145                 150                 155                 160

Lys Ile Trp Lys Lys Pro Thr Arg His Glu Asp Ile Ala Asp Phe Phe
                165                 170                 175

Pro Phe Gly Asn Pro Ile His Asn Asn Thr Met Ile Met Arg Arg Ser
            180                 185                 190

Val Ile Asp Gly Gly Leu Arg Tyr Asn Thr Glu Arg Asp Trp Ala Glu
        195                 200                 205

Asp Tyr Gln Phe Trp Tyr Asp Val Ser Lys Leu Gly Arg Leu Ala Tyr
    210                 215                 220

Tyr Pro Glu Ala Leu Val Lys Tyr Arg Leu His Ala Asn Gln Val Ser
225                 230                 235                 240

Ser Lys Tyr Ser Ile Arg Gln His Glu Ile Ala Gln Gly Ile Gln Lys
                245                 250                 255

Thr Ala Arg Asn Asp Phe Leu Gln Ser Met Gly Phe Lys Thr Arg Phe
            260                 265                 270

Asp Ser Leu Glu Tyr Arg Gln Ile Lys Ala Val Ala Tyr Glu Leu Leu
        275                 280                 285

Glu Lys His Leu Pro Glu Glu Asp Phe Glu Arg Ala Arg Arg Phe Leu
    290                 295                 300

Tyr Gln Cys Phe Lys Arg Thr Asp Thr Leu Pro Ala Gly Val Trp Leu
305                 310                 315                 320

Asp Phe Ala Ala Asn Gly Arg Met Arg Arg Leu Phe Thr Leu Arg Gln
                325                 330                 335

Tyr Phe Gly Ile Leu His Arg Leu Leu Lys Asn Arg
            340                 345

<210> 26
<211> 265
<212> PRT
<213> Escherichia coli O55:H7

<400> 26

Met Ile Ile Asp Glu Ala Glu Ser Ala Glu Ser Thr His Pro Val Val
1               5                   10                  15

Ser Val Ile Leu Pro Val Asn Lys Lys Asn Pro Phe Leu Asp Glu Ala
            20                  25                  30

Ile Asn Ser Ile Leu Ser Gln Thr Phe Ser Ser Phe Glu Ile Ile Ile
        35                  40                  45

Val Ala Asn Cys Cys Thr Asp Asp Phe Tyr Asn Glu Leu Lys His Lys
    50                  55                  60

Val Asn Asp Lys Ile Lys Leu Ile Arg Thr Asn Ile Ala Tyr Leu Pro
65                  70                  75                  80

Tyr Ser Leu Asn Lys Ala Ile Asp Leu Ser Asn Gly Glu Phe Ile Ala
                85                  90                  95

Arg Met Asp Ser Asp Asp Ile Ser His Pro Asp Arg Phe Thr Lys Gln
            100                 105                 110

Val Asp Phe Leu Lys Asn Asn Pro Tyr Val Asp Val Val Gly Thr Asn
        115                 120                 125

Ala Ile Phe Ile Asp Asp Lys Gly Arg Glu Ile Asn Lys Thr Lys Leu
    130                 135                 140

Pro Glu Glu Asn Leu Asp Ile Val Lys Asn Leu Pro Tyr Lys Cys Cys
145                 150                 155                 160

Ile Val His Pro Ser Val Met Phe Arg Lys Lys Val Ile Ala Ser Ile
                165                 170                 175

Gly Gly Tyr Met Phe Ser Asn Tyr Ser Glu Asp Tyr Glu Leu Trp Asn
            180                 185                 190

Arg Leu Ser Leu Ala Lys Ile Lys Phe Gln Asn Leu Pro Glu Tyr Leu
        195                 200                 205

Phe Tyr Tyr Arg Leu His Glu Gly Gln Ser Thr Ala Lys Lys Asn Leu
    210                 215                 220

Tyr Met Val Met Val Asn Asp Leu Val Ile Lys Met Lys Cys Phe Phe
225                 230                 235                 240

Leu Thr Gly Asn Ile Asn Tyr Leu Phe Gly Gly Ile Arg Thr Ile Ala
                245                 250                 255

Ser Phe Ile Tyr Cys Lys Tyr Ile Lys
            260                 265

<210>  27
<211>  275
<212>  PRT
<213>  Neisseria meningitidis MC58

<400>  27

Met Gln Asn His Val Ile Ser Leu Ala Ser Ala Ala Glu Arg Arg Ala
1               5                   10                  15

His Ile Ala Asp Thr Phe Gly Arg His Gly Ile Pro Phe Gln Phe Phe
            20                  25                  30

Asp Ala Leu Met Pro Ser Glu Arg Leu Glu Gln Ala Met Ala Glu Leu
        35                  40                  45

Val Pro Gly Leu Ser Ala His Pro Tyr Leu Ser Gly Val Glu Lys Ala
    50                  55                  60

Cys Phe Met Ser His Ala Val Leu Trp Lys Gln Ala Leu Asp Glu Gly
65                  70                  75                  80

Leu Pro Tyr Ile Thr Val Phe Glu Asp Asp Val Leu Leu Gly Glu Gly
                85                  90                  95

Ala Glu Lys Phe Leu Ala Glu Asp Ala Trp Leu Gln Glu Arg Phe Asp
            100                 105                 110

Pro Asp Thr Ala Phe Ile Val Arg Leu Glu Thr Met Phe Met His Val
        115                 120                 125

Leu Thr Ser Pro Ser Gly Val Ala Asp Tyr Cys Gly Arg Ala Phe Pro
    130                 135                 140

Leu Leu Glu Ser Glu His Trp Gly Thr Ala Gly Tyr Ile Ile Ser Arg
145                 150                 155                 160

Lys Ala Met Arg Phe Phe Leu Asp Arg Phe Ala Ala Leu Pro Pro Glu
                165                 170                 175

Gly Leu His Pro Val Asp Leu Met Met Phe Ser Asp Phe Phe Asp Arg
            180                 185                 190

Glu Gly Met Pro Val Cys Gln Leu Asn Pro Ala Leu Cys Ala Gln Glu
        195                 200                 205

Leu His Tyr Ala Lys Phe His Asp Gln Asn Ser Ala Leu Gly Ser Leu
    210                 215                 220

Ile Glu His Asp Arg Leu Leu Asn Arg Lys Gln Gln Arg Arg Asp Ser
225                 230                 235                 240

Pro Ala Asn Thr Phe Lys His Arg Leu Ile Arg Ala Leu Thr Lys Ile
                245                 250                 255

Ser Arg Glu Arg Glu Lys Arg Arg Gln Arg Arg Glu Gln Phe Ile Val
            260                 265                 270

Pro Phe Gln
        275

<210>  28
<211>  338
<212>  PRT
<213>  Escherichia coli

<400>  28

Met Arg Val Leu Val Thr Gly Gly Ser Gly Tyr Ile Gly Ser His Thr
1               5                   10                  15

Cys Val Gln Leu Leu Gln Asn Gly His Asp Val Ile Ile Leu Asp Asn
            20                  25                  30

Leu Cys Asn Ser Lys Arg Ser Val Leu Pro Val Ile Glu Arg Leu Gly
        35                  40                  45

Gly Lys His Pro Thr Phe Val Glu Gly Asp Ile Arg Asn Glu Ala Leu
    50                  55                  60

Met Thr Glu Ile Leu His Asp His Ala Ile Asp Thr Val Ile His Phe
65                  70                  75                  80

Ala Gly Leu Lys Ala Val Gly Glu Ser Val Gln Lys Pro Leu Glu Tyr
                85                  90                  95

Tyr Asp Asn Asn Val Asn Gly Thr Leu Arg Leu Ile Ser Ala Met Arg
            100                 105                 110

Ala Ala Asn Val Lys Asn Phe Ile Phe Ser Ser Ser Ala Thr Val Tyr
        115                 120                 125

Gly Asp Gln Pro Lys Ile Pro Tyr Val Glu Ser Phe Pro Thr Gly Thr
    130                 135                 140

Pro Gln Ser Pro Tyr Gly Lys Ser Lys Leu Met Val Glu Gln Ile Leu
145                 150                 155                 160

Thr Asp Leu Gln Lys Ala Gln Pro Asp Trp Ser Ile Ala Leu Leu Arg
                165                 170                 175

Tyr Phe Asn Pro Val Gly Ala His Pro Ser Gly Asp Met Gly Glu Asp
            180                 185                 190

Pro Gln Gly Ile Pro Asn Asn Leu Met Pro Tyr Ile Ala Gln Val Ala
        195                 200                 205

Val Gly Arg Arg Asp Ser Leu Ala Ile Phe Gly Asn Asp Tyr Pro Thr
    210                 215                 220

Glu Asp Gly Thr Gly Val Arg Asp Tyr Ile His Val Met Asp Leu Ala
225                 230                 235                 240

Asp Gly His Val Val Ala Met Glu Lys Leu Ala Asn Lys Pro Gly Val
                245                 250                 255

His Ile Tyr Asn Leu Gly Ala Gly Val Gly Asn Ser Val Leu Asp Val
            260                 265                 270

Val Asn Ala Phe Ser Lys Ala Cys Gly Lys Pro Val Asn Tyr His Phe
        275                 280                 285

Ala Pro Arg Arg Glu Gly Asp Leu Pro Ala Tyr Trp Ala Asp Ala Ser
    290                 295                 300

Lys Ala Asp Arg Glu Leu Asn Trp Arg Val Thr Arg Thr Leu Asp Glu
305                 310                 315                 320

Met Ala Gln Asp Thr Trp His Trp Gln Ser Arg His Pro Gln Gly Tyr
                325                 330                 335

Pro Asp

<210>  29
<211>  587
<212>  PRT
<213>  Kluyveromyces lactis

<400>  29

Met Ala Asp His Ser Ser Ser Ser Ser Ser Leu Gln Lys Lys Pro Ile
1               5                   10                  15

Asn Thr Ile Glu His Lys Asp Thr Leu Gly Asn Asp Arg Asp His Lys
            20                  25                  30

Glu Ala Leu Asn Ser Asp Asn Asp Asn Thr Ser Gly Leu Lys Ile Asn
        35                  40                  45

Gly Val Pro Ile Glu Asp Ala Arg Glu Glu Val Leu Leu Pro Gly Tyr
    50                  55                  60

Leu Ser Lys Gln Tyr Tyr Lys Leu Tyr Gly Leu Cys Phe Ile Thr Tyr
65                  70                  75                  80

Leu Cys Ala Thr Met Gln Gly Tyr Asp Gly Ala Leu Met Gly Ser Ile
                85                  90                  95

Tyr Thr Glu Asp Ala Tyr Leu Lys Tyr Tyr His Leu Asp Ile Asn Ser
            100                 105                 110

Ser Ser Gly Thr Gly Leu Val Phe Ser Ile Phe Asn Val Gly Gln Ile
        115                 120                 125

Cys Gly Ala Phe Phe Val Pro Leu Met Asp Trp Lys Gly Arg Lys Pro
    130                 135                 140

Ala Ile Leu Ile Gly Cys Leu Gly Val Val Ile Gly Ala Ile Ile Ser
145                 150                 155                 160

Ser Leu Thr Thr Thr Lys Ser Ala Leu Ile Gly Gly Arg Trp Phe Val
                165                 170                 175

Ala Phe Phe Ala Thr Ile Ala Asn Ala Ala Ala Pro Thr Tyr Cys Ala
            180                 185                 190

Glu Val Ala Pro Ala His Leu Arg Gly Lys Val Ala Gly Leu Tyr Asn
        195                 200                 205

Thr Leu Trp Ser Val Gly Ser Ile Val Ala Ala Phe Ser Thr Tyr Gly
    210                 215                 220

Thr Asn Lys Asn Phe Pro Asn Ser Ser Lys Ala Phe Lys Ile Pro Leu
225                 230                 235                 240

Tyr Leu Gln Met Met Phe Pro Gly Leu Val Cys Ile Phe Gly Trp Leu
                245                 250                 255

Ile Pro Glu Ser Pro Arg Trp Leu Val Gly Val Gly Arg Glu Glu Glu
            260                 265                 270

Ala Arg Glu Phe Ile Ile Lys Tyr His Leu Asn Gly Asp Arg Thr His
        275                 280                 285

Pro Leu Leu Asp Met Glu Met Ala Glu Ile Ile Glu Ser Phe His Gly
    290                 295                 300

Thr Asp Leu Ser Asn Pro Leu Glu Met Leu Asp Val Arg Ser Leu Phe
305                 310                 315                 320

Arg Thr Arg Ser Asp Arg Tyr Arg Ala Met Leu Val Ile Leu Met Ala

                325                 330                 335

Trp Phe Gly Gln Phe Ser Gly Asn Asn Val Cys Ser Tyr Tyr Leu Pro
            340                 345                 350

Thr Met Leu Arg Asn Val Gly Met Lys Ser Val Ser Leu Asn Val Leu
        355                 360                 365

Met Asn Gly Val Tyr Ser Ile Val Thr Trp Ile Ser Ser Ile Cys Gly
    370                 375                 380

Ala Phe Phe Ile Asp Lys Ile Gly Arg Arg Glu Gly Phe Leu Gly Ser
385                 390                 395                 400

Ile Ser Gly Ala Ala Leu Ala Leu Thr Gly Leu Ser Ile Cys Thr Ala
                405                 410                 415

Arg Tyr Glu Lys Thr Lys Lys Lys Ser Ala Ser Asn Gly Ala Leu Val
            420                 425                 430

Phe Ile Tyr Leu Phe Gly Gly Ile Phe Ser Phe Ala Phe Thr Pro Met
        435                 440                 445

Gln Ser Met Tyr Ser Thr Glu Val Ser Thr Asn Leu Thr Arg Ser Lys
    450                 455                 460

Ala Gln Leu Leu Asn Phe Val Val Ser Gly Val Ala Gln Phe Val Asn
465                 470                 475                 480

Gln Phe Ala Thr Pro Lys Ala Met Lys Asn Ile Lys Tyr Trp Phe Tyr
                485                 490                 495

Val Phe Tyr Val Phe Phe Asp Ile Phe Glu Phe Ile Val Ile Tyr Phe
            500                 505                 510

Phe Phe Val Glu Thr Lys Gly Arg Ser Leu Glu Glu Leu Glu Val Val
        515                 520                 525

Phe Glu Ala Pro Asn Pro Arg Lys Ala Ser Val Asp Gln Ala Phe Leu
    530                 535                 540

Ala Gln Val Arg Ala Thr Leu Val Gln Arg Asn Asp Val Arg Val Ala
545                 550                 555                 560

Asn Ala Gln Asn Leu Lys Glu Gln Glu Pro Leu Lys Ser Asp Ala Asp
                565                 570                 575

His Val Glu Lys Leu Ser Glu Ala Glu Ser Val
                580                 585

<210>  30
<211>  291
<212>  PRT
<213>  Bifidobacterium longum subsp. infantis ATCC 15697

<400>  30

Met Leu Phe Asn Pro Arg Asn Val Asp Val Asp Gln Trp Met Asp Ala
1               5                   10                  15

Leu Val Ala Gly Gly Met Ala Gly Val Ile Leu Thr Cys Lys His His
            20                  25                  30

Asp Gly Phe Cys Leu Trp Pro Ser Arg Leu Thr Arg His Thr Val Ala
        35                  40                  45

Ser Ser Pro Trp Arg Glu Gly Lys Gly Asp Leu Val Arg Glu Val Ser
    50                  55                  60

Glu Ser Ala Arg Arg His Gly Leu Lys Phe Gly Val Tyr Leu Ser Pro
65                  70                  75                  80

Trp Asp Arg Thr Glu Glu Ser Tyr Gly Lys Gly Lys Ala Tyr Asp Asp
                85                  90                  95

Phe Tyr Val Gly Gln Leu Thr Glu Leu Leu Thr Gln Tyr Gly Pro Ile
            100                 105                 110

Phe Ser Val Trp Leu Asp Gly Ala Asn Gly Glu Gly Lys Asn Gly Lys
        115                 120                 125

Thr Gln Tyr Tyr Asp Trp Asp Arg Tyr Tyr Asn Val Ile Arg Ser Leu
    130                 135                 140

Gln Pro Asp Ala Val Ile Ser Val Cys Gly Pro Asp Val Arg Trp Ala
145                 150                 155                 160

Gly Asn Glu Ala Gly His Val Arg Asp Asn Glu Trp Ser Val Val Pro
                165                 170                 175

Arg Arg Leu Arg Ser Ala Glu Leu Thr Met Glu Lys Ser Gln Gln Glu
            180                 185                 190

Asp Asp Ala Ser Phe Ala Thr Thr Val Ser Ser Gln Asp Asp Asp Leu
        195                 200                 205

Gly Ser Arg Glu Ala Val Ala Gly Tyr Gly Asp Asn Val Cys Trp Tyr
    210                 215                 220

Pro Ala Glu Val Asp Thr Ser Ile Arg Pro Gly Trp Phe Tyr His Gln
225                 230                 235                 240

Ser Glu Asp Asp Lys Val Met Ser Ala Asp Gln Leu Phe Asp Leu Trp
                245                 250                 255

Leu Ser Ala Val Gly Gly Asn Ser Ser Leu Leu Leu Asn Ile Pro Pro
            260                 265                 270

Ser Pro Glu Gly Leu Leu Ala Glu Pro Asp Val Gln Ser Leu Lys Gly
        275                 280                 285

Leu Gly Arg
    290

<210>  31
<211>  445
<212>  PRT
<213>  Escherichia coli

<400>  31

Met Ser Asn Arg Lys Tyr Phe Gly Thr Asp Gly Ile Arg Gly Arg Val
1               5                   10                  15

Gly Asp Ala Pro Ile Thr Pro Asp Phe Val Leu Lys Leu Gly Trp Ala
            20                  25                  30

Ala Gly Lys Val Leu Ala Arg His Gly Ser Arg Lys Ile Ile Ile Gly
        35                  40                  45

Lys Asp Thr Arg Ile Ser Gly Tyr Met Leu Glu Ser Ala Leu Glu Ala
    50                  55                  60

Gly Leu Ala Ala Ala Gly Leu Ser Ala Leu Phe Thr Gly Pro Met Pro
65                  70                  75                  80

Thr Pro Ala Val Ala Tyr Leu Thr Arg Thr Phe Arg Ala Glu Ala Gly
                85                  90                  95

Ile Val Ile Ser Ala Ser His Asn Pro Phe Tyr Asp Asn Gly Ile Lys
            100                 105                 110

Phe Phe Ser Ile Asp Gly Thr Lys Leu Pro Asp Ala Val Glu Glu Ala
        115                 120                 125

Ile Glu Ala Glu Met Glu Lys Glu Ile Ser Cys Val Asp Ser Ala Glu
    130                 135                 140

Leu Gly Lys Ala Ser Arg Ile Val Asp Ala Ala Gly Arg Tyr Ile Glu
145                 150                 155                 160

Phe Cys Lys Ala Thr Phe Pro Asn Glu Leu Ser Leu Ser Glu Leu Lys
                165                 170                 175

Ile Val Val Asp Cys Ala Asn Gly Ala Thr Tyr His Ile Ala Pro Asn
            180                 185                 190

Val Leu Arg Glu Leu Gly Ala Asn Val Ile Ala Ile Gly Cys Glu Pro
        195                 200                 205

Asn Gly Val Asn Ile Asn Ala Glu Val Gly Ala Thr Asp Val Arg Ala
    210                 215                 220

Leu Gln Ala Arg Val Leu Ala Glu Lys Ala Asp Leu Gly Ile Ala Phe
225                 230                 235                 240

Asp Gly Asp Gly Asp Arg Val Ile Met Val Asp His Glu Gly Asn Lys
                245                 250                 255

Val Asp Gly Asp Gln Ile Met Tyr Ile Ile Ala Arg Glu Gly Leu Arg
            260                 265                 270

Gln Gly Gln Leu Arg Gly Gly Ala Val Gly Thr Leu Met Ser Asn Met
        275                 280                 285

Gly Leu Glu Leu Ala Leu Lys Gln Leu Gly Ile Pro Phe Ala Arg Ala
    290                 295                 300

Lys Val Gly Asp Arg Tyr Val Leu Glu Lys Met Gln Glu Lys Gly Trp
305                 310                 315                 320

Arg Ile Gly Ala Glu Asn Ser Gly His Val Ile Leu Leu Asp Lys Thr
                325                 330                 335

Thr Thr Gly Asp Gly Ile Val Ala Gly Leu Gln Val Leu Ala Ala Met
            340                 345                 350

Ala Arg Asn His Met Ser Leu His Asp Leu Cys Ser Gly Met Lys Met
        355                 360                 365

Phe Pro Gln Ile Leu Val Asn Val Arg Tyr Thr Ala Gly Ser Gly Asp
    370                 375                 380

Pro Leu Glu His Glu Ser Val Lys Ala Val Thr Ala Glu Val Glu Ala
385                 390                 395                 400

Ala Leu Gly Asn Arg Gly Arg Val Leu Leu Arg Lys Ser Gly Thr Glu
                405                 410                 415

Pro Leu Ile Arg Val Met Val Glu Gly Glu Asp Glu Ala Gln Val Thr
            420                 425                 430

Glu Phe Ala His Arg Ile Ala Asp Ala Val Lys Ala Val
        435                 440                 445

<210>  32
<211>  456
<212>  PRT
<213>  Escherichia coli

<400>  32

Met Leu Asn Asn Ala Met Ser Val Val Ile Leu Ala Ala Gly Lys Gly
1               5                   10                  15

Thr Arg Met Tyr Ser Asp Leu Pro Lys Val Leu His Thr Leu Ala Gly
            20                  25                  30

Lys Ala Met Val Gln His Val Ile Asp Ala Ala Asn Glu Leu Gly Ala
        35                  40                  45

Ala His Val His Leu Val Tyr Gly His Gly Gly Asp Leu Leu Lys Gln
    50                  55                  60

Ala Leu Lys Asp Asp Asn Leu Asn Trp Val Leu Gln Ala Glu Gln Leu
65                  70                  75                  80

Gly Thr Gly His Ala Met Gln Gln Ala Ala Pro Phe Phe Ala Asp Asp
                85                  90                  95

Glu Asp Ile Leu Met Leu Tyr Gly Asp Val Pro Leu Ile Ser Val Glu
            100                 105                 110

Thr Leu Gln Arg Leu Arg Asp Ala Lys Pro Gln Gly Gly Ile Gly Leu
        115                 120                 125

Leu Thr Val Lys Leu Asp Asp Pro Thr Gly Tyr Gly Arg Ile Thr Arg
    130                 135                 140

Glu Asn Gly Lys Val Thr Gly Ile Val Glu His Lys Asp Ala Thr Asp
145                 150                 155                 160

Glu Gln Arg Gln Ile Gln Glu Ile Asn Thr Gly Ile Leu Ile Ala Asn
                165                 170                 175

Gly Ala Asp Met Lys Arg Trp Leu Ala Lys Leu Thr Asn Asn Asn Ala
            180                 185                 190

Gln Gly Glu Tyr Tyr Ile Thr Asp Ile Ile Ala Leu Ala Tyr Gln Glu
        195                 200                 205

Gly Arg Glu Ile Val Ala Val His Pro Gln Arg Leu Ser Glu Val Glu
    210                 215                 220

Gly Val Asn Asn Arg Leu Gln Leu Ser Arg Leu Glu Arg Val Tyr Gln
225                 230                 235                 240

Ser Glu Gln Ala Glu Lys Leu Leu Leu Ala Gly Val Met Leu Arg Asp
                245                 250                 255

Pro Ala Arg Phe Asp Leu Arg Gly Thr Leu Thr His Gly Arg Asp Val
            260                 265                 270

Glu Ile Asp Thr Asn Val Ile Ile Glu Gly Asn Val Thr Leu Gly His
        275                 280                 285

Arg Val Lys Ile Gly Thr Gly Cys Val Ile Lys Asn Ser Val Ile Gly
    290                 295                 300

Asp Asp Cys Glu Ile Ser Pro Tyr Thr Val Val Glu Asp Ala Asn Leu
305                 310                 315                 320

Ala Ala Ala Cys Thr Ile Gly Pro Phe Ala Arg Leu Arg Pro Gly Ala
                325                 330                 335

Glu Leu Leu Glu Gly Ala His Val Gly Asn Phe Val Glu Met Lys Lys
            340                 345                 350

Ala Arg Leu Gly Lys Gly Ser Lys Ala Gly His Leu Thr Tyr Leu Gly
        355                 360                 365

Asp Ala Glu Ile Gly Asp Asn Val Asn Ile Gly Ala Gly Thr Ile Thr
    370                 375                 380

Cys Asn Tyr Asp Gly Ala Asn Lys Phe Lys Thr Ile Ile Gly Asp Asp
385                 390                 395                 400

Val Phe Val Gly Ser Asp Thr Gln Leu Val Ala Pro Val Thr Val Gly
                405                 410                 415

Lys Gly Ala Thr Ile Ala Ala Gly Thr Thr Val Thr Arg Asn Val Gly
            420                 425                 430

Glu Asn Ala Leu Ala Ile Ser Arg Val Pro Gln Thr Gln Lys Glu Gly
        435                 440                 445

Trp Arg Arg Pro Val Lys Lys Lys
    450                 455

## Description

The present invention is in the technical field of synthetic biology and metabolic engineering. More particularly, the present invention is in the technical field of fermentation of metabolically engineered cells. The present invention provides a method for the production of a mixture of at least two different oligosaccharides by a cell as well as the purification of at least one of said oligosaccharides from the cultivation. In addition, the present invention provides a method for the production of a mixture of at least two different oligosaccharides by a metabolically engineered cell as well as the purification of at least one of said oligosaccharides from the cultivation.

### Background

Oligosaccharides, often present as glyco-conjugated forms to proteins and lipids, are involved in many vital phenomena such as differentiation, development and biological recognition processes related to the development and progress of fertilization, embryogenesis, inflammation, metastasis and host pathogen adhesion. Oligosaccharides can also be present as unconjugated glycans in body fluids and human milk wherein they also modulate important developmental and immunological processes (Bode, Early Hum. Dev. 1-4 (2015); Reily et al., Nat. Rev. Nephrol. 15, 346-366 (2019); Varki, Glycobiology 27, 3-49 (2017)). There is large scientific and commercial interest in oligosaccharide mixtures due to the wide functional spectrum of oligosaccharides. Yet, the availability of oligosaccharide mixtures is limited as production relies on chemical or chemo-enzymatic synthesis or on purification from natural sources such as e.g. animal milk. Chemical synthesis methods are laborious and time-consuming and because of the large number of steps involved they are difficult to scale-up. Enzymatic approaches using glycosyltransferases offer many advantages above chemical synthesis. Glycosyltransferases catalyze the transfer of a sugar moiety from an activated nucleotide-sugar donor onto saccharide or non-saccharide acceptors (Coutinho et al., J. Mol. Biol. 328 (2003) 307-317). These glycosyltransferases are the source for biotechnologists to synthesize oligosaccharides and are used both in (chemo)enzymatic approaches as well as in cell-based production systems. However, stereospecificity and regioselectivity of glycosyltransferases are still a formidable challenge. In addition, chemo-enzymatic approaches need to regenerate in situ nucleotide-sugar donors. Cellular production of oligosaccharides needs tight control of spatiotemporal availability of adequate levels of nucleotide-sugar donors in proximity of complementary glycosyltransferases. Due to these difficulties, current methods often result in the synthesis of a single oligosaccharide instead of an oligosaccharide mixture.
It is an object of the present invention to provide for tools and methods by means of which an oligosaccharide mixture comprising at least two different oligosaccharides can be produced by a cell in an efficient, time and cost-effective way and if needed, continuous process.

According to the invention, this and other objects are achieved by providing a cell and a method for the production of an oligosaccharide mixture comprising at least two different oligosaccharides wherein the cell is metabolically engineered for the production of said oligosaccharides.

### Description

Surprisingly, it has now been found that it is possible to produce oligosaccharide mixtures comprising at least two different oligosaccharides by a single cell. The present invention provides a method for the production of an oligosaccharide mixture comprising at least two different oligosaccharides. The method comprises the steps of providing a cell which expresses at least one glycosyltransferase and is capable to synthesize a nucleotide-sugar that is donor for said glycosyltransferase, wherein said cell is cultivated with at least two acceptors for said oligosaccharide production, wherein any one of said acceptors is a di- or oligosaccharide. The present invention also provides methods to separate at least one of said produced oligosaccharides from the oligosaccharide mixture. Furthermore, the present invention provides a metabolically engineered cell for production of an oligosaccharide mixture comprising at least two different oligosaccharides.

### Definitions

The words used in this specification to describe the invention and its various embodiments are to be understood not only in the sense of their commonly defined meanings, but to include by special definition in this specification structure, material or acts beyond the scope of the commonly defined meanings. Thus, if an element can be understood in the context of this specification as including more than one meaning, then its use in a claim must be understood as being generic to all possible meanings supported by the specification and by the word itself.
The various embodiments and aspects of embodiments of the invention disclosed herein are to be understood not only in the order and context specifically described in this specification, but to include any order and any combination thereof. Whenever the context requires, all words used in the singular number shall be deemed to include the plural and vice versa. Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization described herein are those well-known and commonly employed in the art. Standard techniques are used for nucleic acid and peptide synthesis. Generally, purification steps are performed according to the manufacturer's specifications.
In the specification, there have been disclosed embodiments of the invention, and although specific terms are employed, the terms are used in a descriptive sense only and not for purposes of limitation, the scope of the invention being set forth in the following claims. It must be understood that the illustrated embodiments have been set forth only for the purposes of example and that it should not be taken as limiting the invention. It will be apparent to those skilled in the art that alterations, other embodiments, improvements, details and uses can be made consistent with the letter and spirit of the disclosure herein and within the scope of this disclosure, which is limited only by the claims, construed in accordance with the patent law, including the doctrine of equivalents. In the claims which follow, reference characters used to designate claim steps are provided for convenience of description only, and are not intended to imply any particular order for performing the steps.
According to the present invention, the term "polynucleotide(s)" generally refers to any polyribonucleotide or polydeoxyribonucleotide, which may be unmodified RNA or DNA or modified RNA or DNA. "Polynucleotide(s)" include, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. In addition, "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term "polynucleotide(s)" also includes DNAs or RNAs as described above that contain one or more modified bases. Thus, DNAs or RNAs with backbones modified for stability or for other reasons are "polynucleotide(s)" according to the present invention. Moreover, DNAs or RNAs comprising unusual bases, such as inosine, or modified bases, such as tritylated bases, are to be understood to be covered by the term "polynucleotides". It will be appreciated that a great variety of modifications have been made to DNA and RNA that serve many useful purposes known to those of skill in the art. The term "polynucleotide(s)" as it is employed herein embraces such chemically, enzymatically or metabolically modified forms of polynucleotides, as well as the chemical forms of DNA and RNA characteristic of viruses and cells, including, for example, simple and complex cells. The term "polynucleotide(s)" also embraces short polynucleotides often referred to as oligonucleotide(s).
"Polypeptide(s)" refers to any peptide or protein comprising two or more amino acids joined to each other by peptide bonds or modified peptide bonds. "Polypeptide(s)" refers to both short chains, commonly referred to as peptides, oligopeptides and oligomers and to longer chains generally referred to as proteins. Polypeptides may contain amino acids other than the 20 gene encoded amino acids. "Polypeptide(s)" include those modified either by natural processes, such as processing and other post-translational modifications, but also by chemical modification techniques. Such modifications are well described in basic texts and in more detailed monographs, as well as in a voluminous research literature, and they are well known to the skilled person. The same type of modification may be present in the same or varying degree at several sites in a given polypeptide. Furthermore, a given polypeptide may contain many types of modifications. Modifications can occur anywhere in a polypeptide, including the peptide backbone, the amino acid sidechains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, lipid attachment, sulfation, gamma-carboxylation of glutamic acid residues, hydroxylation and ADP-ribosylation, selenoylation, transfer-RNA mediated addition of amino acids to proteins, such as arginylation, and ubiquitination. Polypeptides may be branched or cyclic, with or without branching. Cyclic, branched and branched circular polypeptides may result from post-translational natural processes and may be made by entirely synthetic methods, as well.
The term "polynucleotide encoding a polypeptide" as used herein encompasses polynucleotides that include a sequence encoding a polypeptide of the invention. The term also encompasses polynucleotides that include a single continuous region or discontinuous regions encoding the polypeptide (for example, interrupted by integrated phage or an insertion sequence or editing) together with additional regions that also may contain coding and/or non-coding sequences.
"Isolated" means altered "by the hand of man" from its natural state, i.e., if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Similarly, a "synthetic" sequence, as the term is used herein, means any sequence that has been generated synthetically and not directly isolated from a natural source. "Synthesized", as the term is used herein, means any synthetically generated sequence and not directly isolated from a natural source.
The terms "recombinant" or "transgenic" or "metabolically engineered" or "genetically modified", as used herein with reference to a cell or host cell are used interchangeably and indicates that the cell replicates a heterologous nucleic acid, or expresses a peptide or protein encoded by a heterologous nucleic acid (i.e., a sequence "foreign to said cell" or a sequence "foreign to said location or environment in said cell"). Such cells are described to be transformed with at least one heterologous or exogenous gene, or are described to be transformed by the introduction of at least one heterologous or exogenous gene. Metabolically engineered or recombinant or transgenic cells can contain genes that are not found within the native (non-recombinant) form of the cell. Recombinant cells can also contain genes found in the native form of the cell wherein the genes are modified and re-introduced into the cell by artificial means. The terms also encompass cells that contain a nucleic acid endogenous to the cell that has been modified or its expression or activity has been modified without removing the nucleic acid from the cell; such modifications include those obtained by gene replacement, replacement of a promoter; site-specific mutation; and related techniques. Accordingly, a "recombinant polypeptide" is one which has been produced by a recombinant cell. A "heterologous sequence" or a "heterologous nucleic acid", as used herein, is one that originates from a source foreign to the particular cell (e.g. from a different species), or, if from the same source, is modified from its original form or place in the genome. Thus, a heterologous nucleic acid operably linked to a promoter is from a source different from that from which the promoter was derived, or, if from the same source, is modified from its original form or place in the genome. The heterologous sequence may be stably introduced, e.g. by transfection, transformation, conjugation or transduction, into the genome of the host microorganism cell, wherein techniques may be applied which will depend on the cell and the sequence that is to be introduced. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989). The term "mutant" cell or microorganism as used within the context of the present disclosure refers to a cell or microorganism which is genetically modified.
The term "endogenous," within the context of the present disclosure refers to any polynucleotide, polypeptide or protein sequence which is a natural part of a cell and is occurring at its natural location in the cell chromosome. The term "exogenous" refers to any polynucleotide, polypeptide or protein sequence which originates from outside the cell under study and not a natural part of the cell or which is not occurring at its natural location in the cell chromosome or plasmid.
The term "heterologous" when used in reference to a polynucleotide, gene, nucleic acid, polypeptide, or enzyme refers to a polynucleotide, gene, nucleic acid, polypeptide, or enzyme that is from a source or derived from a source other than the host organism species. In contrast a "homologous" polynucleotide, gene, nucleic acid, polypeptide, or enzyme is used herein to denote a polynucleotide, gene, nucleic acid, polypeptide, or enzyme that is derived from the host organism species. When referring to a gene regulatory sequence or to an auxiliary nucleic acid sequence used for maintaining or manipulating a gene sequence (e.g. a promoter, a 5' untranslated region, 3' untranslated region, poly A addition sequence, intron sequence, splice site, ribosome binding site, internal ribosome entry sequence, genome homology region, recombination site, etc.), "heterologous" means that the regulatory sequence or auxiliary sequence is not naturally associated with the gene with which the regulatory or auxiliary nucleic acid sequence is juxtaposed in a construct, genome, chromosome, or episome. Thus, a promoter operably linked to a gene to which it is not operably linked to in its natural state (i.e. in the genome of a non-genetically engineered organism) is referred to herein as a "heterologous promoter," even though the promoter may be derived from the same species (or, in some cases, the same organism) as the gene to which it is linked.
The term "modified activity" of a protein or an enzyme relates to a change in activity of the protein or the enzyme compared to the wild type, i.e. natural, activity of said protein or enzyme. Said modified activity can either be an abolished, impaired, reduced or delayed activity of said protein or enzyme compared to the wild type activity of the protein or the enzyme but can also be an accelerated or an enhanced activity of said protein or the enzyme compared to the wild type activity of the protein or the enzyme. A modified activity of a protein or an enzyme is obtained by modified expression of said protein or enzyme or is obtained by expression of a modified, i.e. mutant form of the protein or enzyme. A modified activity of an enzyme further relates to a modification in the apparent Michaelis constant Km and/or the apparent maximal velocity (Vmax) of the enzyme.
The term "modified expression" of a gene relates to a change in expression compared to the wild type expression of said gene in any phase of the production process of the encoded protein. Said modified expression is either a lower or higher expression compared to the wild type, wherein the term "higher expression" is also defined as "overexpression" of said gene in the case of an endogenous gene or "expression" in the case of a heterologous gene that is not present in the wild type strain. Lower expression or reduced expression is obtained by means of common well-known technologies for a skilled person (such as the usage of siRNA, CrispR, CrispRi, riboswitches, recombineering, homologous recombination, ssDNA mutagenesis, RNAi, miRNA, asRNA, mutating genes, knocking-out genes, transposon mutagenesis, ...) which are used to change the genes in such a way that they are less-able (i.e. statistically significantly 'less-able' compared to a functional wild-type gene) or completely unable (such as knocked-out genes) to produce functional final products. Next to changing the gene of interest in such a way that lower expression is obtained as described above, lower expression can also be obtained by changing the transcription unit, the promoter, an untranslated region, the ribosome binding site, the Shine Dalgarno sequence or the transcription terminator. Lower expression or reduced expression can for instance be obtained by mutating one or more base pairs in the promoter sequence or changing the promoter sequence fully to a constitutive promoter with a lower expression strength compared to the wild type or an inducible promoter which result in regulated expression or a repressible promoter which results in regulated expression Overexpression or expression is obtained by means of common well-known technologies for a skilled person, wherein said gene is part of an "expression cassette" which relates to any sequence in which a promoter sequence, untranslated region sequence (containing either a ribozyme binding sequence or Shine Dalgarno sequence), a coding sequence and optionally a transcription terminator is present, and leading to the expression of a functional active protein. Said expression is either constitutive or regulated.
The term "constitutive expression" is defined as expression that is not regulated by transcription factors other than the subunits of RNA polymerase (e.g. the bacterial sigma factors) under certain growth conditions. Non-limiting examples of such transcription factors are CRP, LacI, ArcA, Cra, IclR in E. coli. These transcription factors bind on a specific sequence and may block or enhance expression in certain growth conditions. RNA polymerase binds a specific sequence to initiate transcription, for instance via a sigma factor in prokaryotic hosts.
The term "expression by a natural inducer" is defined as a facultative or regulatory expression of a gene that is only expressed upon a certain natural condition of the host (e.g. organism being in labor, or during lactation), as a response to an environmental change (e.g. including but not limited to hormone, heat, cold, light, oxidative or osmotic stress / signaling), or dependent on the position of the developmental stage or the cell cycle of said host cell including but not limited to apoptosis and autophagy.
The term "control sequences" refers to sequences recognized by the cells transcriptional and translational systems, allowing transcription and translation of a polynucleotide sequence to a polypeptide. Such DNA sequences are thus necessary for the expression of an operably linked coding sequence in a particular cell or organism. Such control sequences can be, but are not limited to, promoter sequences, ribosome binding sequences, Shine Dalgarno sequences, Kozak sequences, transcription terminator sequences. The control sequences that are suitable for prokaryotes, for example, include a promoter, optionally an operator sequence, and a ribosome binding site. Eukaryotic cells are known to utilize promoters, polyadenylation signals, and enhancers. DNA for a presequence or secretory leader may be operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it affects the transcription of the sequence; or a ribosome binding site is operably linked to a coding sequence if it is positioned so as to facilitate translation. Said control sequences can furthermore be controlled with external chemicals, such as, but not limited to, IPTG, arabinose, lactose, allo-lactose, rhamnose or fucose via an inducible promoter or via a genetic circuit that either induces or represses the transcription or translation of said polynucleotide to a polypeptide.
Generally, "operably linked" means that the DNA sequences being linked are contiguous, and, in the case of a secretory leader, contiguous and in reading phase. However, enhancers do not have to be contiguous. The term "glycosyltransferase" as used herein refers to an enzyme capable to catalyze the transfer of sugar moieties from activated donor molecules to specific acceptor molecules, forming glycosidic bonds. The as such synthesized oligosaccharides can be of the linear type or of the branched type and can contain multiple monosaccharide building blocks. A classification of glycosyltransferases using nucleotide diphospho-sugar, nucleotide monophospho-sugar and sugar phosphates and related proteins into distinct sequence-based families has been described (Campbell et al., Biochem. J. 326, 929-939 (1997)) and is available on the CAZy (CArbohydrate-Active EnZymes) website (www.cazy.org).
As used herein the glycosyltransferase can be selected from the list comprising but not limited to: fucosyltransferases, sialyltransferases, galactosyltransferases, N-acetylglucosaminyltransferases, N-acetylgalactosaminyltransferases, glucosyltransferases, mannosyltransferases, N-acetylmannosaminyltransferases, xylosyltransferases, glucuronyltransferases, galacturonyl transferases, glucosaminyltransferases, N-glycolylneuraminyltransferases, rhamnosyltransferases. Fucosyltransferases are glycosyltransferases that transfer a fucose residue (Fuc) from a GDP-fucose (GDP-Fuc) donor onto a glycan acceptor. Fucosyltransferases comprise alpha-1,2-fucosyltransferases, alpha-1,3-fucosyltransferases, alpha-1,4-fucosyltransferases and alpha-1,6-fucosyltransferases that catalyze the transfer of a Fuc residue from GDP-Fuc onto a glycan acceptor via alpha-glycosidic bonds. Fucosyltransferases can be found but are not limited to the GT10, GT11, GT23, GT65 and GT68 CAZy families. Sialyltransferases are glycosyltransferases that transfer a sialyl group (like Neu5Ac or Neu5Gc) from a donor (like CMP-Neu5Ac or CMP-Neu5Gc) onto a glycan acceptor. Sialyltransferases comprise alpha-2,3-sialyltransferases and alpha-2,6-sialyltransferases that catalyze the transfer of a sialyl group onto a glycan acceptor via alpha-glycosidic bonds. Sialyltransferases can be found but are not limited to the GT29, GT42, GT80 and GT97 CAZy families. Galactosyltransferases are glycosyltransferases that transfer a galactosyl group (Gal) from an UDP-galactose (UDP-Gal) donor onto a glycan acceptor. Galactosyltransferases comprise beta-1,3-galactosyltransferases, beta-1,4-galactosyltransferases, alpha-1,3-galactosyltransferases and alpha-1,4-galactosyltransferases that transfer a Gal residue from UDP-Gal onto a glycan acceptor via alpha- or beta-glycosidic bonds. Galactosyltransferases can be found but are not limited to the GT2, GT6, GT8, GT25 and GT92 CAZy families. Glucosyltransferases are glycosyltransferases that transfer a glucosyl group (Glc) from an UDP-glucose (UDP-Glc) donor onto a glycan acceptor. Glucosyltransferases comprise alpha-glucosyltransferases, beta-1,2-glucosyltransferases, beta-1,3-glucosyltransferases and beta-1,4-glucosyltransferases that transfer a Glc residue from UDP-Glc onto a glycan acceptor via alpha- or beta-glycosidic bonds. Glucosyltransferases can be found but are not limited to the GT1, GT4 and GT25 CAZy families. Mannosyltransferases are glycosyltransferases that transfer a mannose group (Man) from a GDP-mannose (GDP-Man) donor onto a glycan acceptor. Mannosyltransferases comprise alpha-1,2-mannosyltransferases, alpha-1,3-mannosyltransferases and alpha-1,6-mannosyltransferases that transfer a Man residue from GDP-Man onto a glycan acceptor via alpha-glycosidic bonds. Mannosyltransferases can be found but are not limited to the GT22, GT39, GT62 and GT69 CAZy families. N-acetylglucosaminyltransferases are glycosyltransferases that transfer an N-acetylglucosamine group (GlcNAc) from an UDP-N-acetylglucosamine (UDP-GlcNAc) donor onto a glycan acceptor. N-acetylglucosaminyltransferases can be found but are not limited to GT2 and GT4 CAZy families.

N-acetylgalactosaminyltransferases are glycosyltransferases that transfer an N-acetylgalactosamine group (GalNAc) from an UDP-N-acetylgalactosamine (UDP-GalNAc) donor onto a glycan acceptor. N-acetylgalactosaminyltransferases can be found but are not limited to GT7, GT12 and GT27 CAZy families. N-acetylmannosaminyltransferases are glycosyltransferases that transfer an N-acetylmannosamine group (ManNAc) from an UDP-N-acetylmannosamine (UDP-ManNAc) donor onto a glycan acceptor. Xylosyltransferases are glycosyltransferases that transfer a xylose residue (Xyl) from an UDP-xylose (UDP-Xyl) donor onto a glycan acceptor. Xylosyltransferases can be found but are not limited to GT61 and GT77 CAZy families. Glucuronyltransferases are glycosyltransferases that transfer a glucuronate from an UDP-glucuronate donor onto a glycan acceptor via alpha- or beta-glycosidic bonds. Glucuronyltransferases can be found but are not limited to GT4, GT43 and GT93 CAZy families.
Galacturonyltransferases are glycosyltransferases that transfer a galacturonate from an UDP-galacturonate donor onto a glycan acceptor. N-glycolylneuraminyltransferases are glycosyltransferases that transfer an N-glycolylneuraminic acid group (Neu5Gc) from a CMP-Neu5Gc donor onto a glycan acceptor. Rhamnosyltransferases are glycosyltransferases that transfer a rhamnose residue from a GDP-rhamnose donor onto a glycan acceptor. Rhamnosyltransferases can be found but are not limited to the GT1, GT2 and GT102 CAZy families.
The terms "nucleotide-sugar" or "activated sugar" as used herein refer to activated forms of monosaccharides. Examples of activated monosaccharides include but are not limited to UDP-galactose (UDP-Gal), UDP-N-acetylglucosamine (UDP-GlcNAc), UDP-N-acetylgalactosamine (UDP-GalNAc), UDP-N-acetylmannosamine (UDP-ManNAc), GDP-fucose (GDP-Fuc), GDP-mannose (GDP-Man), UDP-glucose (UDP-Glc), CMP-N-acetylneuraminic acid (CMP-Neu5Ac), CMP-N-glycolylneuraminic acid (CMP-Neu5Gc), UDP-glucuronate, GDP-rhamnose, or UDP-xylose. Nucleotide-sugars act as glycosyl donors in glycosylation reactions. Those reactions are catalyzed by glycosyltransferases.
"Oligosaccharide" as the term is used herein and as generally understood in the state of the art, refers to a saccharide polymer containing a small number, typically three to twenty, of simple sugars, i.e. monosaccharides. The monosaccharides as used herein are reducing sugars. The oligosaccharides can be reducing or non-reducing sugars and have a reducing and a non-reducing end. A reducing sugar is any sugar that is capable of reducing another compound and is oxidized itself, that is, the carbonyl carbon of the sugar is oxidized to a carboxyl group. The oligosaccharide as used in the present invention can be a linear structure or can include branches. The linkage (e.g. glycosidic linkage, galactosidic linkage, glucosidic linkage, etc.) between two sugar units can be expressed, for example, as 1,4, 1->4, or (1-4), used interchangeably herein. Each monosaccharide can be in the cyclic form (e.g. pyranose of furanose form). Linkages between the individual monosaccharide units may include alpha 1->2, alpha 1->3, alpha 1->4, alpha 1->6, alpha 2->1, alpha 2->3, alpha 2->4, alpha 2->6, beta 1->2, beta 1->3, beta 1->4, beta 1->6, beta 2->1, beta 2->3, beta 2->4, and beta 2->6. An oligosaccharide can contain both alpha- and beta-glycosidic bonds or can contain only beta-glycosidic bonds.
"Disaccharide" as the term is used herein and as generally understood in the state of the art, refers to a saccharide polymer containing two monosaccharides. Examples of disaccharides comprise lactose (Gal-b1,4-Glc), lacto-N-biose (Gal-b1,3-GlcNAc), N-acetyllactosamine (Gal-bl,4-GlcNAc), LacDiNAc (GalNAc-bl,4-GlcNAc), N-acetylgalactosaminylglucose (GalNAc-b1,4-Glc).
The term "monosaccharide" as used herein refers to a sugar that is not decomposable into simpler sugars by hydrolysis, is classed either an aldose or ketose, and contains one or more hydroxyl groups per molecule. Monosaccharides are saccharides containing only one simple sugar. Examples of monosaccharides comprise Hexose, D-Glucopyranose, D-Galactofuranose, D-Galactopyranose, L-Galactopyranose, D-Mannopyranose, D-Allopyranose, L-Altropyranose, D-Gulopyranose, L-Idopyranose, D-Talopyranose, D-Ribofuranose, D-Ribopyranose, D-Arabinofuranose, D-Arabinopyranose, L-Arabinofuranose, L-Arabinopyranose, D-Xylopyranose, D-Lyxopyranose, D-Erythrofuranose, D-Threofuranose, Heptose, L-glycero-D-manno-Heptopyranose (LDmanHep), D-glycero-D-manno-Heptopyranose (DDmanHep), 6-Deoxy-L-altropyranose, 6-Deoxy-D-gulopyranose, 6-Deoxy-D-talopyranose, 6-Deoxy-D-galactopyranose, 6-Deoxy-L-galactopyranose, 6-Deoxy-D-mannopyranose, 6-Deoxy-L-mannopyranose, 6-Deoxy-D-glucopyranose, 2-Deoxy-D-arabino-hexose, 2-Deoxy-D-erythro-pentose, 2,6-Dideoxy-D-arabino-hexopyranose, 3,6-Dideoxy-D-arabino-hexopyranose, 3,6-Dideoxy-L-arabino-hexopyranose, 3,6-Dideoxy-D-xylo-hexopyranose, 3,6-Dideoxy-D-ribo-hexopyranose, 2,6-Dideoxy-D-ribo-hexopyranose, 3,6-Dideoxy-L-xylo-hexopyranose, 2-Amino-2-deoxy-D-glucopyranose, 2-Amino-2-deoxy-D-galactopyranose, 2-Amino-2-deoxy-D-mannopyranose, 2-Amino-2-deoxy-D-allopyranose, 2-Amino-2-deoxy-L-altropyranose, 2-Amino-2-deoxy-D-gulopyranose, 2-Amino-2-deoxy-L-idopyranose, 2-Amino-2-deoxy-D-talopyranose, 2-Acetamido-2-deoxy-D-glucopyranose, 2-Acetamido-2-deoxy-D-galactopyranose, 2-Acetamido-2-deoxy-D-mannopyranose, 2-Acetamido-2-deoxy-D-allopyranose, 2-Acetamido-2-deoxy-L-altropyranose, 2-Acetamido-2-deoxy-D-gulopyranose, 2-Acetamido-2-deoxy-L-idopyranose, 2-Acetamido-2-deoxy-D-talopyranose, 2-Acetamido-2,6-dideoxy-D-galactopyranose, 2-Acetamido-2,6-dideoxy-L-galactopyranose, 2-Acetamido-2,6-dideoxy-L-mannopyranose, 2-Acetamido-2,6-dideoxy-D-glucopyranose, 2-Acetamido-2,6-dideoxy-L-altropyranose, 2-Acetamido-2,6-dideoxy-D-talopyranose, D-Glucopyranuronic acid, D-Galactopyranuronic acid, D-Mannopyranuronic acid, D-Allopyranuronic acid, L-Altropyranuronic acid, D-Gulopyranuronic acid, L-Gulopyranuronic acid, L-Idopyranuronic acid, D-Talopyranuronic acid, Sialic acid, 5-Amino-3,5-dideoxy-D-glycero-D-galacto-non-2-ulosonic acid, 5-Acetamido-3,5-dideoxy-D-glycero-D-galacto-non-2-ulosonic acid, 5-Glycolylamido-3,5-dideoxy-D-glycero-D-galacto-non-2-ulosonic acid, Erythritol, Arabinitol, Xylitol, Ribitol, Glucitol, Galactitol, Mannitol, D-ribo-Hex-2-ulopyranose, D-arabino-Hex-2-ulofuranose (D-fructofuranose), D-arabino-Hex-2-ulopyranose, L-xylo-Hex-2-ulopyranose, D-lyxo-Hex-2-ulopyranose, D-threo-Pent-2-ulopyranose, D-altro-Hept-2-ulopyranose, 3-C-(Hydroxymethyl)-D-erythofuranose, 2,4,6-Trideoxy-2,4-diamino-D-glucopyranose, 6-Deoxy-3-O-methyl-D-glucose, 3-O-Methyl-D-rhamnose, 2,6-Dideoxy-3-methyl-D-ribo-hexose, 2-Amino-3-O-[(R)-1-carboxyethyl]-2-deoxy-D-glucopyranose, 2-Acetamido-3-O-[(R)-carboxyethyl]-2-deoxy-D-glucopyranose, 2-Glycolylamido-3-O-[(R)-1-carboxyethyl]-2-deoxy-D-glucopyranose, 3-Deoxy-D-lyxo-hept-2-ulopyranosaric acid, 3-Deoxy-D-manno-oct-2-ulopyranosonic acid, 3-Deoxy-D-glycero-D-galacto-non-2-ulopyranosonic acid, 5,7-Diamino-3,5,7,9-tetradeoxy-L-glycero-L-manno-non-2-ulopyranosonic acid, 5,7-Diamino-3,5,7,9-tetradeoxy-L-glycero-L-altro-non-2-ulopyranosonic acid, 5,7-Diamino-3,5,7,9-tetradeoxy-D-glycero-D-galacto-non-2-ulopyranosonic acid, 5,7-Diamino-3,5,7,9-tetradeoxy-D-glycero-D-talo-non-2-ulopyranosonic acid, glucose, galactose, N-acetylglucosamine, glucosamine, mannose, xylose, N-acetylmannosamine, N-acetylneuraminic acid, N-glycolylneuraminic acid, N-acetylgalactosamine, galactosamine, fucose, rhamnose, glucuronic acid, gluconic acid, fructose and polyols.
Oligosaccharides are glycan structures that are composed of three or more monosaccharide subunits that are linked to each other via glycosidic bonds in a linear or in a branched structure. Preferably the oligosaccharide as described herein contains monosaccharides selected from the list as used herein above. Examples of oligosaccharides include but are not limited to Lewis-type antigen oligosaccharides, mammalian milk oligosaccharides and human milk oligosaccharides.
As used herein, "mammalian milk oligosaccharide" refers to oligosaccharides such as but not limited to 3-fucosyllactose, 2'-fucosyllactose, 6-fucosyllactose, 2',3-difucosyllactose, 2',2-difucosyllactose, 3,4-difucosyllactose, 6'-sialyllactose, 3'-sialyllactose, 3,6-disialyllactose, 6,6'-disialyllactose, 3,6-disialyllacto-N-tetraose, lactodifucotetraose, lacto-N-tetraose, lacto-N-neotetraose, lacto-N-fucopentaose II, lacto-N-fucopentaose I, lacto-N-fucopentaose III, lacto-N-fucopentaose V, lacto-N-fucopentaose VI, sialyllacto-N-neotetraose c, sialyllacto-N-tetraose b, sialyllacto-N-tetraose a, lacto-N-difucohexaose I, lacto-N-difucohexaose II, lacto-N-hexaose, lacto-N-neohexaose, para-lacto-N-hexaose, monofucosylmonosialyllacto-N-neotetraose c, monofucosyl para-lacto-N-hexaose, monofucosyllacto-N-hexaose III, isomeric fucosylated lacto-N-hexaose III, isomeric fucosylated lacto-N-hexaose I, sialyllacto-N-hexaose, sialyllacto-N-neohexaose II, difucosyl-para-lacto-N-hexaose, difucosyllacto-N-hexaose, difucosyllacto-N-hexaose a, difucosyllacto-N-hexaose c, galactosylated chitosan, fucosylated oligosaccharides, neutral oligosaccharide and/or sialylated oligosaccharides.
As used herein the term "Lewis-type antigens" comprise the following oligosaccharides: H1 antigen, which is Fucα1-2Galβ-3GlcNAc, or in short 2'FLNB; Lewisa, which is the trisaccharide Galβ1-3[Fucα1-4]GlcNAc, or in short 4-FLNB; Lewisb, which is the tetrasaccharide Fucα1-2Galβ1-3[Fucα1-4]GlcNAc, or in short DiFLNB; sialyl Lewisa which is 5-acetylneuraminyl-(2-3)-galactosyl-(1-3)-(fucopyranosyl-(1-4))-N-acetylglucosamine, or written in short Neu5Acα2-3Galβ1-3[Fucα1-4]GlcNAc; H2 antigen, which is Fucα1-2Galβ1-4GlcNAc, or otherwise stated 2'fucosyl-N-acetyl-lactosamine, in short 2'FLacNAc; Lewisx, which is the trisaccharide Galβ1-4[Fucα1-3]GlcNAc, or otherwise known as 3-Fucosyl-N-acetyl-lactosamine, in short 3-FLacNAc, Lewisy, which is the tetrasaccharide Fucα1-2Galβ1-4[Fucα1-3]GlcNAc and sialyl Lewisx which is 5-acetylneuraminyl-(2-3)-galactosyl-(1-4)-(fucopyranosyl-(1-3))-N-acetylglucosamine, or written in short Neu5Acα2-3Galβ1-4[Fucα1-3]GlcNAc.
A 'fucosylated oligosaccharide' as used herein and as generally understood in the state of the art is an oligosaccharide that is carrying a fucose-residue. Such fucosylated oligosaccharide is a saccharide structure comprising at least three monosaccharide subunits linked to each other via glycosidic bonds, wherein at least one of said monosaccharide subunit is a fucose. A fucosylated oligosaccharide can contain more than one fucose residue, e.g. two, three or more. A fucosylated oligosaccharide can be a neutral oligosaccharide or a charged oligosaccharide e.g. also comprising sialic acid structures. Fucose can be linked to other monosaccharide subunits comprising glucose, galactose, GlcNAc via alpha-glycosidic bonds comprising alpha-1,2 alpha-1,3, alpha-1,4, alpha-1,6 linkages.
Examples comprise 2'-fucosyllactose (2'FL), 3-fucosyllactose (3FL), 4-fucosyllactose (4FL), 6-fucosyllactose (6FL), difucosyllactose (diFL), lactodifucotetraose (LDFT), Lacto-N-fucopentaose I (LNFP I), Lacto-N-fucopentaose II (LNFP II), Lacto-N-fucopentaose III (LNFP III), lacto-N-fucopentaose V (LNFP V), lacto-N-fucopentaose VI (LNFP VI), lacto-N-neofucopentaose I, lacto-N-difucohexaose I (LDFH I), lacto-N-difucohexaose II (LDFH II), Monofucosyllacto-N-hexaose III (MFLNH III), Difucosyllacto-N-hexaose (DFLNHa), difucosyl-lacto-N-neohexaose.
The terms "alpha-1,2-fucosyltranferase", "alpha 1,2 fucosyltransferase", "2-fucosyltransferase, "α-1,2-fucosyltransferase", "α 1,2 fucosyltransferase", "2 fucosyltransferase, "2-FT" or "2FT" as used in the present invention, are used interchangeably and refer to a glycosyltransferase that catalyzes the transfer of fucose from the donor GDP-L-fucose, to the acceptor molecule in an alpha-1,2-linkage. The terms "2' fucosyllactose", "2'-fucosyllactose", "alpha-1,2-fucosyllactose", "alpha 1,2 fucosyllactose", "α-1,2-fucosyllactose", "α 1,2 fucosyllactose", "Galβ-4(Fucα1-2)Glc", 2FL" or "2'FL" as used in the present invention, are used interchangeably and refer to the product obtained by the catalysis of the alpha-1,2-fucosyltransferase transferring the fucose residue from GDP-L-fucose to lactose in an alpha-1,2-linkage. The terms "difucosyllactose", "di-fucosyllactose", "lactodifucotetraose", "2',3-difucosyllactose", "2',3 difucosyllactose", "α-2',3-fucosyllactose", "α 2',3 fucosyllactose, "Fucα1-2Galβ 1-4(Fucα1-3)Glc", "DFLac", 2',3 diFL", "DFL", "DiFL" or "diFL" as used in the present invention, are used interchangeably. The terms "alpha-1,3-fucosyltranferase", "alpha 1,3 fucosyltransferase", "3-fucosyltransferase, "α-1,3-fucosyltransferase", "α 1,3 fucosyltransferase", "3 fucosyltransferase, "3-FT" or "3FT" as used in the present invention, are used interchangeably and refer to a glycosyltransferase that catalyzes the transfer of fucose from the donor GDP-L-fucose, to the acceptor molecule in an alpha-1,3-linkage. The terms "3-fucosyllactose", "alpha-1,3-fucosyllactose", "alpha 1,3 fucosyllactose", "α-1,3-fucosyllactose", "α 1,3 fucosyllactose", "Galβ-4(Fucα1-3)Glc", 3FL" or "3-FL" as used in the present invention, are used interchangeably and refer to the product obtained by the catalysis of the alpha-1,3-fucosyltransferase transferring the fucose residue from GDP-L-fucose to lactose in an alpha-1,3-linkage.
As used herein, a 'sialylated oligosaccharide' is to be understood as a charged sialic acid containing oligosaccharide, i.e. an oligosaccharide having a sialic acid residue. It has an acidic nature. A sialylated oligosaccharide contains at least one sialic acid monosaccharide subunit, like e.g. but not limited to Neu5Ac and Neu5Gc. Said sialylated oligosaccharide is a saccharide structure comprising at least three monosaccharide subunits linked to each other via glycosidic bonds, wherein at least one of said monosaccharide subunit is a sialic acid. Said sialylated oligosaccharide can contain more than one sialic acid residue, e.g. two, three or more. Said sialic acid can be linked to other monosaccharide subunits comprising galactose, GlcNAc, sialic acid, via alpha-glycosidic bonds comprising alpha-2,3, alpha-2,6 linkages. Some examples are 3-SL (3'-sialyllactose), 3'-sialyllactosamine, 6-SL (6'-sialyllactose), 6'-sialyllactosamine, oligosaccharides comprising 6'-sialyllactose, SGG hexasaccharide (Neu5Acα-2,3Galβ-1,3GalNacβ-1,3Galα-1,4Galβ-1,4Gal), sialylated tetrasaccharide (Neu5Acα-2,3Galβ-1,4GlcNacβ-14GlcNAc), pentasaccharide LSTD (Neu5Acα-2,3Galβ-1,4GlcNacβ-1,3Galβ-1,4Glc), sialylated lacto-N-triose, sialylated lacto-N-tetraose, sialyllacto-N-neotetraose, monosialyllacto-N-hexaose, disialyllacto-N-hexaose I, monosialyllacto-N-neohexaose I, monosialyllacto-N-neohexaose II, disialyllacto-N-neohexaose, disialyllacto-N-tetraose, disialyllacto-N-hexaose II, sialyllacto-N-tetraose a, disialyllacto-N-hexaose I, sialyllacto-N-tetraose b, sialyllacto-N-neotetraose c, sialyllacto-N-neotetraose d, 3'-sialyl-3-fucosyllactose, disialomonofucosyllacto-N-neohexaose, monofucosylmonosialyllacto-N-octaose (sialyl Lea), sialyllacto-N-fucohexaose II, disialyllacto-N-fucopentaose II, monofucosyldisialyllacto-N-tetraose and oligosaccharides bearing one or several sialic acid residu(s), including but not limited to: oligosaccharide moieties of the gangliosides selected from GM3 (3'sialyllactose, Neu5Acα-2,3Galβ-4Glc) and oligosaccharides comprising the GM3 motif, GD3 Neu5Acα-2,8Neu5Acα-2,3Galβ-1,4Glc GT3 (Neu5Acα-2,8Neu5Acα-2,8Neu5Acα-2,3Galβ-1,4Glc); GM2 GalNAcβ-1,4(Neu5Acα-2,3)Galβ-1,4Glc, GM1 Galβ-1,3GalNAcβ-1,4(Neu5Acα-2,3)Galβ-1,4Glc, GDla Neu5Acα-2,3Galβ-1,3GalNAcβ-1,4(Neu5Acα-2,3)Galβ-1,4Glc, GT1a Neu5Acα-2,8Neu5Acα-2,3Galβ-1,3GalNAcβ-1,4(Neu5Acα-2,3)Galβ-1,4Glc, GD2 GalNAcβ-1,4(Neu5Acα-2,8Neu5Acα2,3)Galβ-1,4Glc, GT2 GalNAcβ-1,4(Neu5Acα-2,8Neu5Acα-2,8Neu5Acα2,3)Galβ-1,4Glc, GD1b, Galβ-1,3GalNAcβ-1,4(Neu5Acα-2,8Neu5Acα2,3)Galβ-1,4Glc, GT1b Neu5Acα-2,3Galβ-1,3GalNAcβ-1,4(Neu5Acα-2,8Neu5Acα2,3)Galβ-1,4Glc, GQ1b Neu5Acα-2,8Neu5Acα-2,3Galβ-1,3GalNAcβ-1,4(Neu5Acα-2,8Neu5Acα2,3)Galβ-1,4Glc, GT1c Galβ-1,3GalNAcβ-1,4(Neu5Acα-2,8Neu5Acα-2,8Neu5Acα2,3)Galβ-1,4Glc, GQ1c Neu5Acα-2,3Galβ-1,3GalNAc β -1,4(Neu5Acα-2,8Neu5Acα-2,8Neu5Acα2,3)Galβ-1,4Glc, GP1c Neu5Acα-2,8Neu5Acα-2,3Galβ-1,3GalNAc β -1,4(Neu5Acα-2,8Neu5Acα-2,8Neu5Acα2,3)Galβ-1,4Glc, GDla Neu5Acα-2,3Galβ-1,3(Neu5Acα-2,6)GalNAcβ -1,4Galβ-1,4Glc, Fucosyl-GM1 Fucα-1,2Galβ-1,3GalNAcβ -1,4(Neu5Acα-2,3)Gal β -1,4Glc; all of which may be extended to the production of the corresponding gangliosides by reacting the above oligosaccharide moieties with ceramide or synthetizing the above oligosaccharides on a ceramide.

The terms "alpha-2,3-sialyltranferase", "alpha 2,3 sialyltransferase", "3-sialyltransferase, "α-2,3-sialyltransferase", "α 2,3 sialyltransferase", "3 sialyltransferase, "3-ST" or "3ST" as used in the present invention, are used interchangeably and refer to a glycosyltransferase that catalyzes the transfer of sialic acid from the donor CMP-Neu5Ac, to the acceptor molecule in an alpha-2,3-linkage. The terms "3' sialyllactose", "3'-sialyllactose", "alpha-2,3-sialyllactose", "alpha 2,3 sialyllactose", "α-2,3-sialyllactose", "α 2,3 sialyllactose", 3SL" or "3'SL" as used in the present invention, are used interchangeably and refer to the product obtained by the catalysis of the alpha-2,3-fucosyltransferase transferring the sialic acid group from CMP-Neu5Ac to lactose in an alpha-2,3-linkage. The terms "alpha-2,6-sialyltranferase", "alpha 2,6 sialyltransferase", "6-sialyltransferase, "α-2,6-sialyltransferase", "α 2,6 sialyltransferase", "6 sialyltransferase, "6-ST" or "6ST" as used in the present invention, are used interchangeably and refer to a glycosyltransferase that catalyzes the transfer of sialic acid from the donor CMP-Neu5Ac, to the acceptor in an alpha-2,6-linkage. The terms "6' sialyllactose", "6'-sialyllactose", "alpha-2,6-sialyllactose", "alpha 2,6 sialyllactose", "α-2,6-sialyllactose", "α 2,6 sialyllactose", 6SL" or "6'SL" as used in the present invention, are used interchangeably and refer to the product obtained by the catalysis of the alpha-2,6-fucosyltransferase transferring the sialic acid group from CMP-Neu5Ac to lactose in an alpha-2,6-linkage. A 'neutral oligosaccharide' as used herein and as generally understood in the state of the art is an oligosaccharide that has no negative charge originating from a carboxylic acid group. Examples of such neutral oligosaccharide comprise 2'-fucosyllactose (2'FL), 3-fucosyllactose (3FL), 2', 3-difucosyllactose (diFL), lacto-N-triose II, lacto-N-tetraose, lacto-N-neotetraose, lacto-N-fucopentaose I, lacto-N-neofucopentaose I, lacto-N-fucopentaose II, lacto-N-fucopentaose III, lacto-N-fucopentaose V, lacto-N-fucopentaose VI, lacto-N-neofucopentaose V, lacto-N-difucohexaose I, lacto-N-difucohexaose II, 6'-galactosyllactose, 3'-galactosyllactose, lacto-N-hexaose, lacto-N-neohexaose, para-lacto-N-hexaose, para-lacto-N-neohexaose, difucosyl-lacto-N-hexaose and difucosyl-lacto-N-neohexaose.
Mammalian milk oligosaccharides comprise oligosaccharides present in milk found in any phase during lactation including colostrum milk from humans and mammals including but not limited to cows (*Bos Taurus),* sheep *(Ovis aries*), goats (*Capra aegagrus hircus),* bactrian camels (*Camelus bactrianus*), horses (*Equus ferus caballus*), pigs (*Sus scropha*), dogs (*Canis lupus familiaris*), ezo brown bears (*Ursus arctos yesoensis*), polar bear (*Ursus maritimus*)*,* Japanese black bears (*Ursus thibetanus japonicus*)*,* striped skunks (*Mephitis mephitis*)*,* hooded seals (*Cystophora cristata*), Asian elephants (*Elephas maximus*), African elephant (*Loxodonta africana*), giant anteater (*Myrmecophaga tridactyla*), common bottlenose dolphins (*Tursiops truncates*), northern minke whales (*Balaenoptera acutorostrata*), tammar wallabies (*Macropus eugenii*), red kangaroos (*Macropus rufus*), common brushtail possum (*Trichosurus Vulpecula*), koalas (*Phascolarctos cinereus*), eastern quolls (*Dasyurus viverrinus*), platypus (*Ornithorhynchus anatinus*).
As used herein, an antigen of the human ABO blood group system is an oligosaccharide. Such antigens of the human ABO blood group system are not restricted to human structures. Said structures involve the A determinant GalNAc-alpha1,3(Fuc-alpha1,2)-Gal-, the B determinant Gal-alphal,3(Fuc-alphal,2)-Gal- and the H determinant Fuc-alphal,2-Gal- that are present on disaccharide core structures comprising Gal-beta1,3-GlcNAc, Gal-betal,4-GlcNAc, Gal-beta1,3-GalNAc and Gal-beta1,4-Glc.
A 'fucosylation pathway' as used herein is a biochemical pathway consisting of the enzymes and their respective genes, mannose-6-phosphate isomerase, phosphomannomutase, mannose-1-phosphate guanylyltransferase, GDP-mannose 4,6-dehydratase, GDP-L-fucose synthase and/or the salvage pathway L-fucokinase/GDP-fucose pyrophosphorylase, combined with a fucosyltransferase leading to α 1,2; α 1,3 α 1,4 or α 1,6 fucosylated oligosaccharides.
A 'sialylation pathway' is a biochemical pathway consisting of the enzymes and their respective genes, L-glutamine-D-fructose-6-phosphate aminotransferase, glucosamine-6-phosphate deaminase, phosphoglucosamine mutase, N-acetylglucosamine-6-phosphate deacetylase, N-acetylglucosamine epimerase, UDP-N-acetylglucosamine 2-epimerase, N-acetylglucosamine-6P 2-epimerase, Glucosamine 6-phosphate N-acetyltransferase, N-AcetylGlucosamine-6-phosphate phosphatase, N-acetylmannosamine-6-phosphate phosphatase, N-acetylmannosamine kinase, phosphoacetylglucosamine mutase, N-acetylglucosamine-1-phosphate uridyltransferase, glucosamine-1-phosphate acetyltransferase, sialic acid synthase, N-acetylneuraminate lyase, N-acylneuraminate-9-phosphate synthase, N-acylneuraminate-9-phosphate phosphatase, and/or CMP-sialic acid synthase, combined with a sialyltransferase leading to α 2,3; α 2,6 α 2,8 sialylated oligosaccharides.
A 'galactosylation pathway' as used herein is a biochemical pathway consisting of the enzymes and their respective genes, galactose-1-epimerase, galactokinase, glucokinase, galactose-1-phosphate uridylyltransferase, UDP-glucose 4-epimerase, glucose-1-phosphate uridylyltransferase, and/or glucophosphomutase, combined with a galactosyltransferase leading to an alpha or beta bound galactose on the 2, 3, 4, 6 hydroxyl group of an oligosaccharide.
An 'N-acetylglucosamine carbohydrate pathway' as used herein is a biochemical pathway consisting of the enzymes and their respective genes, L-glutamine-D-fructose-6-phosphate aminotransferase, glucosamine-6-phosphate deaminase, phosphoglucosamine mutase, N-acetylglucosamine-6-phosphate deacetylase, glucosamine 6-phosphate N-acetyltransferase, N-acetylglucosamine-1-phosphate uridylyltransferase, glucosamine-1-phosphate acetyltransferase, and/or glucosamine-1-phosphate acetyltransferase, combined with a glycosyltransferase leading to an alpha or beta bound N-acetylglucosamine on the 3, 4, 6 hydroxylgroup of an oligosaccharide.
The term "purified" refers to material that is substantially or essentially free from components which interfere with the activity of the biological molecule. For cells, saccharides, nucleic acids, and polypeptides, the term "purified" refers to material that is substantially or essentially free from components which normally accompany the material as found in its native state. Typically, purified saccharides, oligosaccharides, proteins or nucleic acids of the invention are at least about 50 %, 55 %, 60 %, 65 %, 70 %, 75 %, 80 % or 85 % pure, usually at least about 90 %, 91 %, 92 %, 93 %, 94 %, 95 %, 96 %, 97 %, 98 %, or 99 % pure as measured by band intensity on a silver stained gel or other method for determining purity. Purity or homogeneity can be indicated by a number of means well known in the art, such as polyacrylamide gel electrophoresis of a protein or nucleic acid sample, followed by visualization upon staining. For certain purposes high resolution will be needed and HPLC or a similar means for purification utilized. For oligosaccharides, purity can be determined using methods such as but not limited to thin layer chromatography, gas chromatography, NMR, HPLC, capillary electrophoresis or mass spectroscopy.
The term "cultivation" refers to the culture medium wherein the cell is cultivated or fermented, the cell itself, and the oligosaccharides that are produced by the cell in whole broth, i.e. inside (intracellularly) as well as outside (extracellularly) of the cell.
The term "precursor" as used herein refers to substances which are taken up or synthetized by the cell for the specific production of an oligosaccharide according to the present invention. In this sense a precursor can be an acceptor as defined herein, but can also be another substance, metabolite, which is first modified within the cell as part of the biochemical synthesis route of the oligosaccharide. Examples of such precursors comprise the acceptors as defined herein, and/or glucose, galactose, fructose, glycerol, sialic acid, fucose, mannose, maltose, sucrose, lactose, glucose-1-phosphate, galactose-1-phosphate, UDP-glucose, UDP-galactose, glucose-6-phosphate, fructose-6-phosphate, fructose-1,6-bisphosphate, glycerol-3-phosphate, dihydroxyacetone, glyceraldehyde-3-phosphate, dihydroxyacetone-phosphate, glucosamine-6-phosphate, glucosamine, N-acetyl-glucosamine-6-phosphate, N-acetyl-glucosamine, N-acetyl-mannosamine, N-acetylmannosamine-6-phosphate, UDP-N-acetylglucosamine, N-acetylglucosamine-1-phosphate, N-acetylneuraminic acid (sialic acid), N-acetyl-Neuraminic acid-9-phopshate, CMP-sialic acid, mannose-6-phosphate, mannose-1-phosphate, GDP-mannose, GDP-4-dehydro-6-deoxy-α-D-mannose, and/or GDP-fucose.
The term "acceptor" as used herein refers to a di- or an oligosaccharide which is taken up by the cell and modified by one or more glycosyltransferases with the addition of one or more monosaccharide units for oligosaccharide assembly. Said acceptors are produced by methods comprising extraction from natural sources, biotechnological processes, physical processes, chemical processes, and combinations thereof. Examples of such acceptors comprise lactose, 2'FL, 3-FL, lacto-N-biose (LNB), lacto-N-triose, lacto-N-tetraose (LNT), lacto-N-neotetraose (LNnT), N-acetyl-lactosamine (LacNAc), lacto-N-pentaose (LNP), lacto-N-neopentaose, para lacto-N-pentaose, para lacto-N-neopentaose, lacto-N-novopentaose I, lacto-N-hexaose (LNH), lacto-N-neohexaose (LNnH), para lacto-N-neohexaose (pLNnH), para lacto-N-hexaose (pLNH), lacto-N-heptaose, lacto-N-neoheptaose, para lacto-N-neoheptaose, para lacto-N-heptaose, lacto-N-octaose (LNO), lacto-N-neooctaose, iso lacto-N-octaose, para lacto-N-octaose, iso lacto-N-neooctaose, novo lacto-N-neooctaose, para lacto-N-neooctaose, iso lacto-N-nonaose, novo lacto-N-nonaose, lacto-N-nonaose, lacto-N-decaose, iso lacto-N-decaose, novo lacto-N-decaose, lacto-N-neodecaose, galactosyllactose, a lactose extended with 1, 2, 3, 4, 5, or a multiple of N-acetyllactosamine units and/or 1, 2, 3, 4, 5, or a multiple of, Lacto-N-biose units, and oligosaccharide containing 1 or multiple N-acetyllactosamine units and or 1 or multiple lacto-N-biose units or an intermediate into oligosaccharide, fucosylated and sialylated versions thereof.

### Detailed description of the invention

According to a first embodiment, the present invention provides a method for the production of a mixture comprising at least two oligosaccharides. The method comprises the steps of:
i. providing a cell that expresses one glycosyltransferase and is capable to synthesize one nucleotide-sugar, wherein said nucleotide-sugar is donor for said glycosyltransferase, and
ii. cultivating said cell under conditions permissive to express said glycosyltransferase and to synthesize said nucleotide-sugar, and
iii. addition of at least two acceptors to said cultivation enabling said cell to produce at least two oligosaccharides, preferably any one of said acceptors is a di- or oligosaccharide,
iv. preferably, separating at least one of said oligosaccharides from said cultivation, more preferably separating all of said oligosaccharides from said cultivation.

According to the invention, said method for the production of a mixture comprising at least two oligosaccharides can make use of a non-metabolically engineered cell or can make use of a metabolically engineered cell.
In the scope of the present invention, permissive conditions are understood to be conditions relating to physical or chemical parameters including but not limited to temperature, pH, pressure, osmotic pressure and product/educt concentration.
In a particular embodiment, the permissive conditions may include a temperature-range of 30 +/- 20 degrees centigrade, a pH-range of 7 +/- 3.
In another preferred embodiment of the method of the invention, the cultivation is fed with at least two acceptors for the synthesis of any one of said oligosaccharides in said mixture. In a further preferred embodiment of the method, the cultivation is fed with at least two acceptors for the synthesis of all of said oligosaccharides in the mixture. In a more preferred embodiment, the cultivation is fed with at least three acceptors for the synthesis of any one or all of said oligosaccharides in said mixture.
According to the invention, said acceptors are di- or oligosaccharides. Disaccharide acceptors comprise lactose (Gal-b1,4-Glc), lacto-N-biose (Gal-b1,3-GlcNAc), N-acetyllactosamine (Gal-b1,4-GlcNAc), LacDiNAc (GalNAc-b1,4-GlcNAc), N-acetylgalactosaminylglucose (GalNAc-b1,4-Glc). Oligosaccharide acceptors comprise 2'FL, 3-FL, lacto-N-triose, lacto-N-tetraose (LNT), lacto-N-neotetraose (LNnT), lacto-N-pentaose (LNP), lacto-N-neopentaose, para lacto-N-pentaose, para lacto-N-neopentaose, lacto-N-novopentaose I, lacto-N-hexaose (LNH), lacto-N-neohexaose (LNnH), para lacto-N-neohexaose (pLNnH), para lacto-N-hexaose (pLNH), lacto-N-heptaose, lacto-N-neoheptaose, para lacto-N-neoheptaose, para lacto-N-heptaose, lacto-N-octaose (LNO), lacto-N-neooctaose, iso lacto-N-octaose, para lacto-N-octaose, iso lacto-N-neooctaose, novo lacto-N-neooctaose, para lacto-N-neooctaose, iso lacto-N-nonaose, novo lacto-N-nonaose, lacto-N-nonaose, lacto-N-decaose, iso lacto-N-decaose, novo lacto-N-decaose, lacto-N-neodecaose, galactosyllactose, a lactose extended with 1, 2, 3, 4, 5, or a multiple of N-acetyllactosamine units and/or 1, 2, 3, 4, 5, or a multiple of, Lacto-N-biose units, and oligosaccharide containing 1 or multiple N-acetyllactosamine units and or 1 or multiple lacto-N-biose units or an intermediate into oligosaccharide, fucosylated and sialylated versions thereof.
In an embodiment of the method according to the invention, any one of the acceptors added to the cultivation has a degree of polymerization of three (DP3). The degree of polymerization (DP) of an acceptor refers to the number of monosaccharide units present in the acceptor structure. An acceptor with DP3 should be understood to be an oligosaccharide built of three monosaccharide subunits, also known as a trisaccharide. In a preferred embodiment of the present invention, all of said acceptors have a DP3 structure. As used herein, the cultivation is fed with an oligosaccharide mixture comprising at least two acceptors, wherein all acceptors are trisaccharides composed of three monosaccharide subunits. In an alternative embodiment of the method according to the invention, any one of the acceptors added to the cultivation has a DP higher than three. An acceptor with a DP higher than three should be understood to comprise a tetrasaccharide built of 4 monosaccharide subunits (DP4), a pentasaccharide built of 5 monosaccharide subunits (DP5), an oligosaccharide structure built of DP6 or higher till DP20. In a preferred alternative embodiment, all of the acceptors added to the cultivation have a DP higher than three. For example, all acceptors added to the cultivation are tetrasaccharides and/or longer oligosaccharides built of 5, 6 or more monosaccharide subunits.
In an embodiment of the method according to the invention, all acceptors added to the cultivation have a different DP. For example, the cultivation can be fed with two acceptors wherein the first acceptor is a disaccharide and the second acceptor a trisaccharide. Alternatively, the cultivation can be fed with two acceptors wherein the first acceptor is a disaccharide and the second acceptor a tetrasaccharide or a longer saccharide of 5, 6 or more subunits. The cultivation can also be fed with two acceptors wherein the first acceptor is a trisaccharide and the second acceptor a tetrasaccharide or a longer saccharide of 5, 6 or more subunits. In a preferred embodiment, the cultivation is fed with more than two acceptors, wherein all said acceptors are built with a different number of monosaccharide subunits.
In another aspect of the present invention, the cultivation is fed with three or more acceptors for oligosaccharide synthesis, wherein said acceptors can have the same or a different DP relative to each other.
According to a preferred embodiment of the method according to the invention, the metabolically engineered cell used in the oligosaccharide synthesis is modified with gene expression modules wherein the expression from any one of said expression modules is constitutive or is created by a natural inducer. Said expression modules are also known as transcriptional units and comprise polynucleotides for expression of recombinant genes including coding gene sequences and appropriate transcriptional and/or translational control signals that are operably linked to the coding genes. Said control signals comprise promoter sequences, untranslated regions, ribosome binding sites, terminator sequences. Said expression modules can contain elements for expression of one single recombinant gene but can also contain elements for expression of more recombinant genes or can be organized in an operon structure for integrated expression of two or more recombinant genes. Said polynucleotides may be produced by recombinant DNA technology using techniques well-known in the art. Methods which are well known to those skilled in the art to construct expression modules include, for example, in vitro recombinant DNA techniques, synthetic techniques, and in vivo genetic recombination. See, for example, the techniques described in Sambrook et al. (2001) Molecular Cloning: a laboratory manual, 3rd Edition, Cold Spring Harbor Laboratory Press, CSH, New York or to Current Protocols in Molecular Biology, John Wiley and Sons, N.Y. (1989 and yearly updates).
According to a preferred aspect of the present invention, the cell is modified with one or more expression modules. The expression modules can be integrated in the genome of said cell or can be presented to said cell on a vector. Said vector can be present in the form of a plasmid, cosmid, phage, liposome, or virus, which is to be stably transformed/transfected into said metabolically engineered cell. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses, and vectors derived from combinations thereof, such as those derived from plasmid and bacteriophage genetic elements, such as cosmids and phagemids. These vectors may contain selection markers such as but not limited to antibiotic markers, auxotrophic markers, toxin-antitoxin markers, RNA sense/antisense markers. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook et al., see above. For recombinant production, cells can be genetically engineered to incorporate expression systems or portions thereof or polynucleotides of the invention. Introduction of a polynucleotide into the cell can be effected by methods described in many standard laboratory manuals, such as Davis et al., Basic Methods in Molecular Biology, (1986), and Sambrook et al., 1989, supra.

As used herein an expression module comprises polynucleotides for expression of at least one recombinant gene. Said recombinant gene is involved in the expression of a polypeptide acting in the synthesis of said oligosaccharide mixture; or said recombinant gene is linked to other pathways in said host cell that are not involved in the synthesis of said mixture of three or more oligosaccharides. Said recombinant genes encode endogenous proteins with a modified expression or activity, preferably said endogenous proteins are overexpressed; or said recombinant genes encode heterologous proteins that are heterogeneously introduced and expressed in said modified cell, preferably overexpressed. The endogenous proteins can have a modified expression in the cell which also expresses a heterologous protein.
According to a preferred aspect of the present invention, the expression of each of said expression modules is constitutive or created by a natural inducer. As used herein, constitutive expression should be understood as expression of a gene that is transcribed continuously in an organism. Expression that is created by a natural inducer should be understood as a facultative or regulatory expression of a gene that is only expressed upon a certain natural condition of the host (e.g. organism being in labor, or during lactation), as a response to an environmental change (e.g. including but not limited to hormone, heat, cold, light, oxidative or osmotic stress / signaling), or dependent on the position of the developmental stage or the cell cycle of said host cell including but not limited to apoptosis and autophagy.
According to one aspect of the method of the invention, the cell produces a mixture comprising three different oligosaccharides or more than three different oligosaccharides.
In one embodiment, the cell produces a mixture of different oligosaccharides with at least two oligosaccharides differing in degree of polymerization.
In an embodiment of the method according to the invention, the glycosyltransferase is chosen from the list comprising fucosyltransferases, sialyltransferases, galactosyltransferases, glucosyltransferases, mannosyltransferases, N-acetylglucosaminyltransferases, N-acetylgalactosaminyltransferases, N-acetylmannosaminyltransferases, xylosyltransferases, glucuronyltransferases, galacturonyltransferases, glucosaminyltransferases, N-glycolylneuraminyltransferases, rhamnosyltransferases, as defined herein. In one embodiment, the cell expresses more than one glycosyltransferase as described herein.
In a further aspect of the method of the invention, the cell is modified in the expression or activity of said glycosyltransferase. In a preferred embodiment, said glycosyltransferase is an endogenous protein of the cell with a modified expression or activity, preferably said endogenous glycosyltransferase is overexpressed; alternatively said glycosyltransferase is an heterologous protein that is heterogeneously introduced and expressed in said cell, preferably overexpressed. Said endogenous glycosyltransferase can have a modified expression in the cell which also expresses a heterologous glycosyltransferase.
In another embodiment of the method of the present invention, said glycosyltransferase is a fucosyltransferase and the cell is capable to synthesize GDP-Fuc. The GDP-fucose can be provided by an enzyme expressed in the cell or by the metabolism of the cell. Such cell producing GDP-fucose can express an enzyme converting, e.g., fucose, which is to be added to the cell, to GDP-fucose. This enzyme may be, e.g., a bifunctional fucose kinase/fucose-1-phosphate guanylyltransferase, like Fkp from *Bacteroides fragilis,* or the combination of one separate fucose kinase together with one separate fucose-1-phosphate guanylyltransferase like they are known from several species including *Homo sapiens, Sus scrofa* and *Rattus norvegicus.*
Preferably, the cell is modified to produce GDP-fucose. More preferably, the cell is modified for enhanced GDP-fucose production. Said modification can be any one or more chosen from the group comprising knock-out of an UDP-glucose:undecaprenyl-phosphate glucose-1-phosphate transferase encoding gene, over-expression of a GDP-L-fucose synthase encoding gene, over-expression of a GDP-mannose 4,6-dehydratase encoding gene, over-expression of a mannose-1-phosphate guanylyltransferase encoding gene, over-expression of a phosphomannomutase encoding gene and over-expression of a mannose-6-phosphate isomerase encoding gene.
In another aspect of the method of the invention, said glycosyltransferase is a sialyltransferase and the cell is capable to synthesize CMP-Neu5Ac. The CMP-Neu5Ac can be provided by an enzyme expressed in the cell or by the metabolism of the cell. Such cell producing CMP-Neu5Ac can express an enzyme converting, e.g., sialic acid, which is to be added to the cell, to CMP-Neu5Ac. This enzyme may be a CMP-sialic acid synthetase, like the N-acylneuraminate cytidylyltransferase from several species including *Homo sapiens, Neisseria meningitidis,* and *Pasteurella multocida.* Preferably, the cell is modified to produce CMP-Neu5Ac. More preferably, the cell is modified for enhanced CMP-Neu5Ac production. Said modification can be any one or more chosen from the group comprising knock-out of an N-acetylglucosamine-6-phosphate deacetylase, knock-out of an glucosamine-6-phosphate deaminase, over-expression of a sialate synthase encoding gene, and over-expression of an N-acetyl-D-glucosamine-2-epimerase encoding gene.
In another aspect of the method of the invention, said glycosyltransferase is an N-acetylglucosaminyltransferase and the cell is capable to synthesize UDP-GlcNAc. The UDP-GlcNAc can be provided by an enzyme expressed in the cell or by the metabolism of the cell. Such cell producing an UDP-GlcNAc can express enzymes converting, e.g. GlcNAc to UDP-GlcNAc. These enzymes may be an N-acetyl-D-glucosamine kinase, an N-acetylglucosamine-6-phosphate deacetylase, a phosphoglucosamine mutase, and an N-acetylglucosamine-1-phosphate uridyltransferase/glucosamine-1-phosphate acetyltransferase from several species including *Homo sapiens, Escherichia coli.* Preferably, the cell is modified to produce UDP-GlcNAc. More preferably, the cell is modified for enhanced UDP-GlcNAc production. Said modification can be any one or more chosen from the group comprising knock-out of an N-acetylglucosamine-6-phosphate deacetylase, over-expression of an L-glutamine-D-fructose-6-phosphate aminotransferase, over-expression of a a phosphoglucosamine mutase, and over-expression of an N-acetylglucosamine-1-phosphate uridyltransferase/glucosamine-1-phosphate acetyltransferase. In another aspect of the method of the invention, said glycosyltransferase is a galactosyltransferase and the cell is capable to synthesize UDP-Gal. The UDP-Gal can be provided by an enzyme expressed in the cell or by the metabolism of the cell. Such cell producing UDP-Gal can express an enzyme converting, e.g. UDP-glucose, to UDP-Gal. This enzyme may be, e.g., the UDP-glucose-4-epimerase GalE like as known from several species including *Homo sapiens, Escherichia coli,* and *Rattus norvegicus.* Preferably, the cell is modified to produce UDP-Gal. More preferably, the cell is modified for enhanced UDP-Gal production. Said modification can be any one or more chosen from the group comprising knock-out of an bifunctional 5'-nucleotidase/UDP-sugar hydrolase encoding gene, knock-out of a galactose-1-phosphate uridylyltransferase encoding gene and over-expression of an UDP-glucose-4-epimerase encoding gene. In another embodiment of the method of the invention, the cell is capable to synthesize any one of said nucleotide-sugars chosen from the list comprising GDP-Fuc, CMP-Neu5Ac, UDP-GlcNAc, UDP-Gal, UDP-N-acetylgalactosamine (UDP-GalNAc), UDP-N-acetylmannosamine (UDP-ManNAc), GDP-mannose (GDP-Man), UDP-glucose (UDP-Glc), CMP-N-glycolylneuraminic acid (CMP-Neu5Gc), UDP-glucuronate, UDP-galacturonate, GDP-rhamnose, UDP-xylose. In a preferred embodiment, said cell is metabolically engineered for the production of a nucleotide-sugar. In another preferred embodiment, the cell is modified and/or engineered for the optimized production of a nucleotide-sugar.
According to the method of the invention, at least one of said oligosaccharides of said mixture is fucosylated, sialylated, galactosylated, glucosylated, xylosylated, mannosylated, contains an N-acetylglucosamine, contains an N-acetylneuraminate, contains an N-glycolylneuraminate, contains an N-acetylgalactosamine, contains a rhamnose, contains a glucuronate, contains a galacturonate, and/or contains an N-acetylmannosamine.
Preferably, the oligosaccharide mixture comprises at least one fucosylated oligosaccharide as defined herein.
Alternatively or additionally, the oligosaccharide mixture comprises at least one sialylated oligosaccharide as defined herein.
Alternatively or additionally, the mixture of oligosaccharides comprises at least one oligosaccharide of 3 or more monosaccharide subunits linked to each other via glycosidic bonds, wherein at least one of said monosaccharide residues is a GlcNAc residue. Said oligosaccharide can contain more than one GlcNAc residue, e.g. two, three or more. Said oligosaccharide can be a neutral oligosaccharide or a charged oligosaccharide, e.g. also comprising sialic acid structures. GlcNAc can be present at the reducing end of the oligosaccharide. Said GlcNAc can also be present at the non-reducing end of said oligosaccharide. Said GlcNAc can also be present within the oligosaccharide structure. GlcNAc can be linked to other monosaccharide subunits comprising galactose, fucose, Neu5Ac, Neu5Gc.
Alternatively or additionally, the oligosaccharide mixture comprises at least one galactosylated oligosaccharide and contains at least one galactose monosaccharide subunit. Said galactosylated oligosaccharide is a saccharide structure comprising at least three monosaccharide subunits linked to each other via glycosidic bonds, wherein at least one of said monosaccharide subunit is a galactose. Said galactosylated oligosaccharide can contain more than one galactose residue, e.g. two, three or more. Said galactosylated oligosaccharide can be a neutral oligosaccharide or a charged oligosaccharide, e.g. also comprising sialic acid structures. Galactose can be linked to other monosaccharide subunits comprising glucose, GlcNAc, fucose, sialic acid.
Preferably, all oligosaccharides present in the oligosaccharide mixture are fucosylated oligosaccharides. More preferably, the oligosaccharide mixture comprises three fucosylated oligosaccharides as defined herein.

In a preferred embodiment of the method of the invention, any one of said oligosaccharides is translocated to the outside of the cell by a passive transport i.e. without means of an active transport system consuming energy from said cell.

In another preferred embodiment of the method of the invention, the cell is further metabolically engineered for
i) modified expression of an endogenous membrane protein, and/or
ii) modified activity of an endogenous membrane protein, and/or
iii) expression of a homologous membrane protein, and/or
iv) expression of a heterologous membrane protein,
wherein said membrane protein is involved in the secretion of any one of said oligosaccharides outside said cell. Said cell can express one of said membrane proteins that is involved in the secretion of any one of said oligosaccharides from said cell to the outside of said cell. Said cell can also express more than one of said membrane proteins. Any one of said membrane proteins can translocate one or more of said oligosaccharides to the outside of said cell. Said cell producing a mixture of at least three oligosaccharides can translocate any one of said oligosaccharides comprising passive diffusion, channel membrane proteins, membrane transporter proteins, membrane carrier proteins.

In one embodiment, the cell is further metabolically engineered for
i. modified expression of an endogenous membrane protein, and/or
ii. modified activity of an endogenous membrane protein, and/or
iii. expression of a homologous membrane protein, and/or
iv. expression of a heterologous membrane protein,
wherein said membrane protein is involved in the uptake of a precursor for the synthesis of any one of said oligosaccharides of the mixture, preferably wherein said membrane protein is involved in the uptake of all of the required precursors, more preferably wherein said membrane protein is involved in the uptake of all of said acceptors

In another preferred embodiment of the method of the invention, the cell is further metabolically engineered for
i) modified expression of an endogenous membrane protein, and/or
ii) modified activity of an endogenous membrane protein, and/or
iii) expression of a homologous membrane protein, and/or
iv) expression of a heterologous membrane protein,
wherein said membrane protein is involved in the uptake of an acceptor for the synthesis of any one of said oligosaccharides. The term "acceptor" should be understood as explained in the definitions as disclosed herein. Said cell can express one of said membrane proteins that is involved in the uptake of any type of acceptor used in the synthesis of any one of said oligosaccharides. Said cell can also express more than one of said membrane proteins, involved in the uptake of at least one of said acceptors. Said cell can be modified for the uptake of more than one acceptor for the synthesis of any one of said oligosaccharides.

Optionally, the cell is transformed to comprise at least one nucleic acid sequence encoding a protein selected from the group consisting of lactose transporter, N-acetylneuraminic acid transporter, fucose transporter, transporter for a nucleotide-activated sugar wherein said transporter internalizes a to the medium added precursor or acceptor for oligosaccharide synthesis.
In another embodiment of the method, the cell is producing an acceptor for the synthesis of any one of said oligosaccharides. In a preferred embodiment, said cell is producing one or more acceptors for the synthesis of said oligosaccharide mixture. In a more preferred embodiment, said cell is modified for optimized production of any one of said acceptors for the synthesis of any one of said oligosaccharides. In another aspect of the present invention, said acceptors are produced by methods comprising extraction from natural sources, biotechnological processes including synthesis by said cell producing an oligosaccharide mixture, physical processes, chemical processes, and combinations thereof.
In a preferred embodiment of the method of the invention, the cell confers enhanced bacteriophage resistance. Said enhancement of bacteriophage resistance can be derived from reduced expression of an endogenous membrane protein and/or mutation of an endogenous membrane protein encoding gene. The term "phage insensitive" or "phage resistant" or "phage resistance" or "phage resistant profile" is understood to mean a bacterial strain that is less sensitive, and preferably insensitive to infection and/ or killing by phage and/ or growth inhibition. As used herein, the terms "anti-phage activity" or "resistant to infection by at least one phage" refers to an increase in resistance of a bacterial cell expressing a functional phage resistance system to infection by at least one phage family in comparison to a bacterial cell of the same species under the same developmental stage (e.g. culture state) which does not express a functional phage resistance system, as may be determined by e.g. bacterial viability, phage lysogeny, phage genomic replication and phage genomic degradation. The phage can be a lytic phage or a temperate (lysogenic) phage. Membrane proteins involved in bacteriophage resistance of a cell comprise OmpA, OmpC, OmpF, OmpT, BtuB, TolC, LamB, FhuA, TonB, FadL, Tsx, FepA, YncD, PhoE, and NfrA and homologs thereof.
In a preferred embodiment of the method of the invention, the cell confers reduced viscosity. Reduced viscosity of a cell can be obtained by a modified cell wall biosynthesis. Cell wall biosynthesis can be modified comprising reduced or abolished synthesis of for example poly-N-acetyl-glucosamine, the enterobacterial common antigen, cellulose, colanic acid, core oligosaccharides, osmoregulated periplasmic glucans and glucosylglycerol, glycan, and trehalose.
In another embodiment of the method of the invention, at least one of the oligosaccharides produced by said cell is a mammalian milk oligosaccharide. Said cell can produce one mammalian milk oligosaccharide in said produced mixture of at least two oligosaccharides. Said cell can produce more than one mammalian milk oligosaccharide in said produced mixture of at least two oligosaccharides. In a preferred embodiment, all said oligosaccharides in the produced mixture of at least two oligosaccharides are mammalian milk oligosaccharides.
In another embodiment of the method of the invention, any one of said oligosaccharides produced by said cell is an antigen of the human ABO blood group system. In a more preferred embodiment, all said oligosaccharides produced by said cell are antigens of the human ABO blood group system.
According to the present invention, the method as described herein preferably comprises a step of separating at least one of said oligosaccharides from said cultivation.
The terms "separating from said cultivation" means harvesting, collecting, or retrieving any of said oligosaccharides from the cell and/or the medium of its growth.
Any one of said oligosaccharides can be separated in a conventional manner from the aqueous culture medium, in which the cell was grown. In case said oligosaccharide is still present in the cells producing the oligosaccharide mixture, conventional manners to free or to extract said oligosaccharide out of the cells can be used, such as cell destruction using high pH, heat shock, sonication, French press, homogenization, enzymatic hydrolysis, chemical hydrolysis, solvent hydrolysis, detergent, hydrolysis,... The culture medium and/or cell extract together and separately can then be further used for separating said oligosaccharide. This preferably involves clarifying said oligosaccharide containing mixture to remove suspended particulates and contaminants, particularly cells, cell components, insoluble metabolites and debris produced by culturing the genetically modified cell. In this step, said oligosaccharide containing mixture can be clarified in a conventional manner. Preferably, said oligosaccharide containing mixture is clarified by centrifugation, flocculation, decantation and/or filtration. A second step of separating said oligosaccharide from said oligosaccharide containing mixture preferably involves removing substantially all the proteins, as well as peptides, amino acids, RNA and DNA and any endotoxins and glycolipids that could interfere with the subsequent separation step, from said oligosaccharide containing mixture, preferably after it has been clarified. In this step, proteins and related impurities can be removed from said oligosaccharide containing mixture in a conventional manner. Preferably, proteins, salts, by-products, colour and other related impurities are removed from said oligosaccharide containing mixture by ultrafiltration, nanofiltration, reverse osmosis, microfiltration, activated charcoal or carbon treatment, tangential flow high-performance filtration, tangential flow ultrafiltration, affinity chromatography, ion exchange chromatography (such as but not limited to cation exchange, anion exchange, mixed bed ion exchange), hydrophobic interaction chromatography and/or gel filtration (i.e., size exclusion chromatography), particularly by chromatography, more particularly by ion exchange chromatography or hydrophobic interaction chromatography or ligand exchange chromatography. With the exception of size exclusion chromatography, proteins and related impurities are retained by a chromatography medium or a selected membrane, said oligosaccharide remains in the said oligosaccharide containing mixture. Another aspect of the invention provides for a method and a cell wherein a mixture comprising at least two different oligosaccharides is produced in and/or by a fungal, yeast, bacterial, insect, animal and plant expression system or cell as described herein. The expression system or cell is chosen from the list comprising a bacterium, a yeast, or a fungus, or, refers to a plant or animal cell. The latter bacterium preferably belongs to the phylum of the Proteobacteria or the phylum of the Firmicutes or the phylum of the Cyanobacteria or the phylum Deinococcus-Thermus. The latter bacterium belonging to the phylum Proteobacteria belongs preferably to the family Enterobacteriaceae, preferably to the species *Escherichia coli.* The latter bacterium preferably relates to any strain belonging to the species *Escherichia coli* such as but not limited to *Escherichia coli* B, *Escherichia coli* C, *Escherichia coli* W, *Escherichia coli* K12, *Escherichia coli* Nissle. More specifically, the latter term relates to cultivated *Escherichia coli* strains - designated as *E. coli* K12 strains - which are well-adapted to the laboratory environment, and, unlike wild type strains, have lost their ability to thrive in the intestine. Well-known examples of the *E. coli* K12 strains are K12 Wild type, W3110, MG1655, M182, MC1000, MC1060, MC1061, MC4100, JM101, NZN111 and AA200. Hence, the present invention specifically relates to a mutated and/or transformed *Escherichia coli* cell or strain as indicated above wherein said *E. coli* strain is a K12 strain. More preferably, the *Escherichia coli* K12 strain is *E. coli* MG1655. The latter bacterium belonging to the phylum Firmicutes belongs preferably to the Bacilli, preferably Lactobacilliales, with members such as *Lactobacillus lactis, Leuconostoc mesenteroides,* or Bacillales with members such as from the genus Bacillus, such as *Bacillus subtilis* or, *B. amyloliquefaciens.* The latter Bacterium belonging to the phylum Actinobacteria, preferably belonging to the family of the Corynebacteriaceae, with members *Corynebacterium glutamicum* or *C. afermentans,* or belonging to the family of the Streptomycetaceae with members *Streptomyces griseus* or *S. fradiae.* The latter yeast preferably belongs to the phylum of the Ascomycota or the phylum of the Basidiomycota or the phylum of the Deuteromycota or the phylum of the Zygomycetes. The latter yeast belongs preferably to the genus Saccharomyces, Pichia, Komagataella, Hansenula, Kluyveromyces, Yarrowia or Starmerella. The latter fungus belongs preferably to the genus Rhizopus, Dictyostelium, Penicillium, Mucor or Aspergillus.
The microorganism or cell as used herein is capable to grow on a monosaccharide, disaccharide, oligosaccharide, polysaccharide, polyol, a complex medium or a mixture thereof as the main carbon source. With the term main is meant the most important carbon source for the bioproducts of interest, biomass formation, carbon dioxide and/or by-products formation (such as acids and/or alcohols, such as acetate, lactate, and/or ethanol), i.e. 20, 30, 40, 50, 60, 70, 75, 80, 85, 90, 95, 98, 99 % of all the required carbon is derived from the above-indicated carbon source. In one embodiment of the invention, said carbon source is the sole carbon source for said organism, i.e. 100 % of all the required carbon is derived from the above-indicated carbon source. Common main carbon sources comprise but are not limited to glucose, glycerol, fructose, maltose, lactose, arabinose, malto-oligosaccharides, maltotriose, sorbitol, xylose, rhamnose, sucrose, galactose, mannose, methanol, ethanol, trehalose, starch, cellulose, hemicellulose, corn-steep liquor, high-fructose syrup, acetate, citrate, lactate and pyruvate. With the term complex medium is meant a medium for which the exact constitution is not determined. Examples are molasses, corn steep liquor, peptone, tryptone or yeast extract.
In a further preferred embodiment, the microorganism or cell described herein is using a split metabolism having a production pathway and a biomass pathway as described in WO2012/007481, which is herein incorporated by reference. Said organism can, for example, be genetically modified to accumulate fructose-6-phosphate by altering the genes selected from the phosphoglucoisomerase gene, phosphofructokinase gene, fructose-6-phosphate aldolase gene, fructose isomerase gene, and/or fructose:PEP phosphotransferase gene.
In a further preferred embodiment, the methods as described herein also provide for a further purification of any one or more of said oligosaccharide(s) from the oligosaccharide mixture. A further purification of said oligosaccharide(s) may be accomplished, for example, by use of (activated) charcoal or carbon, nanofiltration, ultrafiltration or ion exchange to remove any remaining DNA, protein, LPS, endotoxins, or other impurity. Alcohols, such as ethanol, and aqueous alcohol mixtures can also be used. Another purification step is accomplished by crystallization, evaporation or precipitation of the product. Another purification step is to dry, spray dry or lyophilize the produced oligosaccharide.

In an exemplary embodiment, the separation and purification of at least one of the produced oligosaccharides is made in a process, comprising the following steps in any order:
a) contacting the cultivation or a clarified version thereof with a nanofiltration membrane with a molecular weight cut-off (MWCO) of 600-3500 Da ensuring the retention of produced oligosaccharide(s) and allowing at least a part of the proteins, salts, by-products, colour and other related impurities to pass,
b) conducting a diafiltration process on the retentate from step a), using said membrane, with an aqueous solution of an inorganic electrolyte, followed by optional diafiltration with pure water to remove excess of the electrolyte,
c) and collecting the retentate enriched in the oligosaccharide(s) in the form of a salt from the cation of said electrolyte.

In an alternative exemplary embodiment, the separation and purification of at least one of the produced oligosaccharides is made in a process, comprising the following steps in any order: subjecting the cultivation or a clarified version thereof to two membrane filtration steps using different membranes, wherein - one membrane has a molecular weight cut-off of between about 300 to about 500 Dalton, and - the other membrane as a molecular weight cut-off of between about 600 to about 800 Dalton. In an alternative exemplary embodiment, the separation and purification of at least one of the produced oligosaccharides is made in a process, comprising the following steps in any order comprising the step of treating the cultivation or a clarified version thereof with a strong cation exchange resin in H+-form and a weak anion exchange resin in free base form.

In an alternative exemplary embodiment, the separation and purification of at least one of the produced oligosaccharides is made in the following way. The cultivation comprising the produced oligosaccharide, biomass, medium components and contaminants, and wherein the purity of the produced oligosaccharide in the fermentation broth is < 80 percent, is applied to the following purification steps:
i) separation of biomass from the cultivation,
ii) cationic ion exchanger treatment for the removal of positively charged material,
iii) anionic ion exchanger treatment for the removal of negatively charged material,
iv) nanofiltration step and/or electrodialysis step,
wherein a purified solution comprising the produced oligosaccharide(s) at a purity of greater than or equal to 80 percent is provided. Optionally the purified solution is spray dried.

In an alternative exemplary embodiment, the separation and purificaiton of at least one of the produced oligosaccharides is made in a process, comprising the following steps in any order: enzymatic treatment of the cultivation; removal of the biomass from the cultivation; ultrafiltration; nanofiltration; and a column chromatography step. Preferably such column chromatography is a single column or a multiple column.

Further preferably the column chromatography step is simulated moving bed chromatography. Such simulated moving bed chromatography preferably comprises i) at least 4 columns, wherein at least one column comprises a weak or strong cation exchange resin; and/or ii) four zones I, II, III and IV with different flow rates; and/or iii) an eluent comprising water; and/or iv) an operating temperature of 15 degrees to 60 degrees centigrade.
In a specific embodiment, the present invention provides the produced oligosaccharide or oligosaccharide mixture which is spray-dried to powder, wherein the spray-dried powder contains < 15 percent -wt. of water, preferably < 10 percent -wt. of water, more preferably < 7 percent -wt. of water, most preferably < 5 percent -wt. of water.
In a second aspect, the present invention provides for the use of a method as described herein for the production of a mixture comprising at least two different oligosaccharides.
For identification of the oligosaccharides in the mixture comprising at least two different oligosaccharides produced in the cell as described herein, the monomeric building blocks (e.g. the monosaccharide or glycan unit composition), the anomeric configuration of side chains, the presence and location of substituent groups, degree of polymerization/molecular weight and the linkage pattern can be identified by standard methods known in the art, such as, e.g. methylation analysis, reductive cleavage, hydrolysis, GC-MS (gas chromatography-mass spectrometry), MALDI-MS (Matrix-assisted laser desorption/ionization-mass spectrometry), ESI-MS (Electrospray ionization-mass spectrometry), HPLC (High-Performance Liquid chromatography with ultraviolet or refractive index detection), HPAEC-PAD (High-Performance Anion-Exchange chromatography with Pulsed Amperometric Detection), CE (capillary electrophoresis), IR (infrared)/Raman spectroscopy, and NMR (Nuclear magnetic resonance) spectroscopy techniques. The crystal structure can be solved using, e.g., solid-state NMR, FT-IR (Fourier transform infrared spectroscopy), and WAXS (wide-angle X-ray scattering). The degree of polymerization (DP), the DP distribution, and polydispersity can be determined by, e.g., viscosimetry and SEC (SEC-HPLC, high performance size-exclusion chromatography). To identify the monomeric components of the saccharide methods such as, e.g. acid-catalyzed hydrolysis, HPLC (high performance liquid chromatography) or GLC (gas-liquid chromatography) (after conversion to alditol acetates) may be used. To determine the glycosidic linkages, the saccharide is methylated with methyl iodide and strong base in DMSO, hydrolysis is performed, a reduction to partially methylated alditols is achieved, an acetylation to methylated alditol acetates is performed, and the analysis is carried out by GLC/MS (gas-liquid chromatography coupled with mass spectrometry). To determine the oligosaccharide sequence, a partial depolymerization is carried out using an acid or enzymes to determine the structures. To identify the anomeric configuration, the oligosaccharide is subjected to enzymatic analysis, e.g. it is contacted with an enzyme that is specific for a particular type of linkage, e.g., beta-galactosidase, or alpha-glucosidase, etc., and NMR may be used to analyze the products.

### Products comprising the oligosaccharide mixture

In some embodiments, an oligosaccharide mixture produced as described herein is incorporated into a food (e.g, human food or feed), dietary supplement, pharmaceutical ingredient, cosmetic ingredient or medicine. In some embodiments, the oligosaccharide mixture is mixed with one or more ingredients suitable for food, feed, dietary supplement, pharmaceutical ingredient, cosmetic ingredient or medicine. In some embodiments, the dietary supplement comprises at least one prebiotic ingredient and/or at least one probiotic ingredient.
A "prebiotic" is a substance that promotes growth of microorganisms beneficial to the host, particularly microorganisms in the gastrointestinal tract. In some embodiments, a dietary supplement provides multiple prebiotics, including the oligosaccharide mixture produced and/or purified by a process disclosed in this specification, to promote growth of one or more beneficial microorganisms. Examples of prebiotic ingredients for dietary supplements include other prebiotic molecules (such as HMOs) and plant polysaccharides (such as inulin, pectin, b- glucan and xylooligosaccharide). A "probiotic" product typically contains live microorganisms that replace or add to gastrointestinal microflora, to the benefit of the recipient. Examples of such microorganisms include Lactobacillus species (for example, L. acidophilus and L. bulgaricus), Bifidobacterium species (for example, B. animalis, B. longum and B. infantis (e.g., Bi-26)), and Saccharomyces boulardii. In some embodiments, an oligosaccharide mixture produced and/or purified by a process of this specification is orally administered in combination with such microorganism. Examples of further ingredients for dietary supplements include disaccharides (such as lactose), monosaccharides (such as glucose and galactose), thickeners (such as gum arabic), acidity regulators (such as trisodium citrate), water, skimmed milk, and flavourings.
In some embodiments, the oligosaccharide mixture is incorporated into a human baby food (e.g., infant formula). Infant formula is generally a manufactured food for feeding to infants as a complete or partial substitute for human breast milk. In some embodiments, infant formula is sold as a powder and prepared for bottle- or cup-feeding to an infant by mixing with water. The composition of infant formula is typically designed to be roughly mimic human breast milk. In some embodiments, an oligosaccharide mixture produced and/or purified by a process in this specification is included in infant formula to provide nutritional benefits similar to those provided by the oligosaccharides in human breast milk. In some embodiments, the oligosaccharide mixture is mixed with one or more ingredients of the infant formula. Examples of infant formula ingredients include nonfat milk, carbohydrate sources (e.g., lactose), protein sources (e.g., whey protein concentrate and casein), fat sources (e.g., vegetable oils - such as palm, high oleic safflower oil, rapeseed, coconut and/or sunflower oil; and fish oils), vitamins (such as vitamins A, Bb, Bi2, C and D), minerals (such as potassium citrate, calcium citrate, magnesium chloride, sodium chloride, sodium citrate and calcium phosphate) and possibly human milk oligosaccharides (HMOs). Such HMOs may include, for example, DiFL, lacto-N-triose II, LNT, LNnT, lacto-N-fucopentaose I, lacto-N-neofucopentaose, lacto-N-fucopentaose II, lacto-N- fucopentaose III, lacto-N-fucopentaose V, lacto-N-neofucopentaose V, lacto-N-difucohexaose I, lacto-N-difucohexaose II, 6'-galactosyllactose, 3'-galactosyllactose, lacto-N-hexaose and lacto- N-neohexaose.
In some embodiments, the one or more infant formula ingredients comprise nonfat milk, a carbohydrate source, a protein source, a fat source, and/or a vitamin and mineral.
In some embodiments, the one or more infant formula ingredients comprise lactose, whey protein concentrate and/or high oleic safflower oil.
In some embodiments, the oligosaccharide mixture's concentration in the infant formula is approximately the same concentration as the oligosaccharide's concentration generally present in human breast milk. In some embodiments, the concentration of each of the single oligosaccharides in the mixture of oligosaccharides in the infant formula is approximately the same concentration as the concentration of that oligosaccharide generally present in human breast milk.
In some embodiments, the oligosaccharide mixture is incorporated into a feed preparation, wherein said feed is chosen from the list comprising petfood, animal milk replacer, veterinary product, post weaning feed, or creep feed.
Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization described above and below are those well-known and commonly employed in the art. Standard techniques are used for nucleic acid and peptide synthesis. Generally, purification steps are performed according to the manufacturer's specifications.
Further advantages follow from the specific embodiments, the examples and the attached drawings. It goes without saying that the abovementioned features and the features which are still to be explained below can be used not only in the respectively specified combinations, but also in other combinations or on their own, without departing from the scope of the present invention.

The present invention relates to following specific embodiments:
1. A method to produce a mixture of at least two different oligosaccharides by a cell, the method comprising the steps of:
   i. providing a cell expressing one glycosyltransferase and capable to synthesize one nucleotide-sugar, wherein said nucleotide-sugar is donor for said glycosyltransferase, and
   ii. cultivating said cell under conditions permissive to express said glycosyltransferase and to synthesize said nucleotide-sugar, and
   iii. addition of at least two acceptors to said cultivation enabling said cell to produce at least two oligosaccharides, preferably any one of said acceptors is a di- or oligosaccharide,
   iv. preferably, separating at least one of said oligosaccharides from said cultivation, more preferably separating all of said oligosaccharides from said cultivation.
2. Method according to embodiment 1, wherein said cell is a metabolically engineered cell modified with at least one gene expression module, characterized in that the expression from any one of said at least one expression modules is either constitutive or is created by a natural inducer.
3. Method according to any one of embodiment 1 or 2, wherein said cell produces a mixture of three or more different oligosaccharides.
4. Method according to any one of embodiment 1 to 3, wherein said cell produces a mixture of different oligosaccharides with at least two oligosaccharides differing in degree of polymerization.
5. Method according to any one of embodiment 1 to 4, wherein said glycosyltransferase is chosen from the list comprising fucosyltransferases, sialyltransferases, galactosyltransferases, glucosyltransferases, mannosyltransferases, N-acetylglucosaminyltransferases, N-acetylgalactosaminyltransferases, N-acetylmannosaminyltransferases, xylosyltransferases, glucuronyltransferases, galacturonyltransferases, glucosaminyltransferases, N-glycolylneuraminyltransferases, rhamnosyltransferases.
6. Method according to any one of embodiment 1 to 5 wherein said cell is modified in the expression or activity of said glycosyltransferase.
7. Method according to any one of embodiment 1 to 6 wherein said glycosyltransferase is a fucosyltransferase and said donor nucleotide-sugar is GDP-Fucose (GDP-Fuc).
8. Method according to any one of embodiment 1 to 7 wherein glycosyltransferase is a sialyltransferase and said donor nucleotide-sugar is CMP-N-acetylneuraminic acid (CMP-Neu5Ac).
9. Method according to any one of embodiment 1 to 8 wherein said glycosyltransferase is an N-acetylglucosaminyltransferase and said donor nucleotide-sugar is UDP-N-acetylglucosamine (UDP-GlcNAc).
10. Method according to any one of embodiment 1 to 9 wherein said glycosyltransferase is a galactosyltransferase and said donor nucleotide-sugar is UDP-galactose (UDP-Gal).
11. Method according to any one of embodiment 1 to 10, wherein said nucleotide-sugar is chosen from the list comprising GDP-Fuc, CMP-Neu5Ac, UDP-GlcNAc, UDP-Gal, UDP-N-acetylgalactosamine (UDP-GalNAc), UDP-N-acetylmannosamine (UDP-ManNAc), GDP-mannose (GDP-Man), UDP-glucose (UDP-Glc), CMP-N-glycolylneuraminic acid (CMP-Neu5Gc), UDP-glucuronate, UDP-galacturonate, GDP-rhamnose, UDP-xylose.
12. Method according to any one of embodiments 1 to 11, wherein any one of said acceptors has a degree of polymerization of 3 or more, preferably wherein all of said acceptors have a degree of polymerization of 3 or more.
13. Method according to any one of embodiment 1 to 12, wherein all said acceptors have a different degree of polymerization.
14. Method according to any one of embodiment 1 to 13, wherein said cultivation is supplemented with at least 3 acceptors for the production of said oligosaccharide mixture.
15. Method according to any one of embodiment 1 to 14, wherein said oligosaccharide mixture comprises at least one oligosaccharide that is fucosylated, sialylated, galactosylated, glucosylated, xylosylated, mannosylated, contains an N-acetylglucosamine, contains an N-acetylneuraminate, contains an N-glycolylneuraminate, contains an N-acetylgalactosamine, contains a rhamnose, contains a glucuronate, contains a galacturonate, and/or contains an N-acetylmannosamine.
16. Method according to any one of embodiment 1 to 15, wherein said oligosaccharide mixture comprises at least one fucosylated oligosaccharide.
17. Method according to any one of embodiment 1 to 16, wherein said oligosaccharide mixture comprises at least one sialylated oligosaccharide.
18. Method according to any one of embodiment 1 to 17, wherein said oligosaccharide mixture comprises at least one oligosaccharide that comprises an N-acetylglucosamine monosaccharide unit.
19. Method according to any one of embodiment 1 to 18, wherein said oligosaccharide mixture comprises at least one galactosylated oligosaccharide.
20. Method according to any one of embodiment 1 to 16, wherein all said oligosaccharides in the mixture are fucosylated oligosaccharides.
21. Method according to embodiment 1 to 16 and 20, wherein said cell produces three fucosylated oligosaccharides.
22. Method according to any one of embodiment 1 to 21, wherein said acceptors are produced by methods comprising extraction from natural sources, biotechnological processes, physical processes, chemical processes, and combinations thereof.
23. Method according to any one of embodiment 1 to 22, wherein any one of said acceptors is completely converted into any one of said oligosaccharides.
24. Method according to any one of embodiment 1 to 23, wherein said cell is further metabolically engineered for
   i) modified expression of an endogenous membrane protein, and/or
   ii) modified activity of an endogenous membrane protein, and/or
   iii) expression of a homologous membrane protein, and/or
   iv) expression of a heterologous membrane protein,
   wherein said membrane protein is involved in the secretion of any one of said oligosaccharides from said cell, preferably wherein said membrane protein is involved in the secretion of all of said oligosaccharides from said cell.
25. Method according to any one of embodiment 1 to 24, wherein said cell is further metabolically engineered for
   i) modified expression of an endogenous membrane protein, and/or
   ii) modified activity of an endogenous membrane protein, and/or
   iii) expression of a homologous membrane protein, and/or
   iv) expression of a heterologous membrane protein,
   wherein said membrane protein is involved in the uptake of a precursor for the synthesis of any one of said oligosaccharides of the mixture, preferably wherein said membrane protein is involved in the uptake of all of the required precursors, more preferably wherein said membrane protein is involved in the uptake of all of said acceptors.
26. Method according to any one of embodiment 1 to 25, wherein any one of said oligosaccharides is a mammalian milk oligosaccharide, preferably wherein all said oligosaccharides are mammalian milk oligosaccharides.
27. Method according to any one of embodiment 1 to 25, wherein any one of said oligosaccharide is an antigen of the human ABO blood group system, preferably wherein all said oligosaccharides are antigens of the human ABO blood group system.
28. Method according to any one of embodiment 1 to 27, wherein said cell is selected from the group consisting of microorganism, plant, or animal cells, preferably said microorganism is a bacterium, fungus or a yeast, preferably said plant is a rice, cotton, rapeseed, soy, maize or corn plant, preferably said animal is an insect, fish, bird or non-human mammal, preferably said animal cell is a mammalian cell line.
29. Method according to any one of embodiment 1 to 28, wherein said cell is a cell of a bacterium, preferably of an *Escherichia coli* strain, more preferably of an *Escherichia coli* strain which is a K-12 strain, even more preferably the *Escherichia coli* K-12 strain is *E. coli* MG1655.
30. Method according to any one of embodiment 1 to 28, wherein said cell is a yeast cell.
31. The method according to any one of embodiment 1 to 30, wherein said separation comprises at least one of the following steps: clarification, ultrafiltration, nanofiltration, reverse osmosis, microfiltration, activated charcoal or carbon treatment, tangential flow high-performance filtration, tangential flow ultrafiltration, affinity chromatography, ion exchange chromatography, hydrophobic interaction chromatography and/or gel filtration, ligand exchange chromatography.
32. The method according to any one of embodiment 1 to 31 further comprising purification of any one of said oligosaccharides from said cell.
33. The method according to any one of embodiment 1 to 32, wherein said purification comprises at least one of the following steps: use of activated charcoal or carbon, use of charcoal, nanofiltration, ultrafiltration or ion exchange, use of alcohols, use of aqueous alcohol mixtures, crystallization, evaporation, precipitation, drying, spray drying or lyophilization.
34. Use of a method according to any one of embodiments 1 to 33 for the production of a mixture of at least two different oligosaccharides.

The invention will be described in more detail in the examples. The following examples will serve as further illustration and clarification of the present invention and are not intended to be limiting.

### Examples

### Example 1. Materials and Methods Escherichia coli

### Media

The Luria Broth (LB) medium consisted of 1% tryptone peptone (Difco, Erembodegem, Belgium), 0.5% yeast extract (Difco) and 0.5% sodium chloride (VWR. Leuven, Belgium). The minimal medium used in the cultivation experiments in 96-well plates or in shake flasks contained 2.00 g/L NH4CI, 5.00 g/L (NH4)2SO4, 2.993 g/L KH2PO4, 7.315 g/L K2HPO4, 8.372 g/L MOPS, 0.5 g/L NaCl, 0.5 g/L MgSO4.7H2O, 30 g/L sucrose or 30 g/L glycerol, 1 ml/L vitamin solution, 100 µl/L molybdate solution, and 1 mL/L selenium solution. As specified in the respective examples, 20 g/L lactose, 20 g/L 2'FL, 20 g/L 3-FL, 20 g/L 3'SL, 20 g/L 6'SL, 20 g/L LacNAc and/or 20 g/L LNB were additionally added to the medium as acceptor(s). The minimal medium was set to a pH of 7 with 1M KOH. Vitamin solution consisted of 3.6 g/L FeCl2.4H2O, 5 g/L CaCl2.2H2O, 1.3 g/L MnCl2.2H2O, 0.38 g/L CuCl2.2H2O, 0.5 g/L CoCl2.6H2O, 0.94 g/L ZnCI2, 0.0311 g/L H3BO4, 0.4 g/L Na2EDTA.2H2O and 1.01 g/L thiamine.HCl. The molybdate solution contained 0.967 g/L NaMoO4.2H2O. The selenium solution contained 42 g/L Seo2.
The minimal medium for fermentations contained 6.75 g/L NH4CI, 1.25 g/L (NH4)2SO4, 2.93 g/L KH2PO4 and 7.31 g/L KH2PO4, 0.5 g/L NaCl, 0.5 g/L MgSO4.7H2O, 30 g/L sucrose or 30 g/L glycerol, 1 mL/L vitamin solution, 100 µL/L molybdate solution, and 1 mL/L selenium solution with the same composition as described above. As specified in the respective examples, 20 g/L lactose, 20 g/L 2'FL, 20 g/L 3-FL, 20 g/L 3'SL, 20 g/L LacNAc and/or 20 g/L LNB were additionally added to the medium as acceptor(s).
Complex medium was sterilized by autoclaving (121°C, 21 min) and minimal medium by filtration (0.22 µm Sartorius). When necessary, the medium was made selective by adding an antibiotic: e.g. chloramphenicol (20 mg/L), carbenicillin (100 mg/L), spectinomycin (40 mg/L) and/or kanamycin (50 mg/L).

### Plasmids

pKD46 (Red helper plasmid, Ampicillin resistance), pKD3 (contains an FRT-flanked chloramphenicol resistance (cat) gene), pKD4 (contains an FRT-flanked kanamycin resistance (kan) gene), and pCP20 (expresses FLP recombinase activity) plasmids were obtained from Prof. R. Cunin (Vrije Universiteit Brussel, Belgium in 2007). Plasmids were maintained in the host *E. coli* DH5alpha (F⁻, phi80d/*acZΔ*M15, *Δ*(*lacZYA-argF*) U169, *deoR, recAl, endAl,* hsdR17(rk⁻, mk⁺), *phoA, supE44,* lambda⁻, *thi-1, gyrA96, rel*A1) bought from Invitrogen.

### Strains and mutations

*Escherichia coli* K12 MG1655 [λ⁻, F⁻, rph-1] was obtained from the Coli Genetic Stock Center (US), CGSC Strain#: 7740, in March 2007. Gene disruptions, gene introductions and gene replacements were performed using the technique published by Datsenko and Wanner (PNAS 97 (2000), 6640-6645). This technique is based on antibiotic selection after homologous recombination performed by lambda Red recombinase. Subsequent catalysis of a flippase recombinase ensures removal of the antibiotic selection cassette in the final production strain. Transformants carrying a Red helper plasmid pKD46 were grown in 10 mL LB media with ampicillin, (100 mg/L) and L-arabinose (10 mM) at 30 °C to an OD₆₀₀nm of 0.6. The cells were made electrocompetent by washing them with 50 mL of ice-cold water, a first time, and with 1mL ice cold water, a second time. Then, the cells were resuspended in 50 µL of ice-cold water. Electroporation was done with 50 µL of cells and 10-100 ng of linear double-stranded-DNA product by using a Gene Pulser^{™} (BioRad) (600 Ω, 25 µFD, and 250 volts). After electroporation, cells were added to 1 mL LB media incubated 1 h at 37 °C, and finally spread onto LB-agar containing 25 mg/L of chloramphenicol or 50 mg/L of kanamycin to select antibiotic resistant transformants. The selected mutants were verified by PCR with primers upstream and downstream of the modified region and were grown in LB-agar at 42°C for the loss of the helper plasmid. The mutants were tested for ampicillin sensitivity. The linear ds-DNA amplicons were obtained by PCR using pKD3, pKD4 and their derivates as template. The primers used had a part of the sequence complementary to the template and another part complementary to the side on the chromosomal DNA where the recombination must take place. For the genomic knock-out, the region of homology was designed 50-nt upstream and 50-nt downstream of the start and stop codon of the gene of interest. For the genomic knock-in, the transcriptional starting point (+1) had to be respected. PCR products were PCR-purified, digested with Dpnl, re-purified from an agarose gel, and suspended in elution buffer (5 mM Tris, pH 8.0). Selected mutants were transformed with pCP20 plasmid, which is an ampicillin and chloramphenicol resistant plasmid that shows temperature- sensitive replication and thermal induction of FLP synthesis. The ampicillin-resistant transformants were selected at 30°C, after which a few were colony purified in LB at 42 °C and then tested for loss of all antibiotic resistance and of the FLP helper plasmid. The gene knock outs and knock ins are checked with control primers.
For GDP-fucose production, the mutant strain was derived from *E. coli* K12 MG1655 comprising knock-outs of the *E. coli wcaJ* and *thyA* genes and genomic knock-ins of constitutive transcriptional units containing a sucrose transporter (CscB) from *E. coli* W with SEQ ID NO 01, a fructose kinase (Frk) originating from *Zymomonas mobilis* with SEQ ID NO 02 and a sucrose phosphorylase (SP) originating from *Bifidobacterium adolescentis* with SEQ ID NO 03. For production of fucosylated oligosaccharides, the mutant GDP-fucose production strain was additionally modified with expression plasmids comprising constitutive transcriptional units for the *H. pylori* alpha-1,2-fucosyltransferase with SEQ ID NO 04 (HpFutC) and/or the *H. pylori* alpha-1,3-fucosyltransferase with SEQ ID NO 05 (HpFucT) and with a constitutive transcriptional unit for the *E. coli* thyA with SEQ ID NO 07 as selective marker. The constitutive transcriptional units of the fucosyltransferase genes could also be present in the mutant *E. coli* strain via genomic knock-ins. GDP-fucose production can further be optimized in the mutant *E. coli* strain by genomic knock-outs of the *E. coli* genes comprising *glgC, agp, pfkA, pfkB, pgi, orcA, iclR, pgi* and *Ion* as described in WO2016075243 and WO2012007481. GDP-fucose production can additionally be optimized comprising genomic knock-ins of constitutive transcriptional units for the *E. coli* manA with SEQ ID NO 08, manB with SEQ ID NO 09, manC with SEQ ID NO 10, gmd with SEQ ID NO 11 and fcl with SEQ ID NO 12. GDP-fucose production can also be obtained by genomic knock-outs of the *E. coli fucK* and *fucl* genes and genomic knock-ins of constitutive transcriptional units containing the fucose permease (fucP) from *E. coli* with SEQ ID NO 13 and the bifunctional fucose kinase/fucose-1-phosphate guanylyltransferase (fkp) from *Bacteroides fragilis* with SEQ NO ID 14. If the mutant strains producing GDP-fucose were intended to make fucosylated lactose structures comprising 2'FL, 3-FL and DiFL, the strains were additionally modified with genomic knock-outs of the *E. coli LacZ, LacY* and *LacA* genes and with a genomic knock-in of a constitutive transcriptional unit for the *E. coli* LacY with SEQ ID NO 15.
For sialic acid production, the mutant strain was derived from *E. coli* K12 MG1655 comprising knock-outs of the *E. coli nagA* and *nagB* genes and genomic knock-ins of constitutive transcriptional units containing a glucosamine 6-phosphate N-acetyltransferase (GNA1) from *Saccharomyces cerevisiae* with SEQ ID NO 16, an N-acetylglucosamine 2-epimerase (AGE) from *Bacteroides ovatus* with SEQ ID NO 17 and an N-acetylneuraminate (Neu5Ac) synthase (NeuB) from *Neisseria meningitidis* with SEQ ID NO 18. Sialic acid production can further be optimized in the mutant *E. coli* strain with genomic knock-outs of the *E. coli* genes comprising *nagC, nagD, nagE, nanA, nanE, nanK, manX, manY* and *manZ* as described in WO18122225 and with genomic knock-ins of constitutive transcriptional units comprising a mutated variant of the L-glutamine-D-fructose-6-phosphate aminotransferase (glmS*54) from *E. coli* with SEQ ID NO 19 (encoding the glmS*54 protein differing from the wild-type *E. coli* glmS protein by an A39T, an R250C and an G472S mutation) and the phosphatase (yqaB) from *E. coli* with SEQ ID NO 20. Sialic acid production can also be obtained by knock-outs of the *E. coli nagA* and *nagB* genes and genomic knock-ins of constitutive transcriptional units containing the phosphoglucosamine mutase (glmM) from *E. coli* with SEQ ID NO 31, the N-acetylglucosamine-1-phosphate uridyltransferase/glucosamine-1-phosphate acetyltransferase (glmU) from *E. coli* with SEQ ID NO 32, the UDP-N-acetylglucosamine 2-epimerase (NeuC) from *Campylobacter jejuni* with SEQ ID NO 21 and the N-acetylneuraminate synthase (NeuB) from *N. meningitidis* with SEQ ID NO 18. Also in this mutant strain, sialic acid production can further be optimized with genomic knock-ins of constitutive transcriptional units comprising the mutant glmS*54 from *E. coli* with SEQ ID NO 19 and the phosphatase yqaB from *E. coli* with SEQ ID NO 20. For sialylated oligosaccharide production, the sialic acid production strains further need to express an N-acylneuraminate cytidylyltransferase (NeuA) from *Pasteurella multocida* with SEQ ID NO 22, and a beta-galactoside alpha-2,3-sialyltransferase comprising SEQ ID NO 23 (PmultST3) from *P. multocida* and/or a beta-galactoside alpha-2,6-sialyltransferase comprising SEQ ID NO 24 (PdST6) from *Photobacterium damselae.* Constitutive transcriptional units of the PmNeuA and the sialyltransferases can be delivered to the mutant strain either via genomic knock-in or via expression plasmids. If the mutant strains producing sialic acid and CMP-sialic acid were intended to make sialylated lactose structures, the strains were additionally modified with genomic knock-outs of the *E. coli LacZ, LacYand LacA* genes and with a genomic knock-in of a constitutive transcriptional unit for the *E. coli* LacY with SEQ ID NO 15. All mutant strains producing sialic acid, CMP-sialic acid and/or sialylated oligosaccharides could optionally be adapted for growth on sucrose via genomic knock-ins of constitutive transcriptional units containing a sucrose transporter (CscB) from *E. coli* W with SEQ ID NO 01, a fructose kinase (Frk) originating from Z. *mobilis* with SEQ ID NO 02 and a sucrose phosphorylase originating from *B. adolescentis* with SEQ ID NO 03.
To produce LN3 (GlcNAc-b1,3-Gal-b1,4-Glc) and oligosaccharides originating thereof comprising lacto-*N-*tetraose (LNT) and lacto-*N*-neotetraose (LNnT), the mutant strain was derived from *E. coli* K12 MG1655 and modified with a knock-out of the *E. coli LacZ* and *nagB* genes and with a genomic knock-in of a constitutive transcriptional unit for the galactoside beta-1,3-N-acetylglucosaminyltransferase (LgtA) from *N. meningitidis* with SEQ ID NO 25. For LNT or LNnT production, the mutant strain is further modified with constitutive transcriptional units for the N-acetylglucosamine beta-1,3-galactosyltransferase (WbgO) from *E. coli* O55:H7 with SEQ ID NO 26 or the N-acetylglucosamine beta-1,4-galactosyltransferase (LgtB) from *N. meningitidis* with SEQ ID NO 27, respectively, that can be delivered to the strain either via genomic knock-in or from an expression plasmid. Optionally, multiple copies of the *LgtA, wbgO* and/or *LgtB* genes could be added to the mutant *E. coli* strains. Also, LNT and/or LNnT production can be enhanced by improved UDP-GlcNAc production by modification of the strains with one or more genomic knock-ins of a constitutive transcriptional unit for the mutant L-glutamine-D-fructose-6-phosphate aminotransferase glmS*54 from *E. coli* with SEQ ID NO 19. In addition, the strains can optionally be modified for enhanced UDP-galactose production with genomic knock-outs of the *E. coli ushA* and *galT* genes. The mutant *E. coli* strains can also optionally be adapted with a genomic knock-in of a constitutive transcriptional unit for the UDP-glucose-4-epimerase (galE) from *E. coli* with SEQ ID NO 28, the phosphoglucosamine mutase (glmM) from *E. coli* with SEQ ID NO 31 and the N-acetylglucosamine-1-phosphate uridyltransferase / glucosamine-1-phosphate acetyltransferase (glmU) from *E. coli* with SEQ ID NO 32. The mutant strains could also optionally be adapted for growth on sucrose via genomic knock-ins of constitutive transcriptional units containing a sucrose transporter (CscB) from *E. coli* W with SEQ ID NO 01, a fructose kinase (Frk) originating from Z. *mobilis* with SEQ ID NO 02 and a sucrose phosphorylase originating from *B. adolescentis* with SEQ ID NO 03.
Preferably but not necessarily, the glycosyltransferases were N-terminally fused to an MBP-tag to enhance their solubility (Fox et al., Protein Sci. 2001, 10(3), 622-630).
All constitutive promoters, UTRs and terminator sequences originated from the libraries described by Cambray et al. (Nucleic Acids Res. 2013, 41(9), 5139-5148), De Mey et al. (BMC Biotechnol. 2007, 4(34), 1-14), Dunn et al. (Nucleic Acids Res. 1980, 8(10), 2119-2132), Kim and Lee (FEBS Letters 1997, 407(3), 353-356) and Mutalik et al. (Nat. Methods 2013, No. 10, 354-360).
All genes were ordered synthetically atTwist Bioscience (twistbioscience.com) or IDT (eu.idtdna.com) and the codon usage was adapted using the tools of the supplier.
All strains were stored in cryovials at -80°C (overnight LB culture mixed in a 1:1 ratio with 70% glycerol).

### Cultivation conditions

A preculture of 96-well microtiter plate experiments was started from a cryovial, in 150 µL LB and was incubated overnight at 37 °C on an orbital shaker at 800 rpm. This culture was used as inoculum for a 96well square microtiter plate, with 400 µL minimal medium by diluting 400x. These final 96-well culture plates were then incubated at 37°C on an orbital shaker at 800 rpm for 72h, or shorter, or longer. To measure sugar concentrations at the end of the cultivation experiment whole broth samples were taken from each well by boiling the culture broth for 15 min at 60°C before spinning down the cells (= average of intra- and extracellular sugar concentrations).
A preculture for the bioreactor was started from an entire 1 mL cryovial of a certain strain, inoculated in 250 mL or 500 mL minimal medium in a 1 L or 2.5 L shake flask and incubated for 24 h at 37°C on an orbital shaker at 200 rpm. A 5 L bioreactor was then inoculated (250 mL inoculum in 2 L batch medium); the process was controlled by MFCS control software (Sartorius Stedim Biotech, Melsungen, Germany). Culturing condition were set to 37 °C, and maximal stirring; pressure gas flow rates were dependent on the strain and bioreactor. The pH was controlled at 6.8 using 0.5 M H2S04 and 20% NH4OH. The exhaust gas was cooled. 10% solution of silicone antifoaming agent was added when foaming raised during the fermentation.

### Optical density

Cell density of the cultures was frequently monitored by measuring optical density at 600 nm (Implen Nanophotometer NP80, Westburg, Belgium or with a Spark 10M microplate reader, Tecan, Switzerland).

### Analytical analysis

Standards such as but not limited to sucrose, lactose, LacNAc, lacto-N-biose (LNB), fucosylated LacNAc (2'FLacNAc, 3-FLacNAc), sialylated LacNAc, (3'SLacNAc, 6'SLacNAc), fucosylated LNB (2'FLNB, 4'FLNB), lacto-*N*-triose II (LN3), lacto-*N*-tetraose (LNT), lacto-*N*-neo-tetraose (LNnT), LNFP-I, LNFP-II, LNFP-III, LNFP-V, LNFP-VI, LSTa and LSTc were purchased from Carbosynth (UK), Elicityl (France) and IsoSep (Sweden). Other compounds were analyzed with in-house made standards.
Neutral oligosaccharides were analyzed on a Waters Acquity H-class UPLC with Evaporative Light Scattering Detector (ELSD) or a Refractive Index (RI) detection. A volume of 0.7 µL sample was injected on a Waters Acquity UPLC BEH Amide column (2.1 x 100 mm;130 Å;1.7 µm) column with an Acquity UPLC BEH Amide VanGuard column, 130 Å, 2.1x 5 mm. The column temperature was 50 °C. The mobile phase consisted of a ¼ water and % acetonitrile solution to which 0.2 % triethylamine was added. The method was isocratic with a flow of 0.130 mL/min. The ELS detector had a drift tube temperature of 50 °C and the N2 gas pressure was 50 psi, the gain 200 and the data rate 10 pps. The temperature of the RI detector was set at 35 °C.
Sialylated oligosaccharides were analyzed on a Waters Acquity H-class UPLC with Refractive Index (RI) detection. A volume of 0. 5 µL sample was injected on a Waters Acquity UPLC BEH Amide column (2.1 x 100 mm;130 Å;1.7 µm). The column temperature was 50 °C. The mobile phase consisted of a mixture of 70 % acetonitrile, 26 % ammonium acetate buffer (150 mM) and 4 % methanol to which 0.05 % pyrrolidine was added. The method was isocratic with a flow of 0.150 mL/min. The temperature of the RI detector was set at 35 °C.
Both neutral and sialylated sugars were analyzed on a Waters Acquity H-class UPLC with Refractive Index (RI) detection. A volume of 0.5 µL sample was injected on a Waters Acquity UPLC BEH Amide column (2.1 x 100 mm;130 Å;1.7 µm). The column temperature was 50°C. The mobile phase consisted of a mixture of 72% acetonitrile and 28% ammonium acetate buffer (100 mM) to which 0.1% triethylamine was added. The method was isocratic with a flow of 0.260 mL/min. The temperature of the RI detector was set at 35 °C.
For analysis on a mass spectrometer, a Waters Xevo TQ-MS with Electron Spray lonisation (ESI) was used with a desolvation temperature of 450 °C, a nitrogen desolvation gas flow of 650 L/h and a cone voltage of 20 V. The MS was operated in selected ion monitoring (SIM) in negative mode for all oligosaccharides. Separation was performed on a Waters Acquity UPLC with a Thermo Hypercarb column (2.1 x 100 mm; 3 µm) on 35 °C. A gradient was used wherein eluent A was ultrapure water with 0.1 % formic acid and wherein eluent B was acetonitrile with 0.1 % formic acid. The oligosaccharides were separated in 55 min using the following gradient: an initial increase from 2 to 12 % of eluent B over 21 min, a second increase from 12 to 40 % of eluent B over 11 min and a third increase from 40 to 100 % of eluent B over 5 min. As a washing step 100 % of eluent B was used for 5 min. For column equilibration, the initial condition of 2 % of eluent B was restored in 1 min and maintained for 12 min.
Both neutral and sialylated sugars at low concentrations (below 50 mg/L) were analyzed on a Dionex HPAEC system with pulsed amperometric detection (PAD). A volume of 5 µL of sample was injected on a Dionex CarboPac PA200 column 4 x 250 mm with a Dionex CarboPac PA200 guard column 4 x 50 mm. The column temperature was set to 30 °C. A gradient was used wherein eluent A was deionized water, wherein eluent B was 200 mM Sodium hydroxide and wherein eluent C was 500 mM Sodium acetate. The oligosaccharides were separated in 60 min while maintaining a constant ratio of 25 % of eluent B using the following gradient: an initial isocratic step maintained for 10 min of 75 % of eluent A, an initial increase from 0 to 4 % of eluent C over 8 min, a second isocratic step maintained for 6 min of 71 % of eluent A and 4 % of eluent C, a second increase from 4 to 12 % of eluent C over 2.6 min, a third isocratic step maintained for 3.4 min of 63 % of eluent A and 12 % of eluent C and a third increase from 12 to 48 % of eluent C over 5 min. As a washing step 48 % of eluent C was used for 3 min. For column equilibration, the initial condition of 75 % of eluent A and 0 % of eluent C was restored in 1 min and maintained for 11 min. The applied flow was 0.5 mL/min.

### Example 2. Materials and Methods Saccharomyces cerevisiae

### Media

Strains were grown on Synthetic Defined yeast medium with Complete Supplement Mixture (SD CSM) or CSM drop-out (SD CSM-Ura, SD CSM-Trp, SD CSM-His) containing 6.7 g/L Yeast Nitrogen Base without amino acids (YNB w/o AA, Difco), 20 g/L agar (Difco) (solid cultures), 22 g/L glucose monohydrate or 20 g/L lactose, 20 g/L 2'FL, 20 g/L 3-FL, 20 g/L 3'SL, 20 g/L 6'SL, 20 g/L LacNAc and/or 20 g/L LNB and 0.79 g/L CSM or 0.77 g/L CSM-Ura, 0.77 g/L CSM-Trp, or 0.77 g/L CSM-His (MP Biomedicals).

### Strains

*S. cerevisiae* BY4742 created by Brachmann et al. (Yeast (1998) 14:115-32) was used, available in the Euroscarf culture collection. All mutant strains were created by homologous recombination or plasmid transformation using the method of Gietz (Yeast 11:355-360, 1995).

### Plasmids

To produce GDP-fucose, the yeast expression plasmid p2a_2p_Fuc (Chan 2013, Plasmid 70, 2-17) was used for expression of foreign genes in *S. cerevisiae.* This plasmid contained an ampicillin resistance gene and a bacterial origin of replication to allow for selection and maintenance in *E. coli* and the 2µ yeast ori and the Ura3 selection marker for selection and maintenance in yeast. This plasmid further contained constitutive transcriptional units for the lactose permease (LAC12) from *Kluyveromyces lactis* with SEQ ID NO 29, the GDP-mannose 4,6-dehydratase (gmd) from *E. coli* with SEQ ID NO 11 and the GDP-L-fucose synthase (fcl) from *E. coli with* SEQ ID NO 12. The yeast expression plasmid p2a_2p_Fuc2 was used as an alternative expression plasmid of the p2a_2p_Fuc plasmid comprising next to the ampicillin resistance gene, the bacterial ori, the 2µ yeast ori and the Ura3 selection marker constitutive transcriptional units for the lactose permease (LAC12) from *K. lactis* with SEQ ID NO 29, fucP from *E. coli* with SEQ ID NO 13 and fkp from *B. fragilis* with SEQ NO ID 14. To further produce fucosylated oligosaccharides, the p2a_2p_Fuc and its variant the p2a_2p_Fuc2, additionally contained (a) constitutive transcriptional unit(s) for one or more fucosyltransferases comprising SEQ ID NOs 04 and 05.
To produce sialic acid and CMP-sialic acid, a yeast expression plasmid was derived from the pRS420-plasmid series (Christianson et al., 1992, Gene 110: 119-122) containing the TRP1 selection marker and constitutive transcriptional units for the mutant glmS*54 from *E. coli* with SEQ ID NO 19, the phosphatase yqaB from *E. coli with* SEQ ID NO 20, the N-acetylglucosamine 2-epimerase (AGE) from *B. ovatus* with SEQ ID NO 17, the N-acetylneuraminate synthase (NeuB) from *N. meningitidis* with SEQ ID NO 18 and the N-acylneuraminate cytidylyltransferase (NeuA) from *P. multocida* with SEQ ID NO 22. Optionally, a constitutive transcriptional unit for GNA1 from *S. cerevisiae* with SEQ ID NO 16 was added as well. To produce sialylated oligosaccharides, the plasmid further comprised constitutive transcriptional units for the lactose permease (LAC12) from *K. lactis* with SEQ ID NO 29, and one or more sialyltransferases comprising SEQ ID NOs 23 and 24.
To produce UDP-galactose, a yeast expression plasmid was derived from the pRS420-plasmid series (Christianson et al., 1992, Gene 110: 119-122) containing the HIS3 selection marker and a constitutive transcriptional unit for galE from *E. coli* with SEQ ID NO 30. To produce LN3 and LN3-derived oligosaccharides like LNT or LNnT, this plasmid was further modified with constitutive transcriptional units for LAC12 from *K. lactis* with SEQ ID NO 29, the galactoside beta-1,3-N-acetylglucosaminyltransferase (IgtA) from *N. meningitidis* with SEQ ID NO 25 and, the N-acetylglucosamine beta-1,3-galactosyltransferase (WbgO) from *E. coli* O55:H7 with SEQ ID NO 26 or the N-acetylglucosamine beta-1,4-galactosyltransferase (IgtB) from *N. meningitidis* with SEQ ID NO 27, respectively.
Preferably but not necessarily, the glycosyltransferases were N-terminally fused to a SUMOstar tag (e.g. obtained from pYSUMOstar, Life Sensors, Malvern, PA) to enhance their solubility.
Plasmids were maintained in the host *E. coli* DH5alpha (F⁻, phi80d/*acZ*deltaM15, delta(*lacZYA-argF*)U169, *deoR, recAl, endAl,* hsdR17(rk⁻, mk⁺), *phoA, supE44,* lambda⁻, *thi-1, gyrA96, rel*A1) bought from Invitrogen.

### Heterologous and homologous expression

Genes that needed to be expressed, be it from a plasmid or from the genome were synthetically synthetized with one of the following companies: DNA2.0, Gen9, IDT or Twist Bioscience. Expression could be further facilitated by optimizing the codon usage to the codon usage of the expression host. Genes were optimized using the tools of the supplier.

### Cultivations conditions

In general, yeast strains were initially grown on SD CSM plates to obtain single colonies. These plates were grown for 2-3 days at 30 °C. Starting from a single colony, a preculture was grown over night in 5 mL at 30 °C, shaking at 200 rpm. Subsequent 125 mL shake flask experiments were inoculated with 2% of this preculture, in 25 mL media. These shake flasks were incubated at 30 °C with an orbital shaking of 200 rpm.

### Gene expression promoters

Genes were expressed using synthetic constitutive promoters, as described by Blazeck (Biotechnology and Bioengineering, Vol. 109, No. 11, 2012).

### Example 3. Production of an oligosaccharide mixture comprising 2'FL and DiFL with a modified E. coli host using lactose and 3-FL as acceptors

An *E. coli* K12 strain modified for GDP-fucose production as described in Example 1 was transformed with a plasmid expressing a constitutive transcriptional unit for the *H. pylori* alpha-1,2-fucosyltransferase with SEQ ID NO 04. The novel strain was evaluated in a growth experiment according to the culture conditions provided in Example 1, in which the culture medium contained sucrose as carbon source and lactose and 3-FL as acceptors. Each strain was grown in four biological replicates in a 96-well plate. After 72h of incubation, the culture broth was harvested, and the sugar mixtures were analyzed on UPLC. The experiment demonstrated the novel strain produced an oligosaccharide mixture comprising 2'FL and 2',-3-fucosyllactose (DiFL) in whole broth samples.

### Example 4. Production of an oligosaccharide mixture comprising 2'FL, DiFL and 2'FLacNAc with a modified E. coli host using lactose and LacNAc as acceptors

An *E. coli* K12 strain modified for GDP-fucose production as described in Example 1 was transformed with a plasmid expressing a constitutive transcriptional unit for the *H. pylori* alpha-1,2-fucosyltransferase with SEQ ID NO 04. The novel strain produces an oligosaccharide mixture comprising 2'FL, DiFL and 2'-fucosylated N-acetyllactosamine (2'FLacNAc) in whole broth samples, when evaluated in a growth experiment according to the culture conditions provided in Example 1, in which the culture medium contains sucrose as carbon source and lactose and N-acetyllactosamine (Gal-b1,4-GlcNAc, LacNAc) as acceptors.

### Example 5. Production of an oligosaccharide mixture comprising 2'FL, 3-FL, DiFL, 2'FLacNAc, 3-FLacNAc and DiFLacNAc with a modified E. coli host using lactose and LacNAc as acceptors

An *E. coli* K12 strain modified for GDP-fucose production as described in Example 1 was transformed with a plasmid expressing constitutive transcriptional units for the *H. pylori* alpha-1,2-fucosyltransferase with SEQ ID NO 04 and the *H. pylori* alpha-1,3-fucosyltransferase with SEQ ID NO 05. The novel strain produces an oligosaccharide mixture comprising 2'FL, 3-FL and fucosylated LacNAc (i.e. 2'FLacNAc and 3-FLacNAc) in whole broth samples, when evaluated in a growth experiment according to the culture conditions provided in Example 1, in which the culture medium contains sucrose as carbon source and lactose and LacNAc as acceptors. Since the enzyme with SEQ ID NO 04 also shows fucosyltransferase activity on 2'FL and the enzyme with SEQ ID NO 05 also shows fucosyltransferase activity on 2'FLacNAc the novel strain also produces DiFL and Di-FLacNAc in said oligosaccharide mixture.

### Example 6. Production of an oligosaccharide mixture comprising 3-FL and 3-FLocNAc with a modified E. coli host using lactose and LacNAc as acceptors

An *E. coli* K12 strain modified for GDP-fucose production as described in Example 1 was transformed with a plasmid expressing a constitutive transcriptional unit for the *H. pylori* alpha-1,3-fucosyltransferase with SEQ ID NO 05. The novel strain produces an oligosaccharide mixture comprising 3-FL and 3-fucosylated N-acetyllactosamine (3'FLacNAc) in whole broth samples, when evaluated in a growth experiment according to the culture conditions provided in Example 1, in which the culture medium contains sucrose as carbon source and lactose and LacNAc as acceptors.

### Example 7. Production of an oligosaccharide mixture comprising 2'FL, DiFL and 2'FLNB with a modified E. coli host using lactose and LNB as acceptors

An *E. coli* K12 strain modified for GDP-fucose production as described in Example 1 was transformed with a plasmid expressing a constitutive transcriptional unit for the *H. pylori* alpha-1,2-fucosyltransferase with SEQ ID NO 04. The novel strain produces an oligosaccharide mixture comprising 2'FL, DiFL and 2'-fucosylated lacto-N-biose (2'FLNB) in whole broth samples, when evaluated in a growth experiment according to the culture conditions provided in Example 1, in which the culture medium contains sucrose as carbon source and lactose and lacto-N-biose (Gal-b1,3-GlcNAc, LNB) as acceptors.

### Example 8. Production of an oligosaccharide mixture comprising 2'FL, 3-FL, DiFL, 2'FLNB, 4-FLNB and DiFLNB with a modified E. coli host using lactose and LNB as acceptors

An *E. coli* K12 strain modified for GDP-fucose production as described in Example 1 was transformed with a plasmid expressing constitutive transcriptional units for the *H. pylori* alpha-1,2-fucosyltransferase with SEQ ID NO 04 and the *H. pylori* alpha-1,3-fucosyltransferase with SEQ ID NO 05. Since lactose and LNB are suitable acceptors for both *H. pylori* fucosyltransferases, the novel strain produces an oligosaccharide mixture comprising 2'FL, 3-FL and fucosylated LNB (i.e. 2'FLNB and 4-FLNB) in whole broth samples when evaluated in a growth experiment according to the culture conditions provided in Example 1, in which the culture medium contains sucrose as carbon source and lactose and LNB as acceptors. Since the enzyme with SEQ ID NO 04 also shows fucosyltransferase activity on 2'FL and the enzyme with SEQ ID NO 05 also shows fucosyltransferase activity on 2'FLNB the novel strain also produces DiFL and difucosylated LNB (DiFLNB) in said oligosaccharide mixture.

### Example 9. Production of an oligosaccharide mixture comprising 3-FL and 4-FLNB with a modified E. coli host using lactose and LNB as acceptors

An *E. coli* K12 strain modified for GDP-fucose production as described in Example 1 was transformed with a plasmid expressing a constitutive transcriptional unit for the *H. pylori* alpha-1,3-fucosyltransferase with SEQ ID NO 05. The novel strain produces an oligosaccharide mixture comprising 3-FL and 4-fucosylated lacto-N-biose (4-FLNB) in whole broth samples, when evaluated in a growth experiment according to the culture conditions provided in Example 1, in which the culture medium contains sucrose as carbon source and lactose and LNB as acceptors.

### Example 10. Production of an oligosaccharide mixture comprising 2'FLacNAc and 2'FLNB with a modified E. coli host using LacNAc and LNB as acceptors

An *E. coli* K12 strain modified for GDP-fucose production as described in Example 1 was transformed with a plasmid expressing a constitutive transcriptional unit for the *H. pylori* alpha-1,2-fucosyltransferase with SEQ ID NO 04. The novel strain produces an oligosaccharide mixture comprising 2'FLacNAc and 2'FLNB in whole broth samples, when evaluated in a growth experiment according to the culture conditions provided in Example 1, in which the culture medium contains sucrose as carbon source and LacNAc and LNB as acceptors.

### Example 11. Production of an oligosaccharide mixture comprising 3'FLacNAc and 4-FLNB with a modified E. coli host using LacNAc and LNB as acceptors

An *E. coli* K12 strain modified for GDP-fucose production as described in Example 1 was transformed with a plasmid expressing a constitutive transcriptional unit for the *H. pylori* alpha-1,3-fucosyltransferase with SEQ ID NO 05. The novel strain produces an oligosaccharide mixture comprising 3-FLacNAc and 4-FLNB in whole broth samples, when evaluated in a growth experiment according to the culture conditions provided in Example 1, in which the culture medium contains sucrose as carbon source and LacNAc and LNB as acceptors.

### Example 12. Production of an oligosaccharide mixture comprising 2'FLacNAc, 3-FLacNAc, DiFLacNAc, 2'FLNB, 4-FLNB and DiFLNB with a modified E. coli host using LacNAc and LNB as acceptors

An *E. coli* K12 strain modified for GDP-fucose production as described in Example 1 was transformed with a plasmid expressing constitutive transcriptional units for the *H. pylori* alpha-1,2-fucosyltransferase with SEQ ID NO 04 and the *H. pylori* alpha-1,3-fucosyltransferase with SEQ ID NO 05. The novel strain produces an oligosaccharide mixture comprising 2'FLacNAc, 3-FLacNAc, DiFLacNAc, 2'FLNB, 4-FLNB and DiFLNB in whole broth samples, when evaluated in a growth experiment according to the culture conditions provided in Example 1, in which the culture medium contains sucrose as carbon source and LacNAc and LNB as acceptors.

### Example 13. Production of an oligosaccharide mixture comprising 2'FL, DiFL, 2'FLacNAc and 2'FLNB with a modified E. coli host using lactose, LacNAc and LNB as acceptors

An *E. coli* K12 strain modified for GDP-fucose production as described in Example 1 was transformed with a plasmid expressing a constitutive transcriptional unit for the *H. pylori* alpha-1,2-fucosyltransferase with SEQ ID NO 04. The novel strain produces an oligosaccharide mixture comprising 2'FL, DiFL, 2'FLacNAc and 2'FLNB in whole broth samples, when evaluated in a growth experiment according to the culture conditions provided in Example 1, in which the culture medium contains sucrose as carbon source and lactose, LacNAc and LNB as acceptors.

### Example 14. Production of an oligosaccharide mixture comprising 3-FL, 3'FLacNAc and 4-FLNB with a modified E. coli host using lactose, LacNAc and LNB as acceptors

An *E. coli* K12 strain modified for GDP-fucose production as described in Example 1 was transformed with a plasmid expressing a constitutive transcriptional unit for the *H. pylori* alpha-1,3-fucosyltransferase with SEQ ID NO 05. The novel strain produces an oligosaccharide mixture comprising 3-FL, 3-FLacNAc and 4-FLNB in whole broth samples, when evaluated in a growth experiment according to the culture conditions provided in Example 1, in which the culture medium contains sucrose as carbon source and lactose, LacNAc and LNB as acceptors.

### Example 15. Production of an oligosaccharide mixture comprising 2'FL, 3-FL, DiFL, 2'FLacNAc, 3-FLacNAc, DiFLacNAc, 2'FLNB, 4-FLNB and DiFLNB with a modified E. coli host using lactose, LacNAc and LNB as acceptors

An *E. coli* K12 strain modified for GDP-fucose production as described in Example 1 was transformed with a plasmid expressing constitutive transcriptional units for the *H. pylori* alpha-1,2-fucosyltransferase with SEQ ID NO 04 and the *H. pylori* alpha-1,3-fucosyltransferase with SEQ ID NO 05. The novel strain produces an oligosaccharide mixture comprising 2'FL, 3-FL, DiFL, 2'FLacNAc, 3-FLacNAc, DiFLacNAc, 2'FLNB, 4-FLNB and DiFLNB in whole broth samples, when evaluated in a growth experiment according to the culture conditions provided in Example 1, in which the culture medium contains sucrose as carbon source and lactose, LacNAc and LNB as acceptors.

### Example 16. Production of an oligosaccharide mixture comprising 3'SL and 3'SLacNAc with a modified E. coli host using lactose and LacNAc as acceptors

An *E. coli* K12 strain modified for sialic acid production as described in Example 1 was transformed with a plasmid expressing constitutive transcriptional units for the α-2,3-sialyltransferase from *P. multocida* with SEQ ID NO 23 and the N-acylneuraminate cytidylyltransferase (NeuA) from *P. multocida* with SEQ ID NO 22. The novel strain produces an oligosaccharide mixture comprising 3'SL and sialylated LacNAc (3'SLacNAc) in whole broth samples, when evaluated in a growth experiment according to the culture conditions provided in Example 1, in which the culture medium contains glycerol as carbon source and lactose and LacNAc as acceptors.

### Example 17. Production of an oligosaccharide mixture comprising 3'SL, 6'SL, 3'SLOcNAc and 6'SLacNAc with a modified E. coli host using lactose and LacNAc as acceptors

An *E. coli* K12 strain modified for sialic acid production as described in Example 1 was transformed with a plasmid expressing constitutive transcriptional units for the α-2,3-sialyltransferase from *P. multocida* with SEQ ID NO 23, the alpha-2,6-sialyltransferase from *P. damselae* with SEQ ID NO 24 and the N-acylneuraminate cytidylyltransferase (NeuA) from *P. multocida* with SEQ ID NO 22. The novel strain produces an oligosaccharide mixture comprising 3'SL, 6'SL and sialylated LacNAc (i.e. 3'SLacNAc and 6'SLacNAc) in whole broth samples, when evaluated in a growth experiment according to the culture conditions provided in Example 1, in which the culture medium contains glycerol as carbon source and lactose and LacNAc as acceptors.

### Example 18. Production of an oligosaccharide mixture comprising 6'SL and 6'SLacNAc with a modified E. coli host using lactose and LacNAc as acceptors

An *E. coli* K12 strain modified for sialic acid production as described in Example 1 was transformed with a plasmid expressing constitutive transcriptional units for the alpha-2,6-sialyltransferase from *P. damselae* with SEQ ID NO 24 and NeuA from *P. multocida* with SEQ ID NO 22. The novel strain produces an oligosaccharide mixture comprising 6'SL and sialylated LacNAc (6'SLacNAc) in whole broth samples, when evaluated in a growth experiment according to the culture conditions provided in Example 1, in which the culture medium contains glycerol as carbon source and lactose and LacNAc as acceptors.

### Example 19. Production of an oligosaccharide mixture comprising 3'SL and 3'SLNB with a modified E. coli host using lactose and LNB as acceptors

An *E. coli* K12 strain modified for sialic acid production as described in Example 1 was transformed with a plasmid expressing constitutive transcriptional units for the α-2,3-sialyltransferase from *P. multocida* with SEQ ID NO 23 and NeuA from *P. multocida* with SEQ ID NO 22. The novel strain produces an oligosaccharide mixture comprising 3'SL and sialylated LNB (3'SLNB) in whole broth samples, when evaluated in a growth experiment according to the culture conditions provided in Example 1, in which the culture medium contains glycerol as carbon source and lactose and LNB as acceptors.

### Example 20. Production of an oligosaccharide mixture comprising 3'SL, 6'SL, 3'SLNB and 6'SLNB with a modified E. coli host using lactose and LNB as acceptors

An *E. coli* K12 strain modified for sialic acid production as described in Example 1 was transformed with a plasmid expressing constitutive transcriptional units for the α-2,3-sialyltransferase from *P. multocida* with SEQ ID NO 23, the alpha-2,6-sialyltransferase from *P. damselae* with SEQ ID NO 24 and NeuA from *P*. *multocida* with SEQ ID NO 22. The novel strain produces an oligosaccharide mixture comprising 3'SL, 6'SL and sialylated LNB (i.e. 3'SLNB and 6'SLNB) in whole broth samples, when evaluated in a growth experiment according to the culture conditions provided in Example 1, in which the culture medium contains glycerol as carbon source and lactose and LNB as acceptors.

### Example 21. Production of an oligosaccharide mixture comprising 6'SL and 6'SLNB with a modified E. coli host using lactose and LNB as acceptors

An *E. coli* K12 strain modified for sialic acid production as described in Example 1 was transformed with a plasmid expressing constitutive transcriptional units for the alpha-2,6-sialyltransferase from *P. damselae* with SEQ ID NO 24 and NeuA from *P. multocida* with SEQ ID NO 22. The novel strain produces an oligosaccharide mixture comprising 6'SL and sialylated LNB (6'SLNB) in whole broth samples, when evaluated in a growth experiment according to the culture conditions provided in Example 1, in which the culture medium contains glycerol as carbon source and lactose and LNB as acceptors.

### Example 22. Production of an oligosaccharide mixture comprising 3'SLacNAc and 3'SLNB with a modified E. coli host using LacNAc and LNB as acceptors

An *E. coli* K12 strain modified for sialic acid production as described in Example 1 was transformed with a plasmid expressing constitutive transcriptional units for the α-2,3-sialyltransferase from *P. multocida* with SEQ ID NO 23 and NeuA from *P. multocida* with SEQ ID NO 22. The novel strain produces an oligosaccharide mixture comprising 3'SLacNAc and 3'SLNB in whole broth samples, when evaluated in a growth experiment according to the culture conditions provided in Example 1, in which the culture medium contains glycerol as carbon source and LacNAc and LNB as acceptors.

### Example 23. Production of an oligosaccharide mixture comprising 6'SLacNAc and 6'SLNB with a modified E. coli host using LacNAc and LNB as acceptors

An *E. coli* K12 strain modified for sialic acid production as described in Example 1 was transformed with a plasmid expressing constitutive transcriptional units for the α-2,6-sialyltransferase from *P. damselae* with SEQ ID NO 24 and NeuA from *P. multocida* with SEQ ID NO 22. The novel strain produces an oligosaccharide mixture comprising 6'SLacNAc and 6'SLNB in whole broth samples, when evaluated in a growth experiment according to the culture conditions provided in Example 1, in which the culture medium contains glycerol as carbon source and LacNAc and LNB as acceptors.

### Example 24. Production of an oligosaccharide mixture comprising 3'SLacNAc, 6'SLacNAc, 3'SLNB and 6'SLNB with a modified E. coli host using LacNAc and LNB as acceptors

An *E. coli* K12 strain modified for sialic acid production as described in Example 1 was transformed with a plasmid expressing constitutive transcriptional units for the α-2,3-sialyltransferase from *P. multocida* with SEQ ID NO 23, the α-2,6-sialyltransferase from *P. damselae* with SEQ ID NO 24 and NeuA from *P. multocida* with SEQ ID NO 22. The novel strain produces an oligosaccharide mixture comprising 3'SLacNAc, 6'SLacNAc, 3'SLNB and 6'SLNB in whole broth samples, when evaluated in a growth experiment according to the culture conditions provided in Example 1, in which the culture medium contains glycerol as carbon source and LacNAc and LNB as acceptors.

### Example 25. Production of an oligosaccharide mixture comprising 3'SL, 3'SLacNAc and 3'SLNB with a modified E. coli host using lactose, LacNAc and LNB as acceptors

An *E. coli* K12 strain modified for sialic acid production as described in Example 1 was transformed with a plasmid expressing constitutive transcriptional units for the α-2,3-sialyltransferase from *P. multocida* with SEQ ID NO 23 and NeuA from *P. multocida* with SEQ ID NO 22. The novel strain produces an oligosaccharide mixture comprising 3'SL, 3'SLacNAc and 3'SLNB in whole broth samples, when evaluated in a growth experiment according to the culture conditions provided in Example 1, in which the culture medium contains glycerol as carbon source and lactose, LacNAc and LNB as acceptors.

### Example 26. Production of an oligosaccharide mixture comprising 6'SL, 6'SLacNAc and 6'SLNB with a modified E. coli host using lactose, LacNAc and LNB as acceptors

An *E. coli* K12 strain modified for sialic acid production as described in Example 1 was transformed with a plasmid expressing constitutive transcriptional units for the α-2,6-sialyltransferase from *P. damselae* with SEQ ID NO 24 and NeuA from *P. multocida* with SEQ ID NO 22. The novel strain produces an oligosaccharide mixture comprising 6'SL, 6'SLacNAc and 6'SLNB in whole broth samples, when evaluated in a growth experiment according to the culture conditions provided in Example 1, in which the culture medium contains glycerol as carbon source and lactose, LacNAc and LNB as acceptors.

### Example 27. Production of an oligosaccharide mixture comprising 3'SL, 6'SL, 3'SLacNAc, 6'SLacNAc, 3'SLNB and 6'SLNB with a modified E. coli host using lactose, LacNAc and LNB as acceptors

An *E. coli* K12 strain modified for sialic acid production as described in Example 1 was transformed with a plasmid expressing constitutive transcriptional units for the α-2,3-sialyltransferase from *P. multocida* with SEQ ID NO 23, the α-2,6-sialyltransferase gene *P. damselae* with SEQ ID NO 24 and NeuA from *P. multocida* with SEQ ID NO 22. The novel strain produces an oligosaccharide mixture comprising 3'SL, 6'SL, 3'SLacNAc, 6'SLacNAc, 3'SLNB and 6'SLNB in whole broth samples, when evaluated in a growth experiment according to the culture conditions provided in Example 1, in which the culture medium contains glycerol as carbon source and lactose, LacNAc and LNB as acceptors.

### Example 28. Production of an oligosaccharide mixture comprising, 2'FL, DiFL, 2'FLacNAc, LN3, LNT and LNFP-I with a modified E. coli host

An *E. coli* strain modified for GDP-fucose production as described in Example 1 was further adapted for lacto-N-triose (LN3, LNT-II, GlcNAc-b1,3-Gal-b1,4-Glc) and LNT (Gal-b1,3-GlcNAc-b1,3-Gal-b1,4-Glc) production by genomic knock-ins of constitutive transcriptional units for the mutant glmS*54 from *E. coli* with SEQ ID NO 19, the galactoside beta-1,3-N-acetylglucosaminyltransferase (LgtA) from *N. meningitidis* with SEQ ID NO 25 and the N-acetylglucosamine beta-1,3-galactosyltransferase (WbgO) from *E. coli* O55:H7 with SEQ ID NO 26. In a next step, the novel strain was further transformed with an expression plasmid containing a constitutive transcriptional unit for the a-1,2-fucosyltransferase from *H. pylori* with SEQ ID NO 04. The novel strain produces an oligosaccharide mixture comprising 2'FL, DiFL, 2'FLacNAc, LN3, LNT and lacto-N-fucopentaose I (LNFP-I, Fuc-a1,2-Gal-b1,3-GlcNAc-b1,3-Gal-b1,4-Glc) in whole broth samples when evaluated in a growth experiment according to the culture conditions provided in Example 1, in which the culture medium contains sucrose as carbon source and lactose and LacNAc as acceptors.

### Example 29. Production of an oligosaccharide mixture comprising, 2'FL, DiFL, 2'FLacNAc, LN3, LNT, LNFP-I and LNFP-II with a modified E. coli host

An E. coli strain adapted for LN3 (GlcNAc-b1,3-Gal-b1,4-Glc) and LNT (Gal-b1,3-GlcNAc-b1,3-Gal-b1,4-Glc) production as described in Example 28, was further transformed with an expression plasmid containing a constitutive transcriptional unit for the mutant a1,3/4 fucosidase from *Bifidobacterium longum* subsp. *infantis* ATCC 15697 with SEQ ID NO 30. The novel strain produces an oligosaccharide mixture comprising 2'FL, DiFL, 2'FLacNAc, LN3, LNT, LNFP-I and lacto-N-fucopentaose II (LNFP-II, Gal-b1,3-(Fuc-a1,4)-GlcNAc-b1,3-Gal-b1,4-Glc) in whole broth samples when evaluated in a growth experiment according to the culture conditions provided in Example 1, in which the culture medium contains sucrose as carbon source and lactose and LacNAc as acceptors.

### Example 30. Production of an oligosaccharide mixture comprising 3-FL, 3-FLacNAc, LN3, LNnT, LNFP-III and LNFP-VI with a modified E. coli host

An *E. coli* strain modified for GDP-fucose production as described in Example 1 was further adapted for LN3 and LNnT production by a genomic knock-ins of constitutive transcriptional units for the mutant *glmS*54* from *E. coli* with SEQ ID NO 19, LgtA from *N. meningitidis* with SEQ ID NO 25 and LgtB from N. *meningitidis* with SEQ ID NO 27. In a next step, the novel strain was further transformed with an expression plasmid containing constitutive transcriptional units for both the *H. pylori* a-1,3-fucosyltransferase with SEQ ID NO 05 and its truncated variant with SEQ ID NO 06. The novel strain produces an oligosaccharide mixture comprising 3-FL, 3-FLacNAc, LN3 and LNnT, lacto-N-fucopentaose III (LNFP-III, Gal-b1,4-(Fuc-a1,3)-GlcNAc-b1,3-Gal-b1,4-Glc) and lacto-N-fucopentaose VI (LNFP-VI, Gal-b1,4-GlcNAc-b1,3-Gal-b1,4-(Fuc-a1,3-)Glc) in whole broth samples when evaluated in a growth experiment according to the culture conditions provided in Example 1, in which the culture medium contains sucrose as carbon source and lactose and LacNAc as acceptors.

### Example 31. Production of an oligosaccharide mixture comprising LN3, sialylated LN3, LNT, 3'SL, 3'SLacNAc and LSTa with a modified E. coli host

An *E. coli* strain modified to produce sialic acid as described in Example 1 was further modified with a genomic knock-in of constitutive transcriptional units for the galactoside beta-1,3-N-acetylglucosaminyltransferase (LgtA) from *N. meningitidis* with SEQ ID NO 25 and for the N-acetylglucosamine beta-1,3-galactosyltransferase (WbgO) from *E. coli* O55:H7 with SEQ ID NO 26 to allow production of lacto-N-tetraose (LNT, Gal-b1,3-GlcNAc-b1,3-Gal-b1,4-Glc). In a next step, the novel strain was further modified with a genomic knock-out of the *E. coli lacZ* gene and transformed with an expression plasmid having constitutive transcriptional units for NeuA from *P. multocida* with SEQ ID NO 22 and the α-2,3-sialyltransferase from *P. multocida* with SEQ ID NO 23. The novel strain produces an oligosaccharide mixture comprising LN3, 3'-sialylated LN3 (Neu5Ac-a2,3-GlcNAc-b1,3-Gal-b1,4-Glc), LNT, 3'SL, 3'SLacNAc and LSTa (Neu5Ac-a2,3-Gal-b1,3-GlcNAc-b1,3-Gal-b1,4-Glc) when evaluated in a growth experiment according to the culture conditions provided in Example 1, in which the culture medium contains glycerol as carbon source and lactose and LacNAc as acceptors.

### Example 32. Production of an oligosaccharide mixture comprising LN3, sialylated LN3, LNnT, 6'SL, 6'SLacNAc and LSTc with a modified E. coli host

An *E. coli* strain modified to produce sialic acid as described in Example 1 was further modified with a genomic knock-in of constitutive transcriptional units for LgtA from *N. meningitidis* with SEQ ID NO 25 and for LgtB from *N. meningitidis* with SEQ ID NO 27 to allow production of lacto-*N*-neotetraose (LNnT, Gal-b1,4-GlcNAc-b1,3-Gal-b1,4-Glc). In a next step, the novel strain was further modified with a genomic knock-out of the *E. coli lacZ* gene and transformed with an expression plasmid having constitutive transcriptional units for NeuA from *P. multocida* with SEQ ID NO 22 and the α-2,6-sialyltransferase from *P. damselae* with SEQ ID NO 24. The novel strain produces an oligosaccharide mixture comprising LN3, 6'-sialylated LN3 (Neu5Ac-a2,6-(GlcNAc-b1,3)-Gal-b1,4-Glc), 6'SL, 6'SLacNAc, LNnT and LSTc (Neu5Ac-a2,6-Gal-b1,4-GlcNAc-b1,3-Gal-b1,4-Glc) when evaluated in a growth experiment according to the culture conditions provided in Example 1, in which the culture medium contains glycerol as carbon source and lactose and LacNAc as acceptors.

### Example 33. Production of an oligosaccharide mixture comprising 2'FL and DiFL with a modified S. cerevisiae host using lactose and 3-FL as acceptors

An *S. cerevisiae* strain was adapted for GDP-fucose production and fucosyltransferase expression as described in Example 2 with a yeast expression plasmid (a variant of p2a_2p_Fuc) comprising constitutive transcriptional units for the lactose permease (LAC12) from *K. lactis* with SEQ ID NO 29, the GDP-mannose 4,6-dehydratase (gmd) from *E. coli* with SEQ ID NO 11, the GDP-L-fucose synthase (fcl) from *E. coli* with SEQ ID NO 12 and the alpha-1,2-fucosyltransferase from *H. pylori* with SEQ ID NO 04. When evaluated on SD CSM-Ura drop-out medium comprising lactose and 3-FL as acceptors, the mutant yeast strain produces an oligosaccharide mixture comprising 2'FL and DiFL.

### Example 34. Production of an oligosaccharide mixture comprising 2'FL, 3-FL, DiFL, 2'FLacNAc, 3-FLacNAc and DiFLacNAc with a modified S. cerevisiae host using lactose and LacNAc as acceptors

An *S. cerevisiae* strain was adapted for GDP-fucose production and fucosyltransferase expression as described in Example 2 with a yeast expression plasmid (a variant of p2a_2µ_Fuc) comprising constitutive transcriptional units for the lactose permease (LAC12) from *K. lactis* with SEQ ID NO 29, the GDP-mannose 4,6-dehydratase (gmd) from *E. coli* with SEQ ID NO 11, the GDP-L-fucose synthase (fcl) from *E. coli* with SEQ ID NO 12, the alpha-1,2-fucosyltransferase from *H. pylori* with SEQ ID NO 04 and the alpha-1,3-fucosyltransferase from *H. pylori* with SEQ ID NO 05. When evaluated on SD CSM-Ura drop-out medium comprising lactose and LacNAc as acceptors, the mutant yeast strain produces an oligosaccharide mixture comprising 2'FL, 3-FL, DiFL, 2'FLacNAc, 3-FLacNAc and DiFLacNAc.

### Example 35. Production of an oligosaccharide mixture comprising 3'SL, 6'SL, 3'SLacNAc and 6'SLacNAc with a modified S. cerevisiae host using lactose and LacNAc as acceptors

An *S. cerevisiae* strain was adapted for production of CMP-sialic acid and for expression of two sialyltransferases as described in Example 2 with a yeast expression plasmid (a pRS420-plasmid variant) comprising constitutive transcriptional units for the mutant glmS*54 from *E. coli* with SEQ ID NO 19, the phosphatase yqaB from E. coli with SEQ ID NO 20, AGE from *B. ovatus* with SEQ ID NO 17, neuB from N. *meningitidis* with SEQ ID NO 18, neuA from *P. multocida* with SEQ ID NO 22, the α-2,3-sialyltransferase from *P. multocida* with SEQ ID NO 23 and the α-2,6-sialyltransferase from *P. damselae* with SEQ ID NO 24. When evaluated on SD CSM-Trp drop-out medium comprising lactose and LacNAc as acceptors, the mutant yeast strain produces an oligosaccharide mixture comprising 3'SL, 6'SL, 3'SLacNAc and 6'SLacNAc.

## Claims

1. A method to produce a mixture of at least two different oligosaccharides by a cell, the method comprising the steps of:
i. providing a cell expressing one glycosyltransferase and capable to synthesize one nucleotide-sugar, wherein said nucleotide-sugar is donor for said glycosyltransferase, and
ii. cultivating said cell under conditions permissive to express said glycosyltransferase and to synthesize said nucleotide-sugar, and
iii. addition of at least two acceptors to said cultivation enabling said cell to produce at least two oligosaccharides, preferably any one of said acceptors is a di- or oligosaccharide,
iv. preferably, separating at least one of said oligosaccharides from said cultivation, more preferably separating all of said oligosaccharides from said cultivation.

2. Method according to claim 1, wherein said cell is a metabolically engineered cell modified with at least one gene expression module, **characterized in that** the expression from any one of said expression modules is either constitutive or is created by a natural inducer.

3. Method according to any one of claim 1 and 2, wherein said cell produces a mixture of three or more different oligosaccharides.

4. Method according to any one of claim 1 to 3, wherein said cell produces a mixture of different oligosaccharides with at least two oligosaccharides differing in degree of polymerization.

5. Method according to any one of claims 1 to 4, wherein said glycosyltransferase is chosen from the list comprising fucosyltransferases, sialyltransferases, galactosyltransferases, glucosyltransferases, mannosyltransferases, N-acetylglucosaminyltransferases, N-acetylgalactosaminyltransferases, N-acetylmannosaminyltransferases, xylosyltransferases, glucuronyltransferases, galacturonyltransferases, glucosaminyltransferases, N-glycolylneuraminyltransferases, rhamnosyltransferases, preferably said cell is modified in the expression or activity of said glycosyltransferase.

6. Method according to any one of claim 1 to 5, wherein said nucleotide-sugar is chosen from the list comprising GDP-Fuc, CMP-Neu5Ac, UDP-GlcNAc, UDP-Gal, UDP-N-acetylgalactosamine (UDP-GalNAc), UDP-N-acetylmannosamine (UDP-ManNAc), GDP-mannose (GDP-Man), UDP-glucose (UDP-Glc), CMP-N-glycolylneuraminic acid (CMP-Neu5Gc), UDP-glucuronate, UDP-galacturonate, GDP-rhamnose, UDP-xylose.

7. Method according to any one of claim 1 to 6, wherein any one of said acceptors has a degree of polymerization of 3 or more, preferably wherein all of said acceptors have a degree of polymerization of 3 or more.

8. Method according to any one of claim 1 to 7, wherein all said acceptors have a different degree of polymerization.

9. Method according to any one of claim 1 to 8, wherein said cultivation is supplemented with at least 3 acceptors for the production of said oligosaccharide mixture.

10. Method according to any one of claim 1 to 9, wherein said oligosaccharide mixture comprises at least one oligosaccharide that is fucosylated, sialylated, galactosylated, glucosylated, xylosylated, mannosylated, contains an N-acetylglucosamine, contains an N-acetylneuraminate, contains an N-glycolylneuraminate, contains an N-acetylgalactosamine, contains a rhamnose, contains a glucuronate, contains a galacturonate, and/or contains an N-acetylmannosamine.

11. Method according to any one of claims 1 to 10, wherein said cell is further metabolically engineered for
i. modified expression of an endogenous membrane protein, and/or
ii. modified activity of an endogenous membrane protein, and/or
iii. expression of a homologous membrane protein, and/or
iv. expression of a heterologous membrane protein,
wherein said membrane protein is involved in the secretion of any one of said oligosaccharides from said cell, preferably wherein said membrane protein is involved in the secretion of all of said oligosaccharides from said cell.

12. Method according to any one of claims 1 to 11, wherein said cell is further metabolically engineered for
i. modified expression of an endogenous membrane protein, and/or
ii. modified activity of an endogenous membrane protein, and/or
iii. expression of a homologous membrane protein, and/or
iv. expression of a heterologous membrane protein,
wherein said membrane protein is involved in the uptake of a precursor for the synthesis of any one of said oligosaccharides of the mixture, preferably wherein said membrane protein is involved in the uptake of all of the required precursors, more preferably wherein said membrane protein is involved in the uptake of all of said acceptors.

13. Method according to any one of claims 1 to 12, wherein any one of said oligosaccharides is a mammalian milk oligosaccharide, preferably wherein all said oligosaccharides are mammalian milk oligosaccharides.

14. Use of a method according to any one of claims 1 to 13 for the production of a mixture of at least two different oligosaccharides.
